(19) Europäisches Patentamt / European Patent Office / Office européen des brevets

(11) **EP 4 467 536 A1**

(12) **EUROPEAN PATENT APPLICATION**
published in accordance with Art. 153(4) EPC

(43) Date of publication:
**27.11.2024 Bulletin 2024/48**

(21) Application number: **23742962.6**

(22) Date of filing: **19.01.2023**

(51) International Patent Classification (IPC):
***C07D 401/14*** *(2006.01)* ***A61K 31/454*** *(2006.01)*
***A61P 35/00*** *(2006.01)*

(52) Cooperative Patent Classification (CPC):
**A61K 31/454; A61P 35/00; C07D 401/14**

(86) International application number:
**PCT/CN2023/073077**

(87) International publication number:
**WO 2023/138647 (27.07.2023 Gazette 2023/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.01.2022 CN 202210060175**

(71) Applicants:
- **Jiangsu Hengrui Pharmaceuticals Co., Ltd.**
  **Lianyungang, Jiangsu 222047 (CN)**
- **Shanghai Hengrui Pharmaceutical Co., Ltd.**
  **Shanghai 200245 (CN)**

(72) Inventors:
- **ZHANG, Baolei**
  **Shanghai 200245 (CN)**
- **XI, Zhuoxun**
  **Shanghai 200245 (CN)**
- **FENG, Jun**
  **Shanghai 200245 (CN)**
- **HE, Feng**
  **Shanghai 200245 (CN)**
- **YANG, Junran**
  **Lianyungang, Jiangsu 222047 (CN)**
- **DU, Zhenxing**
  **Lianyungang, Jiangsu 222047 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) CRYSTALLINE FORM OF SULFUR-CONTAINING ISOINDOLINE DERIVATIVE

(57) The present disclosure relates to a crystalline form of a sulfur-containing isoindoline derivative. Specifically, the present disclosure relates to a crystalline form of a compound as represented by formula (I) and a preparation method therefor.

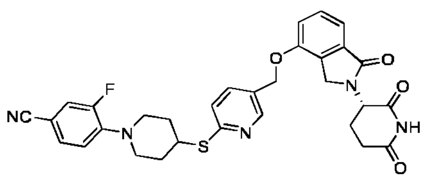

(I)



Processed by Luminess, 75001 PARIS (FR)

## Description

The present application claims the right of the priority of Chinese patent application 202210060175.6 filed on January 19, 2022. The content of the above Chinese patent application is incorporated herein by reference in its entirety.

TECHNICAL FIELD

The present disclosure relates to a crystal form of a sulfur-containing isoindoline derivative and a preparation method therefor. Specifically, the present disclosure provides a crystal form of a compound of formula I and a preparation method therefor.

BACKGROUND

Multiple myeloma (MM) is a malignant tumor, the main symptoms of which include hypercalcemia, renal injury, anemia, and bone disease. MM is the second most common hematological malignancy following non-Hodgkin's lymphoma. The current treatment methods are mainly drug therapy and autologous stem cell transplantation.

At present, there are four main classes of drugs widely used in clinical practice, which are alidomide immunomodulator, proteasome inhibitors, hormones and monoclonal antibodies. The drugs in clinical research stage include diabody, ADC, CAR-T, etc. Those drugs have different mechanisms of action, and they can often achieve better efficacy when used in combination. In clinical practice, dual, triple or even quadruple therapy is generally adopted, usually a combination therapy of an immunomodulator, a proteasome inhibitor and a hormone, sometimes with an additional antibody. Lenalidomide is the most commonly used immunomodulator, and is used in first-line therapy, maintenance therapy after stem cell transplantation, and second-line and third-line therapies after relapse. The drug reached $9.7 billion in sales in 2018/2019. In addition, the overall MM market is considerable and growing rapidly due to longer patient survival, and correspondingly longer duration of medication after continuous improvements and refinements in the diagnosis and treatment of MM. The MM market is expected to reach a scale of $33 billion in 2022, with the largest proportion still being immunomodulators such as lenalidomide.

The mechanism of action of immunomodulators (IMiD) for treating MM is mainly that IMiD drugs can activate the E3 ligase activity of CRBN after binding to Cereblon (CRBN) protein to selectively bind to transcription factors Ikaros (IKZF 1) and Aiolos (IKZF3), thereby resulting in rapid ubiquitination and degradation of Ikaros and Aiolos. Downregulation of Ikaros/Aiolos results in downregulation of c-Myc, followed by the downregulation of IRF4, ultimately resulting in inhibition of growth and apoptosis of myeloma cells. In addition, IKZF3 can also inhibit the transcription of cytokines IL2 and TNF in T/NK cells. After the degradation of IKZF3, this inhibition can be relieved, and the release of these cytokines is promoted, so that an immunomodulatory effect is achieved. Clinical trials have also shown that the clinical benefit of IMiD drugs correlates with the expression level of CRBN. It was found that the inhibitory activity of lenalidomide for cell growth was lost and drug resistance was developed after CRBN was knocked down in lenalidomide-sensitive cell lines (OPM2 and KMS18), which indicates that the level of CRBN knock-down correlates with the degree of drug resistance. In a cell proliferation experiment, after the expression level of CRBN in cells (U266-CRBN60 and U266-CRBN75) was reduced, the inhibitory activity of both lenalidomide and pomalidomide for cell growth is reduced.

Currently, the IMiD drugs that have been approved for marketing include thalidomide, lenalidomide and pomalidomide, all from Celgene (now incorporated by BMS). For those three compounds, the binding forces to CRBN are increased sequentially, so the clinical doses are reduced sequentially. The primary indication for the three compounds is MM, and there are other indications for thalidomide and lenalidomide (especially lenalidomide), such as myelodysplastic syndrome (MDS). In terms of side effects, lenalidomide and pomalidomide are similar in performance, with significant myelosuppressive effect caused by target-related toxicity; thalidomide has some other side effects such as sedation, constipation, and neurological side effects.

The adipimide moiety of all IMiDs binds to a hydrophobic pocket defined by the three tryptophan residues in CRBN (referred to as "thalidomide binding pocket"). In contrast, the phthalimide/isoindolone ring is exposed to the solvent and alters the molecular surface of CRBN, thereby modulating substrate recognition. Different IMiDs result in significant modification of the surface of CRBN molecules and different preferences for substrate recognition. Thus, modifications of IMiDs may lead to degradation of other transcription factors, causing unwanted toxic and side effects. This mode of action of IMiDs is also known as molecular glue, which vividly expresses the bonding effect of this small molecule on two protein substrates.

Since the median survival for multiple myeloma is now more than five years, the prolonged survival leads to a high proportion of resistance to currently available drugs such as lenalidomide and pomalidomide in most patients, which seriously reduces the therapeutic effect of such drugs. Therefore, it is contemplated to develop more active drug molecules to overcome the problem of drug resistance whilst minimizing the toxic and side effects of such compounds.

Disclosed patent applications for Cereblon regulators include WO2008115516A2, WO2011100380A1,

WO2019226770A1, WO2019014100A1, WO2020064002A1, etc.

The applicant's patent application WO2022017365 discloses a compound of formula I, chemically named (*S*)-4-(4-((5-(((2-(2,6-dioxopiperidin-3-yl)-1-oxoisoindolin-4-yl)oxy)methyl)pyridin-2-yl)thio)piperidin-1-yl)-3-fluorobenzonitrile. This compound exhibits a relatively good Cereblon modulating activity.

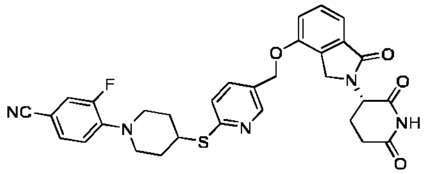

Formula I

Generally, the crystal forms of the active ingredients of a drug and its salts not only affect the physical and chemical stability of the drug itself, but also affect the difficulty of later drug preparation and production costs. Different crystallization and storage conditions may lead to changes in the crystal forms of the compound and its salts, sometimes resulting in the formation of other forms of crystal forms. Therefore, considering comprehensively from the perspectives of stability, ease of drug preparation process, production costs, *etc.,* it is necessary to conduct an in-depth study of the crystal forms of the compound of formula (I).

CONTENT OF THE PRESENT INVENTION

The present disclosure provides a crystal form of a compound of formula (I) and a preparation method therefor, wherein the compound of formula I is chemically named (*S*)-4-(4-((5-(((2-(2,6-dioxopiperidin-3-yl)-1-oxoisoindolin-4-yl)oxy)methyl)pyridin-2-yl)thio)piperidin-1-yl)-3-fluorobenzonitrile,

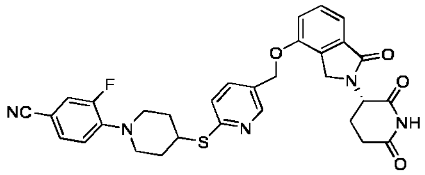

formula I.

The present disclosure provides an amorphous form of the compound of formula (I), which has an X-ray powder diffraction pattern comprising no obvious characteristic peaks at a diffraction angle 2θ in the range of 2 to 48°.

The present disclosure further provides a method for preparing the amorphous form of the compound of formula (I), comprising method 1: a) mixing the compound of formula (I) with propylene glycol methyl ether, n-heptane, or petroleum ether; b) slurrying for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of the compound of formula (I) according to the present disclosure further includes steps of filtering, washing, or drying.

The present disclosure provides a crystal form A of the compound of formula (I), which has an X-ray powder diffraction pattern, represented by a diffraction angle 2θ, having characteristic peaks at 5.765, 8.061, 9.925, 16.632, 17.900, 19.469, and 21.115. In some embodiments, the crystal form A of the compound of formula (I) has characteristic peaks at 5.765, 7.465, 8.061, 9.925, 12.890, 15.085, 16.632, 17.900, 19.469, and 21.115. In some embodiments, the crystal form A of the compound of formula (I) has characteristic peaks at 5.765, 7.465, 8.061, 9.925, 11.674, 12.890, 14.270, 15.085, 16.632, 17.900, 18.715, 19.469, and 21.115. In some embodiments, the crystal form A of the compound of formula (I) has the X-ray powder diffraction pattern represented by the diffraction angle 2θ as shown in Figure 2.

The present disclosure further provides a method for preparing the crystal form A of the compound of formula (I), comprising method 1: a) mixing the compound of formula (I) with tetrahydrofuran and dissolving to clarification; b) adding *n*-heptane for crystallization;

or method 2: a) mixing the compound of formula (I) with dichloromethane and dissolving to clarification; b) adding ethyl

acetate for crystallization;

or method 3: a) mixing the compound of formula (I) with water, isopropyl acetate, acetone, ethyl acetate/ethanol, or ethyl acetate/*n*-heptane; b) slurrying for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form A of the compound of formula (I) according to the present disclosure further includes steps of filtering, washing, drying, etc.

The present disclosure provides a crystal form B of the compound of formula (I), which has an X-ray powder diffraction pattern, represented by a diffraction angle 2θ, having characteristic peaks at 4.977, 6.788, 10.047, 14.143, 15.684, 18.547, and 20.840. In some embodiments, the crystal form B of the compound of formula (I) has characteristic peaks at 4.977, 6.788, 10.047, 14.143, 15.684, 18.547, 20.840, 24.096, and 25.505. In some embodiments, the crystal form B of the compound of formula (I) has the X-ray powder diffraction pattern represented by the diffraction angle 2θ as shown in Figure 3.

The present disclosure further provides a method for preparing the crystal form B of the compound of formula (I), comprising a) mixing the compound of formula (I) with ethyl acetate or acetonitrile/methanol; b) slurrying for crystallization, and drying at 30°C.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form B of the compound of formula (I) according to the present disclosure further includes steps of filtering, washing, drying, etc.

The present disclosure provides a crystal form C of the compound of formula (I), which has an X-ray powder diffraction pattern, represented by a diffraction angle 2θ, having characteristic peaks at 5.653, 7.974, 9.989, 16.143, 17.860, 18.992, and 20.972. In some embodiments, the crystal form C of the compound of formula (I) has characteristic peaks at 5.653, 7.974, 9.989, 11.505, 12.798, 14.265, 16.143, 17.860, 18.992, and 20.972. In some embodiments, the crystal form C of the compound of formula (I) has characteristic peaks at 3.533, 5.653, 7.974, 8.790, 9.989, 11.505, 12.798, 14.265, 15.277, 16.143, 17.860, 18.992, and 20.972. In some embodiments, the crystal form C of the compound of formula (I) has the X-ray powder diffraction pattern represented by the diffraction angle 2θ as shown in Figure 4.

The present disclosure further provides a method for preparing the crystal form C of the compound of formula (I), comprising method 1: a) mixing the compound of formula I with ethanol, isopropanol, methyl *tert*-butyl ether, methyl isobutyl ketone, water/ethanol, water/isopropanol, water/methanol (1:1), cyclohexane, or isopropyl ether; b) slurrying for crystallization;

or method 2: a) mixing the compound of formula (I) with dichloromethane, water/acetone, or tetrahydrofuran/ethanol, and dissolving to clarification; b) evaporating for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form C of the compound of formula (I) according to the present disclosure further includes steps of filtering, washing, drying, etc.

The present disclosure provides a crystal form D of the compound of formula (I), which has an X-ray powder diffraction pattern, represented by a diffraction angle 2θ, having characteristic peaks at 6.831, 9.845, 13.453, 18.225, 20.117, 20.891, and 23.006. In some embodiments, the crystal form D of the compound of formula (I) has characteristic peaks at 6.831, 9.845, 10.927, 13.453, 16.096, 18.225, 20.117, 20.891, 23.006, and 26.132. In some embodiments, the crystal form D of the compound of formula (I) has the X-ray powder diffraction pattern represented by the diffraction angle 2θ as shown in Figure 5.

The present disclosure further provides a method for preparing the crystal form D of the compound of formula (I), comprising:

method 1: a) mixing the compound of formula (I) with acetonitrile; b) slurrying for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form D of the compound of formula (I) according to the present disclosure further includes steps of filtering, washing, drying, etc.

The present disclosure provides a crystal form E of the compound of formula (I), which has an X-ray powder diffraction pattern, represented by a diffraction angle 2θ, having characteristic peaks at 5.864, 7.573, 8.087, 10.003, 16.444, 19.349, and 20.553. In some embodiments, the crystal form E of the compound of formula (I) has characteristic

peaks at 5.864, 7.573, 8.087, 10.003, 12.471, 15.165, 16.444, 17.432, 19.349, and 20.553. In some embodiments, the crystal form E of the compound of formula (I) has characteristic peaks at 5.864, 7.573, 8.087, 10.003, 11.701, 12.471, 15.165, 16.444, 17.432, 19.349, 20.553, 21.067, and 21.709. In some embodiments, the crystal form E of the compound of formula (I) has the X-ray powder diffraction pattern represented by the diffraction angle 2θ as shown in Figure 6.

The present disclosure further provides a method for preparing the crystal form E of the compound of formula (I), comprising method 1: a) mixing the compound of formula I with 2-butanone or 10% water/methanol; b) slurrying for crystallization;
or method 2: a) mixing the compound of formula I with DCM and dissolving to clarification; b) adding *n*-heptane for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form E of the compound of formula (I) according to the present disclosure further includes steps of filtering, washing, drying, etc.

The present disclosure provides a crystal form F of the compound of formula (I), which has an X-ray powder diffraction pattern, represented by a diffraction angle 2θ, having characteristic peaks at 5.062, 7.820, 10.077, 14.231, 16.672, 18.586, and 20.435. In some embodiments, the crystal form F of the compound of formula (I) has characteristic peaks at 5.062, 7.820, 10.077, 14.231, 15.192, 16.672, 18.586, 20.435, 21.868, and 25.442. In some embodiments, the crystal form F of the compound of formula (I) has characteristic peaks at 5.062, 7.820, 10.077, 14.231, 15.192, 16.672, 18.586, 20.435, 21.868, 24.193, 25.442, 26.303, and 28.629. In some embodiments, the crystal form F of the compound of formula (I) has the X-ray powder diffraction pattern represented by the diffraction angle 2θ as shown in Figure 7.

The present disclosure further provides a method for preparing the crystal form F of the compound of formula (I), comprising:

method 1: a) mixing the compound of formula (I) with a solvent II, wherein the solvent II is selected from *p*-xylene, methanol, 2-methyltetrahydrofuran, *o*-xylene, or toluene; b) slurrying for crystallization;
or method 2: a) mixing the compound of formula (I) with dichloromethane or tetrahydrofuran and dissolving to clarification; b) adding isopropyl acetate, methyl *tert*-butyl ether, or methyl isobutyl ketone for crystallization;
or method 3: a) mixing the compound of formula (I) with 1,4-dioxane and dissolving to clarification; b) evaporating for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form F of the compound of formula (I) according to the present disclosure further includes steps of filtering, washing, drying, etc.

The present disclosure provides a crystal form G of the compound of formula (I), which has an X-ray powder diffraction pattern, represented by a diffraction angle 2θ, having characteristic peaks at 5.999, 7.972, 9.951, 11.388, 17.812, 20.975, and 25.819. In some embodiments, the crystal form G of the compound of formula (I) has characteristic peaks at 5.999, 7.396, 7.972, 8.637, 9.951, 11.388, 15.291, 17.812, 20.975, and 25.819. In some embodiments, the crystal form G of the compound of formula (I) has characteristic peaks at 5.999, 7.396, 7.972, 8.637, 9.951, 11.388, 12.763, 15.291, 17.812, 20.975, 23.408, 25.819, and 27.400. In some embodiments, the crystal form G of the compound of formula (I) has the X-ray powder diffraction pattern represented by the diffraction angle 2θ as shown in Figure 8.

The present disclosure further provides a method for preparing the crystal form G of the compound of formula (I), comprising:

method 1: a) mixing the compound of formula (I) with chloroform and dissolving to clarification; b) evaporating for crystallization;
or method 2: a) mixing the compound of formula (I) with 1,2-dichloroethane; b) slurrying for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form G of the compound of formula (I) according to the present disclosure further includes steps of filtering, washing, drying, etc.

The present disclosure provides a crystal form H of the compound of formula (I), which has an X-ray powder diffraction pattern, represented by a diffraction angle 2θ, having characteristic peaks at 5.758, 7.533, 9.901, 14.267, 16.420, 18.103, and 26.356. In some embodiments, the crystal form H of the compound of formula (I) has characteristic

peaks at 5.758, 7.533, 9.901, 14.267, 16.420, 18.103, 18.917, 20.489, 24.049, and 26.356. In some embodiments, the crystal form H of the compound of formula (I) has characteristic peaks at 5.758, 7.533, 9.901, 14.267, 16.420, 18.103, 18.917, 20.489, 24.049, and 26.356. In some embodiments, the crystal form H of the compound of formula (I) has the X-ray powder diffraction pattern represented by the diffraction angle 2θ as shown in Figure 9.

The present disclosure further provides a method for preparing the crystal form H of the compound of formula (I), comprising:

method 1: a) mixing the compound of formula (I) with *n*-octane or *n*-hexane; b) slurrying for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form H of the compound of formula (I) according to the present disclosure further includes steps of filtering, washing, drying, etc.

The present disclosure provides a crystal form I of the compound of formula (I), which has an X-ray powder diffraction pattern, represented by a diffraction angle 2θ, having characteristic peaks at 5.294, 6.826, 7.564, 10.739, 13.699, 16.812, and 20.709. In some embodiments, the crystal form I of the compound of formula (I) has the X-ray powder diffraction pattern represented by the diffraction angle 2θ as shown in Figure 10.

The present disclosure further provides a method for preparing the crystal form I of the compound of formula (I), comprising:

method 1: a) mixing the compound of formula (I) with tetrahydrofuran and dissolving to clarification; b) evaporating for crystallization;

or method 2: a) mixing the compound of formula (I) with tetrahydrofuran and dissolving to clarification; b) adding water for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form I of the compound of formula (I) according to the present disclosure further includes steps of filtering, washing, drying, etc.

The present disclosure provides a crystal form J of the compound of formula (I), which has an X-ray powder diffraction pattern, represented by a diffraction angle 2θ, having characteristic peaks at 5.041, 10.068, 16.424, 20.544, 21.190, 24.077, and 25.433. In some embodiments, the crystal form J of the compound of formula (I) has characteristic peaks at 5.041, 8.212, 10.068, 14.101, 15.167, 16.424, 20.544, 21.190, 24.077, and 25.433. In some embodiments, the crystal form J of the compound of formula (I) has characteristic peaks at 5.041, 8.212, 10.068, 14.101, 15.167, 16.424, 20.544, 21.190, 22.036, 22.679, 24.077, 25.433, and 26.454. In some embodiments, the crystal form J of the compound of formula (I) has the X-ray powder diffraction pattern represented by the diffraction angle 2θ as shown in Figure 11.

The present disclosure further provides a method for preparing the crystal form J of the compound of formula (I), comprising: a) mixing the compound of formula (I) with dimethyl sulfoxide and dissolving to clarification; b) adding water, isopropyl acetate, or methyl *tert*-butyl ether for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form J of the compound of formula (I) according to the present disclosure further includes steps of filtering, washing, drying, etc.

The present disclosure provides a crystal form K of the compound of formula (I), which has an X-ray powder diffraction pattern, represented by a diffraction angle 2θ, having characteristic peaks at 5.156, 7.699, 10.339, 14.334, 16.203, 18.327, and 23.418. In some embodiments, the crystal form K of the compound of formula (I) has characteristic peaks at 5.156, 7.699, 10.339, 14.334, 16.203, 18.327, 23.418, 25.348, 25.919, and 26.446. In some embodiments, the crystal form K of the compound of formula (I) has the X-ray powder diffraction pattern represented by the diffraction angle 2θ as shown in Figure 12.

The present disclosure further provides a method for preparing the crystal form K of the compound of formula (I), comprising: a) mixing the compound of formula (I) with *N*-methylpyrrolidone and dissolving to clarification; b) evaporating for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form K of the compound of formula (I) further includes steps of filtering, washing, drying, etc.

The present disclosure provides a crystal form L of the compound of formula (I), which has an X-ray powder diffraction pattern, represented by a diffraction angle 2θ, having characteristic peaks at 4.932, 5.360, 9.831, 14.844, 18.244, 20.104, and 24.914. In some embodiments, the crystal form L of the compound of formula (I) has characteristic peaks at 4.932, 5.360, 9.831, 10.753, 14.844, 16.369, 18.244, 20.104, 23.129, and 24.914. In some embodiments, the crystal form L of the compound of formula (I) has the X-ray powder diffraction pattern represented by the diffraction angle 2θ as shown in Figure 13.

The present disclosure further provides a method for preparing the crystal form L of the compound of formula (I), comprising: a) mixing the compound of formula (I) with *N,N*-dimethylacetamide and dissolving to clarification; b) evaporating for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form L of the compound of formula (I) according to the present disclosure further includes steps of filtering, washing, drying, etc.

The present disclosure provides a crystal form M of the compound of formula (I), which has an X-ray powder diffraction pattern, represented by a diffraction angle 2θ, having characteristic peaks at 14.959, 16.322, 18.410, 20.748, 22.067, 23.670, and 26.863. In some embodiments, the crystal form M of the compound of formula (I) has characteristic peaks at 14.959, 16.322, 18.410, 20.748, 22.067, 23.670, 24.839, 25.873, 26.863, and 27.811. In some embodiments, the crystal form M of the compound of formula (I) has characteristic peaks at 14.959, 16.322, 18.410, 20.748, 22.067, 23.670, 24.322, 24.839, 25.873, 26.863, and 27.811. In some embodiments, the crystal form M of the compound of formula (I) has the X-ray powder diffraction pattern represented by the diffraction angle 2θ as shown in Figure 14.

The present disclosure further provides a method for preparing the crystal form M of the compound of formula (I), comprising: a) heating the crystal form A, crystal form B, crystal form C, crystal form E, crystal form H, or crystal form I of the compound of formula (I) to 225°C.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form M of the compound of formula (I) according to the present disclosure further includes steps of filtering, washing, drying, etc.

The present disclosure provides a crystal form N of the compound of formula (I), which has an X-ray powder diffraction pattern, represented by a diffraction angle 2θ, having characteristic peaks at 5.028, 9.942, 10.900, 15.428, 18.410, 20.274, and 25.252. In some embodiments, the crystal form N of the compound of formula (I) has characteristic peaks at 5.028, 7.671, 9.942, 10.900, 15.428, 16.560, 18.410, 20.274, 24.036, and 25.252. In some embodiments, the crystal form N of the compound of formula (I) has characteristic peaks at 5.028, 7.671, 9.942, 10.900, 12.677, 15.428, 16.560, 18.410, 20.274, 24.036, 25.252, and 26.385. In some embodiments, the crystal form N of the compound of formula (I) has the X-ray powder diffraction pattern represented by the diffraction angle 2θ as shown in Figure 15.

The present disclosure further provides a method for preparing the crystal from N of the compound of formula (I), comprising: a) mixing the compound of formula (I) with ethyl acetate/tetrahydrofuran (1:1), heating and dissolving to clarification; b) cooling for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form N of the compound of formula (I) according to the present disclosure further includes steps of filtering, washing, drying, etc.

The present disclosure provides a crystal form O of the compound of formula (I), which has an X-ray powder diffraction pattern, represented by a diffraction angle 2θ, having characteristic peaks at 11.796, 17.423, 18.081, 19.136, 21.707, 22.165, and 25.719. In some embodiments, the crystal form O of the compound of formula (I) has characteristic peaks at 7.843, 11.796, 17.423, 18.081, 19.136, 21.707, 22.165, 24.412, 25.719, and 28.521. In some embodiments, the crystal form O of the compound of formula (I) has characteristic peaks at 7.843, 11.796, 15.455, 17.423, 18.081, 19.136, 21.055, 21.707, 22.165, 24.412, 25.719, 27.538, and 28.521. In some embodiments, the crystal form O of the compound of formula (I) has the X-ray powder diffraction pattern represented by the diffraction angle 2θ as shown in Figure 16.

The present disclosure further provides a method for preparing the crystal from O of the compound of formula (I), comprising: a) mixing the compound of formula I with acetonitrile and benzenesulfonic acid, and heating; b) cooling for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form O of the compound of formula (I) according to the

present disclosure further includes steps of filtering, washing, drying, etc.

The present disclosure provides a crystal form P of the compound of formula (I), which has an X-ray powder diffraction pattern, represented by a diffraction angle 2θ, having characteristic peaks at 5.359, 7.491, 10.786, 14.249, 16.527, 17.729, and 20.798. In some embodiments, the crystal form P of the compound of formula (I) has characteristic peaks at 5.359, 7.491, 9.905, 10.786, 13.192, 14.249, 16.527, 17.729, 18.862, and 20.798. In some embodiments, the crystal form P of the compound of formula (I) has characteristic peaks at 5.359, 7.491, 9.905, 10.786, 13.192, 14.249, 16.527, 17.729, 18.862, 20.798, 23.799, and 26.555. In some embodiments, the crystal form P of the compound of formula (I) has the X-ray powder diffraction pattern represented by the diffraction angle 2θ as shown in Figure 17.

The present disclosure further provides a method for preparing the crystal form P of the compound of formula (I), comprising: a) mixing the crystal form C of the compound of formula (I) with 1,4-dioxane; b) slurrying for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form P of the compound of formula (I) according to the present disclosure further includes steps of filtering, washing, drying, etc.

The present disclosure provides a crystal form Q of the compound of formula (I), which has an X-ray powder diffraction pattern, represented by a diffraction angle 2θ, having characteristic peaks at 5.038, 10.152, 15.850, 16.574, 18.892, 20.760, and 21.835. In some embodiments, the crystal form Q of the compound of formula (I) has characteristic peaks at 5.038, 10.152, 11.175, 15.850, 16.574, 18.892, 20.760, 21.835, 23.905, and 25.784. In some embodiments, the crystal form Q of the compound of formula (I) has characteristic peaks at 5.038, 7.682, 10.152, 11.175, 14.218, 15.850, 16.574, 18.892, 20.760, 21.835, 23.905, 25.784, and 26.418. In some embodiments, the crystal form Q of the compound of formula (I) has the X-ray powder diffraction pattern represented by the diffraction angle 2θ as shown in Figure 18.

The present disclosure further provides a method for preparing the crystal form Q of the compound of formula (I), comprising method 1: a) mixing the compound of formula (I) with ethyl acetate; b) slurrying for crystallization, and drying the solid at 70°C or 130°C.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form Q of the compound of formula (I) according to the present disclosure further includes steps of filtering, washing, drying, etc.

The present disclosure provides a crystal form U of the compound of formula (I), which has an X-ray powder diffraction pattern, represented by a diffraction angle 2θ, having characteristic peaks at 14.155, 15.745, 17.314, 17.997, 18.838, 20.512, and 21.415. In some embodiments, the crystal form U of the compound of formula (I) has characteristic peaks at 14.155, 15.745, 16.564, 17.314, 17.997, 18.838, 20.512, 21.415, 23.557, and 26.313. In some embodiments, the crystal form U of the compound of formula (I) has characteristic peaks at 7.657, 14.155, 15.745, 16.564, 17.314, 17.997, 18.838, 20.512, 21.415, 23.557, 25.711, 26.313, and 28.029. In some embodiments, the crystal form U of the compound of formula (I) has the X-ray powder diffraction pattern represented by the diffraction angle 2θ as shown in Figure 19.

The present disclosure further provides a method for preparing the crystal form U of the compound of formula (I), comprising method 1: a) mixing the crystal form Q of the compound of formula (I) with water; b) slurrying for crystallization; or method 2: a) mixing the compound of formula I with DMSO, heating and dissolving to clarification; b) adding water for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form U of the compound of formula (I) according to the present disclosure further includes steps of filtering, washing, drying, etc.

The present disclosure provides a crystal form X of the compound of formula (I), which has an X-ray powder diffraction pattern, represented by a diffraction angle 2θ, having characteristic peaks at 6.864, 9.873, 10.963, 13.801, 16.089, 18.006, and 20.929. In some embodiments, the crystal form X of the compound of formula (I) has characteristic peaks at 6.864, 9.873, 10.963, 13.801, 16.089, 18.006, 20.929, and 26.203. In some embodiments, the crystal form X of the compound of formula (I) has the X-ray powder diffraction pattern represented by the diffraction angle 2θ as shown in Figure 20.

The present disclosure further provides a method for preparing the crystal form X of the compound of formula (I): comprising: method 1: a) mixing the crystal form D of the compound of formula (I) with isopropyl ether or *n*-heptane; b) slurrying for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190,

200. In some embodiments, the method for preparing the crystal form X of the compound of formula (I) according to the present disclosure further includes steps of filtering, washing, drying, etc.

The present disclosure provides a crystal form Y of the compound of formula (I), which has an X-ray powder diffraction pattern, represented by a diffraction angle 2θ, having characteristic peaks at 5.676, 7.666, 14.260, 16.562, 18.020, 21.802, and 26.425. In some embodiments, the crystal form Y of the compound of formula (I) has characteristic peaks at 5.676, 7.666, 9.985, 12.634, 14.260, 16.562, 18.020, 21.802, 26.425, and 26.974. In some embodiments, the crystal form Y of the compound of formula (I) has characteristic peaks at 5.676, 7.666, 9.985, 12.634, 14.260, 16.562, 18.020, 21.802, 24.051, 25.846, 26.425, and 26.974. In some embodiments, the crystal form Y of the compound of formula (I) has the X-ray powder diffraction pattern represented by the diffraction angle 2θ as shown in Figure 21.

The present disclosure further provides a method for preparing the crystal form Y of the compound of formula (I): comprising: a) mixing the crystal form Q of the compound of formula (I) with isopropyl ether or *n*-heptane; b) slurrying for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form Y of the compound of formula (I) according to the present disclosure further includes steps of filtering, washing, drying, etc.

The present disclosure provides a crystal form V of the compound of formula (I), which has an X-ray powder diffraction pattern, represented by a diffraction angle 2θ, having characteristic peaks at 5.649, 6.154, 6.720, 11.651, 18.757, 19.813, and 23.948. In some embodiments, the crystal form V of the compound of formula (I) has characteristic peaks at 5.649, 6.154, 6.720, 9.778, 11.651, 17.570, 18.757, 19.813, 23.948, and 26.995. In some embodiments, the crystal form V of the compound of formula (I) has characteristic peaks at 5.649, 6.154, 6.720, 9.778, 11.651, 13.576, 17.570, 18.757, 19.813, 21.905, 23.948, 25.825, and 26.995. In some embodiments, the crystal form V of the compound of formula (I) has the X-ray powder diffraction pattern represented by the diffraction angle 2θ as shown in Figure 22.

The present disclosure further provides a method for preparing the crystal form V of the compound of formula (I), comprising method 1: a) mixing the compound of formula (I) with *N,N*-dimethylformamide and dissolving to clarification; b) adding acetone or acetonitrile for crystallization;

or method 2: a) mixing the compound of formula (I) with X,X-dimethylformamide; b) cooling for crystallization or stirring for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form V of the compound of formula (I) according to the present disclosure further includes steps of filtering, washing, drying, etc.

The present disclosure provides a crystal form R of the compound of formula (I), which has an X-ray powder diffraction pattern, represented by a diffraction angle 2θ, having characteristic peaks at 5.534, 7.611, 10.033, 15.782, 17.101, 19.017, and 20.567. In some embodiments, the crystal form R of the compound of formula (I) has characteristic peaks at 5.534, 7.611, 10.033, 11.857, 12.737, 15.782, 17.101, 19.017, 20.567, and 23.692. In some embodiments, the crystal form R of the compound of formula (I) has characteristic peaks at 5.534, 7.611, 10.033, 11.148, 11.857, 12.737, 14.179, 15.782, 17.101, 19.017, 20.567, 21.871, and 23.692. In some embodiments, the crystal form R of the compound of formula (I) has the X-ray powder diffraction pattern represented by the diffraction angle 2θ as shown in Figure 23.

The present disclosure further provides a method for preparing the crystal form R of the compound of formula (I), comprising: a) mixing the compound of formula (I) with tetrahydrofuran and dissolving; b) concentrating for crystallization.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form R of the compound of formula (I) according to the present disclosure further includes steps of filtering, washing, drying, etc.

The present disclosure provides a crystal form S of the compound of formula (I), which has an X-ray powder diffraction pattern, represented by a diffraction angle 2θ, having characteristic peaks at 6.576, 9.082, 10.921, 13.592, 19.965, 21.403, and 24.207. In some embodiments, the crystal form S of the compound of formula (I) has characteristic peaks at 6.576, 9.082, 10.921, 13.592, 16.805, 19.965, 21.403, 24.207, 25.662, and 27.457. In some embodiments, the crystal form S of the compound of formula (I) has characteristic peaks at 6.576, 7.890, 9.082, 10.921, 13.592, 15.043, 16.805, 19.965, 21.403, 24.207, 25.662, 26.537, and 27.457. In some embodiments, the crystal form S of the compound of formula (I) has the X-ray powder diffraction pattern represented by the diffraction angle 2θ as shown in Figure 24. The present disclosure further provides a method for preparing the crystal form S of the compound of formula (I), comprising method 1: a) mixing the compound of formula (I) with DMF, heating to dissolve, and cooling to precipitate; b) filtering, mixing the filter cake with acetonitrile, and slurrying; c) filtering, mixing the filter cake with water, and slurrying; d) filtering, drying,

then slurrying with acetonitrile for crystallization, and drying.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form S of the compound of formula (I) according to the present disclosure further includes steps of filtering, washing, drying, etc.

The present disclosure provides a crystal form T of the compound of formula (I), which has an X-ray powder diffraction pattern, represented by a diffraction angle 2θ, having characteristic peaks at 6.915, 9.177, 9.984, 11.012, 13.595, 16.156, and 20.138. In some embodiments, the crystal form T of the compound of formula (I) has characteristic peaks at 6.915, 9.177, 9.984, 11.012, 13.595, 15.174, 16.156, 20.138, 24.261, and 26.391. In some embodiments, the crystal form T of the compound of formula (I) has characteristic peaks at 6.915, 9.177, 9.984, 11.012, 13.595, 15.174, 16.156, 18.509, 20.138, 22.954, 24.261, 26.391, and 27.514. In some embodiments, the crystal form T of the compound of formula (I) has the X-ray powder diffraction pattern represented by the diffraction angle 2θ as shown in Figure 25.

The present disclosure further provides a method for preparing the crystal form T of the compound of formula (I), comprising method 1: a) mixing the compound of formula (I) with DMF, heating to dissolve, and cooling for crystallization; b) filtering, mixing the filter cake with water and stirring; c) filtering, again mixing the filter cake with water and stirring; d) filtering, drying, then mixing with acetonitrile, stirring for crystallization, and drying.

In some embodiments, the volume (μl) of the solvent used in the present disclosure can be 1 to 200 times the mass (mg) of the compound of formula (I); in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form T of the compound of formula (I) according to the present disclosure further includes steps of filtering, washing, drying, etc.

The present disclosure further provides a pharmaceutical composition prepared from the aforementioned crystal forms of the compound of formula (I).

The present disclosure further provides a pharmaceutical composition comprising the aforementioned crystal form, and optionally a pharmaceutically acceptable carrier, a diluent, or an excipient.

The present disclosure further provides a method for preparing a pharmaceutical composition, the method comprising the step of mixing the aforementioned crystal form with a pharmaceutically acceptable carrier, a diluent, or an excipient.

The present disclosure further provides a use of the aforementioned crystal form, the aforementioned composition, or the composition prepared by the aforementioned method in the manufacture of a medicament for treating and/or preventing a CRBN protein-related disease.

The present disclosure further provides a use of the aforementioned crystal form, the aforementioned composition, or the composition prepared by the aforementioned method in the manufacture of a medicament for treating and/or preventing a cancer, an angiogenesis-related condition, pain, macular degeneration or related syndrome, a skin disease, a pulmonary disease, an asbestos-related disease, a parasitic disease, an immunodeficiency disease, a CNS disease, a CNS injury, atherosclerosis or related condition, sleep disorder or related condition, an infectious disease, hemoglobinopathy or related condition, or a TNFα-related condition; preferably, a use in the manufacture of a medicament for treating and/or preventing a cancer or a CNS injury.

In some embodiments, the cancer is selected from the group consisting of leukemia, myeloma, lymphoma, melanoma, skin cancer, liver cancer, kidney cancer, lung cancer, nasopharyngeal cancer, gastric cancer, esophageal cancer, colorectal cancer, gallbladder cancer, bile duct cancer, chorionic epithelioma, pancreatic cancer, polycythemia vera, pediatric tumor, cervical cancer, ovarian cancer, breast cancer, bladder cancer, urothelial cancer, ureteral tumor, prostate cancer, seminoma, testicular tumor, head and neck tumor, head and neck squamous cell carcinoma, endometrial cancer, thyroid cancer, sarcoma, osteoma, neuroblastoma, neuroendocrine cancer, brain tumor, CNS cancer, astrocytoma, and glioma; preferably, the liver cancer is hepatocellular carcinoma; the colorectal cancer is colon cancer or rectal cancer; the sarcoma is osteosarcoma or soft tissue sarcoma; and the glioma is glioblastoma.

In some embodiments, the myeloma is multiple myeloma (MM) and myelodysplastic syndrome (MDS); preferably, the multiple myeloma is relapsed, refractory, or resistant.

In some embodiments, the multiple myeloma is refractory or resistant to lenalidomide or pomalidomide.

The "2θ" or "2θ angle" in the present disclosure refers to the diffraction angle, where θ represents the Bragg angle with units in ° or degrees. The error range of 2θ for each characteristic peak is ± 0.20 (including cases where numbers exceeding one decimal place are rounded), and may be -0.20, -0.19, -0.18, -0.17, -0.16, -0.15, -0.14, -0.13, -0.12, -0.11, -0.10, - 0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, and 0.20.

According to the "9103 Guideline for Hygroscopicity for Pharmaceuticals" in Part IV of the 2015 edition of the Chinese Pharmacopoeia, descriptions of hygroscopic characteristics and definitions of hygroscopic weight gain are as follows:

Deliquescent: Absorbs sufficient moisture to form a liquid;

Extremely hygroscopic: Hygroscopic weight gain is no less than 15%;

Hygroscopic: Hygroscopic weight gain is less than 15% but no less than 2%;

Slightly hygroscopic: Hygroscopic weight gain is less than 2% but no less than 0.2%;

Non-hygroscopic or nearly non-hygroscopic: Hygroscopic weight gain is less than 0.2%.

The term "differential scanning calorimetry or DSC" described in the present disclosure refers to measuring the temperature difference and heat flow difference between the sample and the reference during the process of heating or constant temperature of the sample, so as to characterize all physical changes and chemical changes related to thermal effects and obtain the phase change information of the sample.

The drying temperature described in the present disclosure generally ranges from 25°C to 150°C, preferably from 40°C to 80°C. Drying can be carried out under atmospheric pressure or under reduced pressure.

"Pharmaceutical composition" refers to a mixture containing one or more than one compound of formula (I) described herein or a pharmaceutically acceptable salt thereof and other chemical components, and other components, for example, a pharmaceutically acceptable carrier and an excipient. The pharmaceutical composition is intended to promote the administration to an organism, so as to facilitate the absorption of the active ingredient, thereby exerting biological activities.

The crystal forms in the present disclosure include, but are not limited to, solvates of the compound of formula (I). The solvents include, but are not limited to, water, methanol, ethanol, isopropanol, acetone, ethyl acetate, acetonitrile, isopropyl acetate, methyl *tert*-butyl ether, 2-butanone, tetrahydrofuran, dimethyl sulfoxide, *N*-methylpyrrolidone, methyl isobutyl ketone, dichloromethane, *n*-heptane, 1,4-dioxane, nitromethane, propylene glycol methyl ether, chloroform, *N,N*-dimethylformamide, *N*,*N*-dimethylacetamide, *p*-xylene, cyclohexane, dichloroethane, *n*-hexane, petroleum ether, *n*-octane, *o*-xylene, toluene, and isopropyl ether.

The term "solvate" described in the present disclosure includes, but is not limited to, a complex formed by combining the compound of formula (I) with a solvent.

BRIEF DESCRIPTION OF THE DRAWINGS

Figure 1 is the XRPD pattern of an amorphous form of the compound of formula (I).
Figure 2 is the XRPD pattern of the crystal form A of the compound of formula (I).
Figure 3 is the XRPD pattern of the crystal form B of the compound of formula (I).
Figure 4 is the XRPD pattern of the crystal form C of the compound of formula (I).
Figure 5 is the XRPD pattern of the crystal form D of the compound of formula (I).
Figure 6 is the XRPD pattern of the crystal form E of the compound of formula (I).
Figure 7 is the XRPD pattern of the crystal form F of the compound of formula (I).
Figure 8 is the XRPD pattern of the crystal form G of the compound of formula (I).
Figure 9 is the XRPD pattern of the crystal form H of the compound of formula (I).
Figure 10 is the XRPD pattern of the crystal form I of the compound of formula (I).
Figure 11 is the XRPD pattern of the crystal form J of the compound of formula (I).
Figure 12 is the XRPD pattern of the crystal form K of the compound of formula (I).
Figure 13 is the XRPD pattern of the crystal form L of the compound of formula (I).
Figure 14 is the XRPD pattern of the crystal form M of the compound of formula (I).
Figure 15 is the XRPD pattern of the crystal form N of the compound of formula (I).
Figure 16 is the XRPD pattern of the crystal form O of the compound of formula (I).
Figure 17 is the XRPD pattern of the crystal form P of the compound of formula (I).
Figure 18 is the XRPD pattern of the crystal form Q of the compound of formula (I).
Figure 19 is the XRPD pattern of the crystal form U of the compound of formula (I).
Figure 20 is the XRPD pattern of the crystal form X of the compound of formula (I).
Figure 21 is the XRPD pattern of the crystal form Y of the compound of formula (I).
Figure 22 is the XRPD pattern of the crystal form V of the compound of formula (I).
Figure 23 is the XRPD pattern of the crystal form R of the compound of formula (I).
Figure 24 is the XRPD pattern of the crystal form S of the compound of formula (I).
Figure 25 is the XRPD pattern of the crystal form T of the compound of formula (I).
Figure 26: The efficacy data of the compound of Example 1 and the control example CC-92480 on NCI-H929 xenograft tumor in CB-17 SCID mice *in vivo.*
Figure 27: Effects of the compound of Example 1 and the control example CC-92480 on the body weight of CB-17 SCID mice.

DETAILED DESCRIPTION OF THE PREFERRED EMBODIMENT

The present disclosure will be explained in more detail below with reference to examples or experimental examples. The examples or experimental examples of the present disclosure are only used to illustrate the technical solutions of the present disclosure and do not limit the essence and scope of the present disclosure.

Experimental methods without specifying specific conditions in the examples of the present disclosure are generally in accordance with conventional conditions, or in accordance with the conditions recommended by the manufacturers of the raw materials or commercial products. Reagents without specifying a particular source are conventional reagents purchased from the market.

The structure of the compound was determined by nuclear magnetic resonance (NMR) spectroscopy and/or mass spectrometry (MS). NMR shift ($\delta$) is given in a unit of $10^{-6}$ (ppm). NMR spectra were determined using a Bruker AVANCE-400 nuclear magnetic resonance instrument, with deuterated dimethyl sulfoxide (DMSO-$d_6$), deuterated chloroform ($CDCl_3$), and deuterated methanol ($CD_3OD$) as determination solvents and tetramethylsilane (TMS) as an internal standard.

Mass spectra were determined using Agilent 1200/1290 DAD- 6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters, MS model: Waters ACQuity Qda Detector/Waters SQ Detector), and THERMO Ultimate 3000-Q Exactive (manufacturer: THERMO, MS model: THERMO Q Exactive).

High performance liquid chromatography (HPLC) analysis was performed using Agilent HPLC 1200DAD, Agilent HPLC 1200VWD, and Waters HPLC e2695-2489 high performance liquid chromatographs.

Chiral HPLC analysis was performed on Agilent 1260 DAD high performance liquid chromatograph.

HPLC preparation was performed using Waters 2767, Waters 2767-SQ Detecor2, Shimadzu LC-20AP, and Gilson-281 preparative chromatographs.

Chiral preparation was performed on a Shimadzu LC-20AP preparative chromatograph.

A CombiFlash rapid preparation instrument used was Combiflash Rf200 (TELEDYNE ISCO).

Yantai Huanghai HSGF254 or Qingdao GF254 silica gel plates of specifications 0.15 mm to 0.2 mm were adopted for thin layer chromatography (TLC) analysis and 0.4 mm to 0.5 mm for TLC separation and purification.

Yantai Huanghai silica gel of 200 to 300-mesh was generally used as a carrier in silica gel column chromatography.

The mean inhibition of kinase and the $IC_{50}$ value were determined using a NovoStar microplate reader (BMG, Germany).

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can be performed in an argon atmosphere or a nitrogen atmosphere unless otherwise specified.

The argon atmosphere or nitrogen atmosphere means that the reaction flask is connected to a balloon containing about 1 L of argon or nitrogen.

A hydrogen atmosphere means that the reaction flask is connected to a balloon containing about 1 L of hydrogen.

Parr 3916EKX hydrogenator, Qinglan QL-500 hydrogenator, or HC2-SS hydrogenator was used in the pressurized hydrogenation reactions.

The hydrogenation reactions usually involve 3 cycles of vacuumization and hydrogen purge.

A CEM Discover-S 908860 microwave reactor was used in the microwave reactions.

In the examples, a solution refers to an aqueous solution unless otherwise specified.

In the examples, the reaction temperature was room temperature, *i.e.*, 20°C to 30°C, unless otherwise specified.

The monitoring of the reaction progress in the examples was conducted by thin layer chromatography (TLC). The developing solvent for reactions, the eluent system for column chromatography purification and the developing solvent system for thin layer chromatography included: A: n-hexane/ethyl acetate system, and B: dichloromethane/methanol system. The volume ratio of the solvents was adjusted according to the polarity of the compound, or by adding a small amount of basic or acidic reagents such as triethylamine and acetic acid.

Test conditions for the instruments used in the experiments described in the present disclosure are as follows:

1. Differential Scanning Calorimeter (DSC)
Instrument model: Mettler Toledo DSC 3+ STARe System
Purge gas: Nitrogen; Nitrogen flow rate: 50 mL/min
Heating rate: 10.0°C/min
Temperature range: 25 to 350°C
2. X-ray Powder Diffraction (XRPD)

Instrument model: BRUKER D8 Discover X-ray Powder Diffractometer
Radiation: Monochromatic Cu-Kα radiation (λ = 1.5418 Å)
Scanning method: θ/2θ, Scanning range (2θ range): 3 to 50°
Voltage: 40 kV, Current: 40 mA
3. Thermogravimetric Analysis (TGA)
Instrument model: Mettler Toledo TGA2
Purge gas: Nitrogen; Nitrogen flow rate: 50 mL/min
Heating rate: 10.0°C/min
Temperature range: 25 to 350°C

**Example 1: Preparation of Compound of Formula (I)**

(*S*)-4-(4-((5-(((2-(2,6-dioxopiperidin-3-yl)-1-oxoisoindolin-4-yl)oxy)methyl)pyridin-2-yl)thio)piperidin-1-yl)-3-fluorobenzonitrile (compound 1)

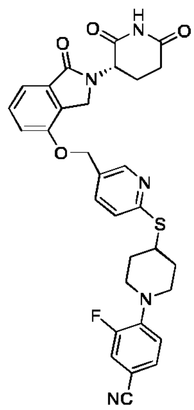

1

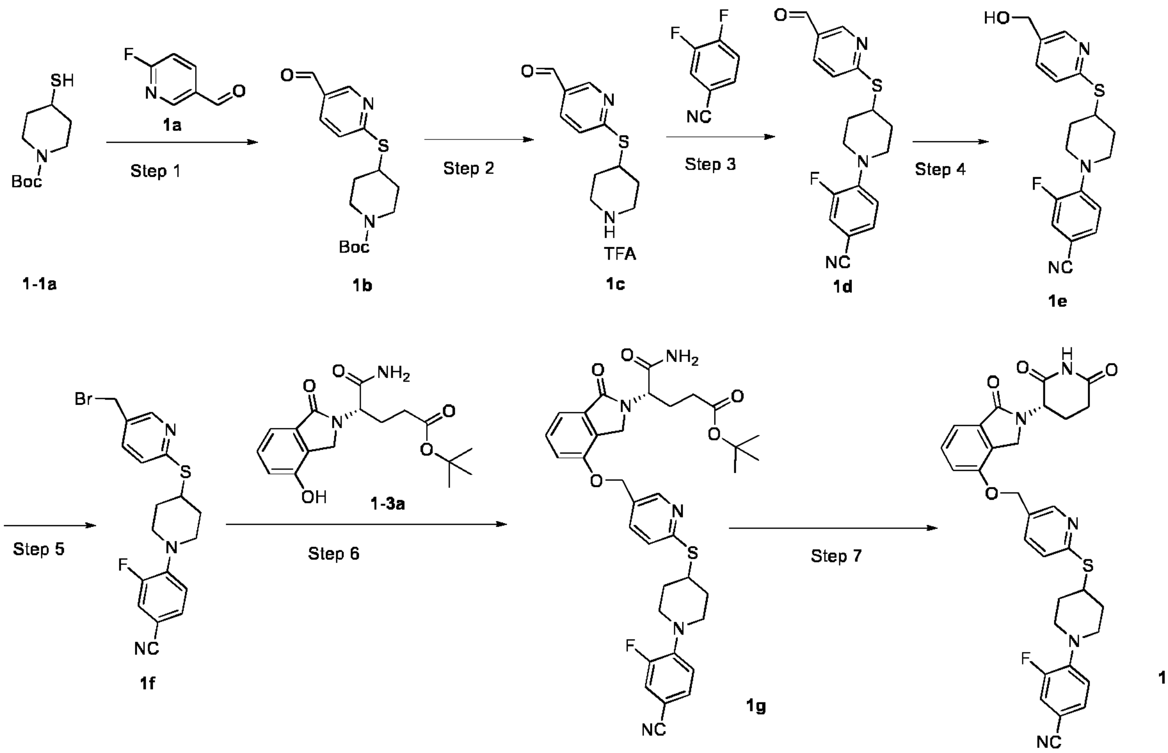

Step 1

tert-Butyl 4-((5-formylpyridin-2-yl)thio)piperidine-1-carboxylate **1b**

Compound **1-1a** (700 mg, 3.22 mmol), 6-fluoropyridine-3-carboxaldehyde **1a** (443 mg, 3.54 mmol), and potassium carbonate (1.11 g, 8.05 mmol) were added to *N,N*-dimethylformamide (10 mL). The reaction mixture was heated to 80°C and reacted for 1 hour. The reaction mixture was diluted with water (30 mL) and extracted with ethyl acetate (40 mL × 3). The organic phases were combined, washed with a saturated sodium chloride solution (100 mL), dried over anhydrous sodium sulfate, and filtered, and the filtrate was concentrated under reduced pressure. The residue was purified by column chromatography with an eluent system B to give the title compound **1b** (1.0 g, yield: 96%).
MS m/z (ESI): 267.1 [M-55].

Step 2

6-(Piperidin-4-ylthio)nicotinaldehyde trifluoroacetate **1c**

Compound **1b** (950 mg, 2.95 mmol) was dissolved in dichloromethane (10 mL), and trifluoroacetic acid (2 mL) was slowly added thereto under an ice bath. The reaction mixture was reacted for 1 hour. The reaction mixture was concentrated and dried to give the title compound **1c** (, and the crude product was directly used in the next step without purification.
MS m/z (ESI): 223.1 [M+1].

Step 3

3-Fluoro-4-(4-((5-formylpyridin-2-yl)thio)piperidin-1-yl)benzonitrile **1d**

Compound **1c** (760 mg, 2.94 mmol), 3,4-difluorobenzonitrile (817 mg, 5.87 mmol), and potassium carbonate (1.22 g, 8.81 mmol) were added to *N,N*-dimethylformamide (15 mL), and the reaction mixture was heated to 80°C and reacted overnight. The reaction mixture was added with water (50 mL) and extracted with ethyl acetate (50 mL × 3). The organic phases were combined, washed with a saturated sodium chloride solution (50 mL × 3), dried over anhydrous sodium sulfate, and filtered, and the filtrate was concentrated under reduced pressure. The resulting residue was purified by column chromatography with an eluent system B to give the title compound **1d** (850 mg, yield: 84%).
MS m/z (ESI): 342.1 [M+1].

Step 4

3-Fluoro-4-(4-((5-(hydroxymethyl)pyridin-2-yl)thio)piperidin-1-yl)benzonitrile **1e**

Compound **1d** (600 mg, 1.76 mmol) was added to methanol (10 mL) under an ice bath, followed by the slow addition of sodium borohydride (133 mg, 3.51 mmol), and the reaction mixture was reacted for 1 hour. The reaction was quenched with water (10 mL), and the reaction mixture was extracted with ethyl acetate (30 mL × 3). The organic phases were combined, washed with a saturated sodium chloride solution (30 mL), dried over anhydrous sodium sulfate, and filtered, and the filtrate was concentrated under reduced pressure. The residue was purified by column chromatography with an eluent system B to give the title compound **1e** (580 mg, yield: 96%).
MS m/z (ESI): 344.1 [M+1].

Step 5

4-(4-((5-(Bromomethyl)pyridin-2-yl)thio)piperidin-1 -yl)-3 -fluorobenzonitrile **1f**

Compound **1e** (200 mg, 0.582 mmol) was added to dichloromethane (6 mL), followed by the sequential addition of triphenylphosphine (199 mg, 0.757 mmol) and carbon tetrabromide (251 mg, 0.757 mmol), and the reaction mixture was reacted for 2 hours. The reaction mixture was concentrated. The residue was purified by column chromatography with an eluent system B to give the title compound **1f** (190 mg, yield: 80%).
MS m/z (ESI): 406.0 [M+1]; 408.0 [M+3].

Step 6

*tert*-Butyl (*S*)-5-amino-4-(4-((6-((1-(4-cyano-2-fluorophenyl)piperidin-4-yl)thio)pyridin-3-yl)methoxy)-1-oxoisoindolin-2-yl)-5-oxopentanoate **1g**

*tert*-Butyl (*S*)-5-amino-4-(4-hydroxy-1-oxoisoindolin-2-yl)-5-oxopentanoate **1-3a** (82 mg, 0.246 mmol, prepared using the well-known method described in "Journal of Medicinal Chemistry", 2020, 63 (13), 6648-6676) and anhydrous potassium carbonate (65 mg, 0.468 mmol) were added to *N,N*-dimethylformamide (3 mL), followed by the addition of compound **1f** (95 mg, 0.244 mmol), and the reaction mixture was reacted for 2 hours. The reaction mixture was poured into ice water (10 mL), and the resulting mixture was extracted with ethyl acetate (30 mL × 3). The organic phases were combined, washed with a saturated sodium chloride solution (20 mL × 2), dried over anhydrous sodium sulfate, and filtered, and the filtrate was concentrated under reduced pressure. The residue was purified by column chromatography with an eluent system B to give the title compound **1g** (145 mg, yield: 94%).

MS m/z (ESI): 660.2 [M+1],

Step 7

(*S*)-4-(4-((5-(((2-(2,6-Dioxopiperidin-3-yl)-1-oxoisoindolin-4-yl)oxy)methyl)pyridin-2-yl)thio)piperidin-1-yl)-3-fluorobenzonitrile 1

Compound **1g** (60 mg, 0.091 mmol) was added to acetonitrile (5 mL), followed by the addition of benzenesulfonic acid (16 mg, 0.091 mmol) at room temperature, and the reaction mixture was reacted at 80°C for 8 hours. The reaction mixture was concentrated under reduced pressure to remove the solvent. The resulting residue was subjected to high performance liquid chromatography (Gilson GX-281, elution system: 10 mmol/L aqueous ammonium bicarbonate solution and acetonitrile, gradient of acetonitrile: 60%-80%, flow rate: 30 mL/min) to give the title compound 1 (42 mg, yield: 78%).

Upon detection using an X-ray powder diffractometer, the title compound 1 was detected as amorphous.

MS m/z (ESI): 586.4 [M+1].

$^1$H NMR (500 MHz, DMSO-*d6*): δ 10.98 (s, 1H), 8.60 (s, 1H), 7.78 (dd, 1H), 7.69 (dd, 1H), 7.59-7.48 (m, 2H), 7.38-7.33 (m, 3H), 7.16 (t, 1H), 5.24 (s, 2H), 5.12 (dd, 1H), 4.42 (d, 1H), 4.26 (d, 1H), 4.07-3.99 (m, 1H), 3.56-3.48 (m, 2H), 3.13-3.03 (m, 2H), 2.96-2.87 (m, 1H), 2.63-2.55 (m, 1H), 2.46-2.38 (m, 1H), 2.21-2.12 (m, 2H), 2.02-1.94 (m, 1H), 1.82-1.70 (m, 2H).

Biological Evaluation

Test Example 1. Biological Evaluation of NCI-H929 Proliferation Experiment

The following method was used to determine the inhibitory activity of the compounds of the present disclosure on the proliferation of NCI-H929 cells. The experimental method was briefly described as follows.

NCI-H929 cells (ATCC, CRL-9068) were cultured in a complete medium (RPMI 1640 medium (Hyclone, SH30809.01) containing 10% fetal bovine serum (Corning, 35-076-CV) and 0.05 mM 2-mercaptoethanol (Sigma, M3148)). On the first day of the experiment, NCI-H929 cells were seeded in a 96-well plate at a density of 6000 cells/well with a complete medium to form 100 μL of cell suspension per well, and 10 μL of test compound prepared in a complete medium and diluted in a gradient was added to each well. The compound was firstly dissolved in DMSO, with an initial concentration of 10 mM, and then subjected to serial dilution at a 5-fold concentration gradient for a total of 9 concentration points, with a blank control of 100% DMSO. Another 5 μL of the compound dissolved in DMSO was added to 95 μL of complete medium, i.e., the compound was diluted 20-fold with the complete medium. Finally the compound diluted in the complete medium at 10 μL/well was added to the cell suspension, wherein the final concentrations of the compound were 9 concentration points obtained by 5-fold gradient dilution starting from 50 μM. A blank control containing 0.5% DMSO was set. The plate was incubated in a cell incubator at 37°C with 5% $CO_2$ for 5 days. On the sixth day, the 96-well cell culture plate was taken out, added with a CellTiter-Glo® luminescent cell activity detect reagent (Promega, G7573) at 50 μL/well, left to stand at room temperature for 10 min, and read for the luminescence signal values using a multi-mode microplate reader (PerkinElmer, EnVision 2015). $IC_{50}$ values for the inhibitory activity of the compounds were calculated using Graphpad Prism software, with the results shown in Table I.

Table I. $IC_{50}$ values for the inhibition of proliferation of NCI-H929 cells by the compounds of the present disclosure

| Compound | $IC_{50}$ (nM) |
|---|---|
| 1 | 0.02 |

Conclusion: Compound 1 of the present disclosure has good inhibitory activity for the proliferation of NCI-H929 cells.

Test Example 2. Pharmacodynamic Test

1. Objective

This experiment was performed to evaluate the inhibition effect of the compound of Example 1 and the control example CC-92480 on the growth of human multiple myeloma cell NCI-H929 xenograft tumor in CB-17 SCID mice.

2. Test Compounds

Compound of Example 1;
control example CC-92480 (see compound 2 of WO2019014100A1, synthesized according to the method disclosed therein)

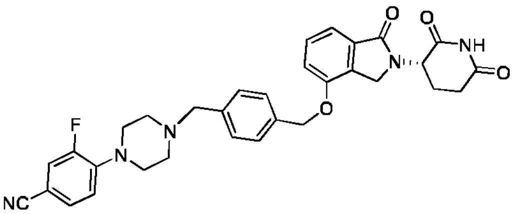

;

Solutions of the compound of Example 1 and the control example CC-92480 were prepared with 5% DMSO + 20% PEG400 + 70% (10% TPGS) + 5% (1% HPMC K100LV).

3. Experimental Procedures and Materials

3.1 Experimental Animals and Housing Conditions

Thirty CB-17 SCID female mice, purchased from Beijing Vital River Laboratory Animal Technology Co., Ltd. (certificate No. 20170011006049, SCXK (Shanghai) 2017-0011), weighing about 19 g at the time of purchase, were bred at 5 mice/cage (in a 12/12 hour light/dark cycle, at a constant temperature of 23 ± 1°C and humidity of 50%-60%) and had free access to food and water.

3.2 Group of Animals:

After adaptive feeding, the CB-17 SCID mice were grouped as follows:

| Group | n | Route of administration |
|---|---|---|
| Vehicle control | 7 | 5% DMSO + 20% PEG400 + 70% (10% TPGS) + 5% (1%HPMC K100LV) (i.g/qd) |
| Example 1 | 7 | 1 mg/kg (i.g/qd) |
| CC-92480 | 7 | 1 mg/kg (i.g/qd) |
| Note: qd denotes once a day; i.g. denotes intragastric administration. | | |

3.3. Procedures:

NCI-H929 cells in a logarithmic growth phase were inoculated subcutaneously into the right flank of 30 female

CB-17 SCID mice at 5 × $10^6$ cells/mouse/100 μL (containing 50 μL of Matrigel). After 10 days, when the tumor volume of the tumor-bearing mice reached about 200 $mm^3$, the mice were randomly divided into 3 groups according to the tumor volume and body weight: vehicle control group, CC-92480-1 mpk, compound of Example 1-1 mpk, with 7 mice in each group. The day of grouping was set as Day 0 (D0), and intragastric administration was performed once a day for 11 days (Table 2). For the tumor-bearing mice, the tumor volume was measured with a vernier caliper and the body weight was measured with a balance twice a week, and the data were recorded. The tumor-bearing animals were euthanized as the experimental endpoint when the tumor volume reached 2000 $mm^3$ or when most tumors showed rupture or when the tumor-bearing animals showed 20% of body weight loss.

3.4 Data Statistics

All data were plotted and statistically analyzed using Excel and GraphPad Prism 5 software.

The tumor volume (V) was calculated as follows: V = 1/2 × a × $b^2$, where a and b represent length and width, respectively.

The relative tumor proliferation rate T/C (%) = ($T-T_0$) / ($C-C_0$) × 100 (%), where T and C are the tumor volume of animals at the end of the experiment in the treatment group and control group, respectively; $T_0$ and $C_0$ are the tumor volume of animals at the beginning of the experiment in the treatment group and control group, respectively.

Tumor growth inhibition TGI (%) = 1 - T/C (%), and when TGI (%) exceeds 100%, no specific value will be shown, and it is expressed only by >100%.

$$\text{Tumor regression } (\%) = [(T_0 - T) / T_0] \times 100\ (\%)$$

4. Results

The data on the efficacy of the compound of Example 1 and the control example CC-92480 on NCI-H929 xenograft tumor in CB-17 SCID mice are shown in Table II below and Figure 26.

The effect of the compound of Example 1 and the control example CC-92480 on the body weight of CB-17 SCID mice is shown in Figure 27.

Table II Efficacy of the compound of the present disclosure on NCI-H929 xenograft tumor in CB-17 SCID mice

| Group | Route of administration | | Mean tumor volume ($mm^3$) | | | | % Tumor regression D11 | p (v.s. Vehicle control) | Number of remaining animals/ group |
|---|---|---|---|---|---|---|---|---|---|
| | | | D0 | SEM | D11 | SEM | | | |
| Vehicle control | qd/11d | po | 156.3 | 15.9 | 1711. 7 | 190.1 | / | / | 7/7 |
| Example 1 | qd/11d | po | 155.4 | 16.5 | 18.6 | 1.8 | 88 | < 0.001 | 7/7 |
| CC-92480 | qd/11d | po | 157.2 | 13.6 | 103.6 | 27.1 | 34 | < 0.001 | 7/7 |
| Note: qd denotes once a day; po denotes oral administration. | | | | | | | | | |

5. Conclusion

The compound of Example 1 was administered once a day starting from 10 days after tumor cell transplantation, and significant tumor volume regressions occurred 11 days after administration. Upon calculation, the tumor inhibition rate is > 100%, and the tumor regression rate is 88%. The statistical difference ($p < 0.05$) is obtained when compared with an equal dose of CC-92480 at the end point of the experiment, and the administration has no influence on the body weight of mice. Under the same conditions, the tumor regression rate of the control example CC-92480 is 34%.

Test Example 3. Pharmacokinetic Evaluation

1. Overview

The drug concentration in the plasma of the test animals (mice) at different time points after intragastric administration of the compound of Example 1 and the control example CC-92480 was determined by an LC/MS/MS

method. The pharmacokinetic behavior of the compound of the present disclosure in mice was studied and its pharmacokinetic profile was evaluated.

2. Test Scheme

2.1. Test Compounds

Compound of Example 1 and control example CC-92480.

2.2. Test Animals

Eighteen mice, female, purchased from Beijing Vital River Laboratory Animal Technology Co., Ltd., with animal production license number of SCXK (Shanghai) 2017-0005.

2.3. Drug Preparation

A certain amount of compound of Example 1 was weighed, dissolved by adding 5% by volume of DMSO and 5% Tween 80 (Shanghai Titan Scientific Co., Ltd.), and then prepared into a 0.1 mg/mL clear solution by adding 90% normal saline.

A certain amount of the control example CC-92480 was weighed, dissolved by adding 5% by volume of DMSO and 5% Tween 80 (Shanghai Titan Scientific Co., Ltd.), and then prepared into a 0.1 mg/mL clear solution by adding 90% normal saline.

2.4. Administration

Nine mice were intragastrically administered with the compound of Example 1 at a dose of 2 mg/kg and at a volume of 0.2 mL/10 g.

Nine mice were intragastrically administered with the control example CC-92480 at a dose of 2 mg/kg and at a volume of 0.2 mL/10 g.

3. Procedures

Mice were intragastrically administered with the compound of Example 1 and the control example CC-92480, and 0.2 mL of blood was collected before the administration and 0.25 h, 0.5 h, 1.0 h, 2.0 h, 4.0 h, 6.0 h, 8.0 h, 11.0 h and 24.0 h after the administration (3 animals at each time point), placed in EDTA-K2 anticoagulation tubes, and centrifuged at 10,000 rpm for 1 min (4°C). Plasma was separated within 1 hour, and stored at -20°C for testing. The process from blood sampling to centrifugation was performed under an ice bath.

The content of the test compounds at different concentrations in mouse plasma after the administration was determined: 25 μL of mouse plasma at each time point after the administration was mixed with 50 μL (100 ng/mL) of internal standard solution camptothecin (National Institutes for Drug Control) and 175 μL of acetonitrile. The mixture was vortexed for 5 min and centrifuged for 10 min (3700 rpm), and 1 μL of the supernatant of the plasma sample was taken for LC/MS/MS assay (API4000 triple quadrupole tandem mass spectrometer, Applied Biosystems, USA; Shimadzu, LC-30AD ultra high performance liquid chromatography system, Shimadzu, Japan).

4. Pharmacokinetic Parameter Results

Pharmacokinetic parameters for the compound of the present disclosure are shown in Table III below.

Table III Pharmacokinetic parameters of the compound of the present disclosure

| No. | Pharmacokinetic experiment (2 mg/kg) | | | | | |
|---|---|---|---|---|---|---|
| | Plasma concentration | Area under curve | Half-life | Residence time | Clearance rate | Apparent volume of distribution |
| | Cmax (ng/mL) | AUC (ng /mL * h) | T1/2 (h) | MRT (h) | CL/F (mL/min/kg) | Vz/F (ml/kg) |
| Example 1 | 799 | 4686 | 2.9 | 4.6 | 7.1 | 1785 |

(continued)

| No. | Pharmacokinetic experiment (2 mg/kg) | | | | | |
|---|---|---|---|---|---|---|
| | Plasma concentration | Area under curve | Half-life | Residence time | Clearance rate | Apparent volume of distribution |
| | Cmax (ng/mL) | AUC (ng /mL * h) | T1/2 (h) | MRT (h) | CL/F (mL/min/kg) | Vz/F (ml/kg) |
| CC-92480 | 692 | 1937 | 1.3 | 2.3 | 17.2 | 1961 |

Conclusion: The compound of the present disclosure demonstrates good absorption profile and has significant pharmacokinetic advantages.

Test Example 4. Evaluation of Plasma Stability of the Compound of the Present Disclosure

1. Abstract

The stability of the compound of Example 1 and the control example CC-92480 after incubation in cryopreserved monkey plasma at 37°C for 0 min, 15 min, 30 min, 60 min, 120 min, 180 min and 240 min was determined quantitatively by LC-MS/MS.

2. Test Protocol

2.1. Test Compounds

Compound of Example 1 and control example CC-92480.

2.2 Test Plasma

Monkey plasma was purchased from Shanghai Medicilon Inc.

2.3 Preparation of Compound Solutions

A certain amount of the compound of Example 1 was weighed and prepared with DMSO to obtain a 30 mM stock solution. A certain volume of the stock solution was diluted into a solution I at a concentration of 1600 μM with DMSO; and a certain volume of the solution I at 1600 μM was diluted with 50% methanol to obtain a working solution II at a concentration of 16 μM. A30 mM stock solution, a 1600 μM solution I', and a 16 μM working solution II' of CC-92480 were prepared as described above.

2.4 Sample Incubation

5 μL of the working solutions at 16 μM of the compound of Example 1 and the control example CC-92480 were each added to 75 μL of plasma to make the final concentration of the compounds of 1 μM. The samples were incubated under a 37°C water bath for 0 min, 15 min, 30 min, 60 min, 90 min, 120 min, and 180 min. After the incubation, 240 μL of acetonitrile containing the internal standard was added, and the plate was shaken on a shaker at 800 rpm for 10 min and centrifuged on a centrifugation at 4°C at 3700 rpm for 20 min. The supernatant was analyzed by LC-MS with a sample injection volume of 2 μL.

3. Results

The conversion of the compound of the present disclosure in monkey plasma is shown in Table IV below.

Table IV Stability data of the compound of the present disclosure in monkey plasma

| No. | $T_{1/2}$ (min)/monkey |
|---|---|
| Example 1 | 806 |
| CC-92480 | 199 |

Conclusion: the compound of the present disclosure has stability advantages in monkey plasma.

**Example 2: Preparation of Amorphous Form of Compound of Formula (I)**

Compound of formula (I) (10 mg) was added to propylene glycol methyl ether (1.0 mL), slurried at room temperature, and centrifuged. The solid was dried to obtain the product. The product was detected as amorphous form by an X-ray powder diffractometer and its XRPD pattern is shown in Figure 1.

**Example 3: Preparation of Crystal Form A of Compound of Formula (I)**

Compound of formula (I) (7.3 g) was added to dichloromethane (300 mL), dissolved with methanol (30 mL), and water (300 mL) was added thereto. The reaction mixture was subjected to rotary evaporation to remove the majority of the dichloromethane and to precipitate a large amount of solid, filtered and the solid was obtained and then dried overnight. The solid was dissolved in dichloromethane (500 mL), filtered under reduced pressure to obtain the filtrate (500 mL), which was dried over anhydrous sodium sulfate, filtered, and ethyl acetate (200 mL) was added. The mixture was dried under rotary evaporation to obtain the product. After X-ray powder diffraction detection, the product was defined as crystal form A. The XRPD pattern is shown in Figure 2, and its characteristic peak positions are shown in Table 1. DSC pattern showed endothermic peaks at 155.26°C, 183.19°C, and 244.74°C. TGApattern showed a weight loss of 9.48% from 25°C to 220°C.

Table 1

| Peak No. | 2θ value [° or degree] | d[Å] | Relative intensity% |
|---|---|---|---|
| 1 | 5.765 | 15.31661 | 67.0 |
| 2 | 7.465 | 11.83234 | 40.7 |
| 3 | 8.061 | 10.95880 | 100.0 |
| 4 | 9.925 | 8.90513 | 80.2 |
| 5 | 11.674 | 7.57437 | 30.1 |
| 6 | 12.890 | 6.86264 | 36.5 |
| 7 | 14.270 | 6.20150 | 17.7 |
| 8 | 15.085 | 5.86862 | 31.7 |
| 9 | 16.632 | 5.32575 | 48.4 |
| 10 | 17.900 | 4.95150 | 56.1 |
| 11 | 18.715 | 4.73745 | 23.5 |
| 12 | 19.469 | 4.55574 | 88.1 |
| 13 | 19.860 | 4.46700 | 43.8 |
| 14 | 20.696 | 4.28837 | 36.6 |
| 15 | 21.115 | 4.20425 | 58.4 |
| 16 | 21.708 | 4.09064 | 22.9 |
| 17 | 24.393 | 3.64618 | 12.3 |
| 18 | 25.669 | 3.46771 | 14.1 |

**Example 4: Preparation of Crystal Form A of Compound of Formula (I)**

Compound of formula (I) (10 mg) was added to water (1 mL), slurried at room temperature, and centrifuged. The solid was dried under vacuum to obtain the product. After X-ray powder diffraction detection, the product was defined as crystal form A.

**Example 5: Preparation of Crystal Form A of Compound of Formula (I)**

Compound of formula (I) (10 mg) was added to acetone (1 mL), slurried, and centrifuged. The solid was dried

under vacuum to obtain the product. After X-ray powder diffraction detection, the product was defined as crystal form A.

**Example 6: Preparation of Crystal Form A of Compound of Formula (I)**

Compound of formula (I) (10 mg) was added to THF (300 uL), dissolved to clarification, then *n*-heptane (450 uL) was added thereto to precipitate a solid. The mixture was slurried at room temperature and centrifuged. The solid was dried under vacuum to obtain the product. After X-ray powder diffraction detection, the product was defined as crystal form A.

**Example 7: Preparation of Crystal Form B of Compound of Formula (I)**

Compound of formula (I) (150 mg) was added to acetonitrile/methanol (1.0 mL, V/V = 1: 1), slurried, and centrifuged. The solid was dried under vacuum to obtain the product. After X-ray powder diffraction detection, the product was defined as crystal form B. The XRPD pattern is shown in Figure 3, and its characteristic peak positions are shown in Table 2. DSC pattern showed endothermic peaks at 182.74°C and 245.37°C. TGA pattern showed a weight loss of 4.57% from 25°C to 225°C.

Table 2

| Peak No. | 2θ value [° or degree] | d[Å] | Relative intensity (%) |
|---|---|---|---|
| 1 | 4.977 | 17.74069 | 51.6 |
| 2 | 6.788 | 13.01194 | 59.7 |
| 3 | 10.047 | 8.79717 | 59.1 |
| 4 | 10.972 | 8.05760 | 47.9 |
| 5 | 14.143 | 6.25730 | 9.8 |
| 6 | 15.684 | 5.64563 | 23.7 |
| 7 | 18.547 | 4.78018 | 37.6 |
| 8 | 20.088 | 4.41673 | 65.3 |
| 9 | 20.840 | 4.25906 | 100.0 |
| 10 | 21.982 | 4.04031 | 10.9 |
| 11 | 22.907 | 3.87923 | 5.8 |
| 12 | 24.096 | 3.69042 | 4.8 |
| 13 | 25.505 | 3.48961 | 2.5 |

**Example 8: Preparation of Crystal Form C of Compound of Formula (I)**

Compound of formula (I) (10 mg) was added to ethanol (1.0 mL), slurried at room temperature, and centrifuged. The solid was dried under vacuum to obtain the product. After X-ray powder diffraction detection, the product was defined as crystal form C. The XRPD pattern is shown in Figure 4, and its characteristic peak positions are shown in Table 3. DSC pattern showed endothermic peaks at 153.48°C, 182.76°C, and 244.05°C. TGApattern showed a weight loss of 3.00% from 25°C to 215°C.

Table 3

| Peak No. | 2θ value [° or degree] | d[Å] | Relative intensity (%) |
|---|---|---|---|
| 1 | 3.533 | 24.98692 | 29.6 |
| 2 | 5.653 | 15.62173 | 51.4 |
| 3 | 7.608 | 11.61088 | 44.3 |
| 4 | 7.974 | 11.07924 | 81.3 |
| 5 | 8.790 | 10.05224 | 20.9 |
| 6 | 9.989 | 8.84772 | 89. |

(continued)

| Peak No. | 2θ value [° or degree] | d[Å] | Relative intensity (%) |
|---|---|---|---|
| 7 | 11.505 | 7.68553 | 45.7 |
| 8 | 12.493 | 7.07950 | 18.7 |
| 9 | 12.798 | 6.91147 | 37.1 |
| 10 | 14.265 | 6.20385 | 38.7 |
| 11 | 15.277 | 5.79513 | 32.5 |
| 12 | 16.143 | 5.48617 | 54.5 |
| 13 | 17.860 | 4.96247 | 57.2 |
| 14 | 18.992 | 4.66919 | 100.0 |
| 15 | 19.943 | 4.44843 | 17.1 |
| 16 | 20.972 | 4.23258 | 50.1 |
| 17 | 21.773 | 4.07853 | 19.0 |
| 18 | 23.995 | 3.70564 | 8.9 |
| 19 | 25.912 | 3.43567 | 12.1 |
| 20 | 26.958 | 3.30473 | 8.0 |

**Example 9: Preparation of Crystal Form C of Compound of Formula (I)**

Compound of formula (I) (10 mg) was added to 10% water/acetone (0.9 mL). The mixture was volatilized and crystallized to obtain the product. After X-ray powder diffraction detection, the product was defined as crystal form C.

**Example 10: Preparation of Crystal Form D of Compound of Formula (I)**

Compound of formula (I) (10 mg) was added to acetonitrile (1.0 mL), slurried, and centrifuged. The solid was dried at 30°C under vacuum to obtain the product. After X-ray powder diffraction detection, the product was defined as crystal form D. The XRPD pattern is shown in Figure 5, and its characteristic peak positions are shown in Table 4. DSC pattern showed endothermic peaks at 156.12°C and 245.38°C. TGApattern showed a weight loss of 1.17% from 25°C to 205°C.

Table 4

| Peak No. | 2θ value [° or degree] | d[Å] | Relative intensity (%) |
|---|---|---|---|
| 1 | 6.831 | 12.92916 | 88.1 |
| 2 | 9.845 | 8.97708 | 59.0 |
| 3 | 10.927 | 8.09061 | 14.6 |
| 4 | 13.453 | 6.57630 | 35.9 |
| 5 | 13.861 | 6.38376 | 32.7 |
| 6 | 16.096 | 5.50187 | 13.2 |
| 7 | 18.225 | 4.86376 | 14.8 |
| 8 | 20.117 | 4.41049 | 56.5 |
| 9 | 20.891 | 4.24885 | 100.0 |
| 10 | 23.006 | 3.86268 | 18.4 |
| 11 | 24.829 | 3.58314 | 2.0 |
| 12 | 26.132 | 3.40731 | 11.4 |
| 13 | 27.001 | 3.29960 | 3.5 |

(continued)

| Peak No. | 2θ value [° or degree] | d[Å] | Relative intensity (%) |
|---|---|---|---|
| 14 | 27.913 | 3.19380 | 6.2 |

**Example 11: Preparation of Crystal Form E of Compound of Formula (I)**

Compound of formula (I) (10 mg) was added to 10% water/methanol (1.0 mL), slurried, and centrifuged. The solid was dried under vacuum to obtain the product. After X-ray powder diffraction detection, the product was defined as crystal form E. The XRPD pattern is shown in Figure 6, and its characteristic peak positions are shown in Table 5. DSC pattern showed endothermic peaks at 182.71°C and 245.00°C. TGApattern showed a weight loss of 0.68% from 25°C to 235°C.

Table 5

| Peak No. | 2θ value [° or degree] | d[Å] | Relative intensity (%) |
|---|---|---|---|
| 1 | 5.864 | 15.05957 | 59.3 |
| 2 | 7.573 | 11.66418 | 100.0 |
| 3 | 8.087 | 10.92345 | 81.0 |
| 4 | 10.003 | 8.83545 | 56.3 |
| 5 | 11.701 | 7.55682 | 37.2 |
| 6 | 12.471 | 7.09202 | 56.2 |
| 7 | 12.941 | 6.83526 | 44.4 |
| 8 | 15.165 | 5.83754 | 52.5 |
| 9 | 16.444 | 5.38629 | 68.9 |
| 10 | 17.432 | 5.08326 | 40.7 |
| 11 | 17.860 | 4.96248 | 17.7 |
| 12 | 19.349 | 4.58380 | 95.4 |
| 13 | 19.998 | 4.43642 | 18.5 |
| 14 | 20.553 | 4.31787 | 86.9 |
| 15 | 21.067 | 4.21361 | 22.4 |
| 16 | 21.709 | 4.09053 | 17.2 |
| 17 | 23.334 | 3.80918 | 6.6 |
| 18 | 24.189 | 3.67638 | 3.1 |
| 19 | 25.772 | 3.45413 | 12.8 |
| 20 | 27.568 | 3.23301 | 13.0 |

**Example 12: Preparation of Crystal Form E of Compound of Formula (I)**

Compound of formula (I) (10 mg) was added to DCM (300 uL), dissolved to clarification, then *n*-heptane (300 uL) was added thereto to precipitate a solid. The mixture was slurried at room temperature and centrifuged. The solid was dried under vacuum to obtain the product. After X-ray powder diffraction detection, the product was defined as crystal form E.

**Example 13: Preparation of Crystal Form F of Compound of Formula (I)**

Compound of formula (I) (10 mg) was added to methanol (1.0 mL), slurried at room temperature, and centrifuged. The solid was dried under vacuum to obtain the product. After X-ray powder diffraction detection, the product was defined as crystal form F. The XRPD pattern is shown in Figure 7, and its characteristic peak positions are shown in Table 6. DSC pattern showed endothermic peaks at 181.22°C and 245.03°C. TGA pattern showed a weight loss of 8.63% from 25°C to 210°C.

Table 6

| Peak No. | 2θ value [° or degree] | d[Å] | Relative intensity (%) |
|---|---|---|---|
| 1 | 5.062 | 17.44355 | 100.0 |
| 2 | 5.330 | 16.56617 | 24.7 |
| 3 | 7.820 | 11.29642 | 55.0 |
| 4 | 10.077 | 8.77126 | 73.2 |
| 5 | 14.231 | 6.21867 | 31.4 |
| 6 | 15.192 | 5.82720 | 20.3 |
| 7 | 16.672 | 5.31317 | 30.7 |
| 8 | 17.992 | 4.92636 | 9.9 |
| 9 | 18.586 | 4.77014 | 39.8 |
| 10 | 20.435 | 4.34261 | 52.7 |
| 11 | 21.308 | 4.16658 | 11.6 |
| 12 | 21.868 | 4.06116 | 22.7 |
| 13 | 24.193 | 3.67579 | 6.0 |
| 14 | 25.442 | 3.49811 | 15.7 |
| 15 | 26.303 | 3.38548 | 13.6 |
| 16 | 28.629 | 3.11554 | 5.1 |

**Example 14: Preparation of Crystal Form F of Compound of Formula (I)**

Compound of formula (I) (10 mg) was added to dichloromethane (0.3 mL), dissolved to clarification, then isopropyl acetate (0.9 mL) was added thereto to precipitate a solid. The mixture was slurried at room temperature and centrifuged. The solid was dried under vacuum to obtain the product. After X-ray powder diffraction detection, the product was defined as crystal form F.

**Example 15: Preparation of Crystal Form F of Compound of Formula (I)**

Compound of formula (I) (10 mg) was added to 1,4-dioxane (900 uL) and dissolved to clarification. The mixture was volatilized to obtain the product. After X-ray powder diffraction detection, the product was defined as crystal form F.

**Example 16: Preparation of Crystal Form G of Compound of Formula (I)**

Compound of formula (I) (10 mg) was added to 1,2-dichloroethane (1.0 mL), slurried at room temperature, and centrifuged. The solid was dried under vacuum to obtain the product. After X-ray powder diffraction detection, the product was defined as crystal form G. The XRPD pattern is shown in Figure 8, and its characteristic peak positions are shown in Table 7. DSC pattern showed endothermic peaks at 160.43°C, 182.11°C, and 244.42°C. TGA pattern showed a weight loss of 9.97% from 25°C to 235°C.

Table 7

| Peak No. | 2θ value [° or degree] | d[Å] | Relative intensity (%) |
|---|---|---|---|
| 1 | 5.999 | 14.72062 | 53.4 |
| 2 | 7.396 | 11.94383 | 11.6 |
| 3 | 7.972 | 11.08119 | 18.7 |
| 4 | 8.637 | 10.22906 | 8.9 |
| 5 | 9.951 | 8.88167 | 100.0 |
| 6 | 11.388 | 7.76422 | 19.6 |

(continued)

| Peak No. | 2θ value [° or degree] | d[Å] | Relative intensity (%) |
|---|---|---|---|
| 7 | 12.763 | 6.93064 | 5.3 |
| 8 | 15.291 | 5.78991 | 16.9 |
| 9 | 17.812 | 4.97566 | 66.2 |
| 10 | 18.386 | 4.82162 | 41.3 |
| 11 | 19.105 | 4.64163 | 4.2 |
| 12 | 20.975 | 4.23185 | 31.6 |
| 13 | 22.920 | 3.87702 | 3.9 |
| 14 | 23.408 | 3.79731 | 7.0 |
| 15 | 25.182 | 3.53364 | 5.2 |
| 16 | 25.819 | 3.44790 | 34.4 |
| 17 | 27.400 | 3.25245 | 6.1 |

**Example 17: Preparation of Crystal Form G of Compound of Formula (I)**

Compound of formula (I) (10 mg) was added to chloroform (500 uL) and dissolved to clarification. The mixture was volatilized to obtain the product. After X-ray powder diffraction detection, the product was defined as crystal form G.

**Example 18: Preparation of Crystal Form H of Compound of Formula (I)**

Compound of formula (I) (10 mg) was added to n-hexane (1.0 mL), slurried at room temperature, and centrifuged. The solid was dried under vacuum to obtain the product. After X-ray powder diffraction detection, the product was defined as crystal form H. The XRPD pattern is shown in Figure 9, and its characteristic peak positions are shown in Table 8. DSC pattern showed endothermic peaks at 184.53°C and 247.90°C. TGA pattern showed a weight loss of 4.13% from 25°C to 215°C.

Table 8

| Peak No. | 2θ value [° or degree] | d[Å] | Relative intensity (%) |
|---|---|---|---|
| 1 | 5.758 | 15.33508 | 86.9 |
| 2 | 7.533 | 11.72609 | 48.4 |
| 3 | 9.901 | 8.92661 | 88.7 |
| 4 | 14.267 | 6.20293 | 87.7 |
| 5 | 16.420 | 5.39403 | 62.6 |
| 6 | 18.103 | 4.89639 | 100.0 |
| 7 | 18.917 | 4.68744 | 29.4 |
| 8 | 20.489 | 4.33118 | 25.9 |
| 9 | 21.563 | 4.11780 | 2.1 |
| 10 | 24.049 | 3.69743 | 23.1 |
| 11 | 26.356 | 3.37879 | 46.6 |

**Example 19: Preparation of Crystal Form I of Compound of Formula (I)**

Compound of formula (I) (10 mg) was added to tetrahydrofuran (0.4 mL) and dissolved to clarification. The mixture was slowly volatilizedat room temperature to obtain the product. After X-ray powder diffraction detection, the product was defined as crystal form I. The XRPD pattern is shown in Figure 10, and its characteristic peak positions are shown in Table 9. DSC pattern showed endothermic peaks at 182.58°C and 245.04°C. TGA pattern showed a weight loss

of 5.44% from 25°C to 225°C.

Table 9

| Peak No. | 2θ value [° or degree] | d[Å] | Relative intensity (%) |
|---|---|---|---|
| 1 | 5.294 | 16.67959 | 57.7 |
| 2 | 6.826 | 12.93958 | 48.2 |
| 3 | 7.564 | 11.67807 | 25.1 |
| 4 | 10.070 | 8.77652 | 19.4 |
| 5 | 10.739 | 8.23157 | 100.0 |
| 6 | 13.699 | 6.45883 | 15.2 |
| 7 | 16.110 | 5.49724 | 9.7 |
| 8 | 16.812 | 5.26944 | 11.9 |
| 9 | 17.601 | 5.03494 | 9.7 |
| 10 | 20.709 | 4.28576 | 31.4 |

**Example 20: Preparation of Crystal Form I of Compound of Formula (I)**

Compound of formula (I) (10 mg) was added to tetrahydrofuran (0.3 mL) and dissolved to clarification. The mixture was added with water (0.6 mL) to precipitate a solid, centrifuged, and dried under vacuum to obtain the product. After X-ray powder diffraction detection, the product was defined as crystal form I.

**Example 21: Preparation of Crystal Form J of Compound of Formula (I)**

Compound of formula (I) (10 mg) was added to DMSO (0.2 mL) and dissolved to clarification. The mixture was added with water (0.2 mL) to precipitate a solid, centrifuged, and dried under vacuum to obtain the product. After X-ray powder diffraction detection, the product was defined as crystal form J. The XRPD pattern is shown in Figure 11, and its characteristic peak positions are shown in Table 10. DSC pattern showed endothermic peaks at 154.09°C, 165.41°C, and 247.39°C. TGA pattern showed a weight loss of 10.48% from 30°C to 220°C.

Table 10

| Peak No. | 2θ value [° or degree] | d[Å] | Relative intensity (%) |
|---|---|---|---|
| 1 | 5.041 | 17.51511 | 50.4 |
| 2 | 8.212 | 10.75805 | 25. |
| 3 | 10.068 | 8.77893 | 100.0 |
| 4 | 12.170 | 7.26645 | 6.2 |
| 5 | 14.101 | 6.27581 | 25.3 |
| 6 | 15.167 | 5.83681 | 23.2 |
| 7 | 16.424 | 5.39294 | 58.1 |
| 8 | 16.962 | 5.22293 | 50.0 |
| 9 | 18.862 | 4.70102 | 5.2 |
| 10 | 19.891 | 4.45999 | 4.7 |
| 11 | 20.544 | 4.31982 | 40.3 |
| 12 | 21.190 | 4.18956 | 27.3 |
| 13 | 22.036 | 4.03053 | 9.6 |
| 14 | 22.679 | 3.91762 | 12.8 |
| 15 | 24.077 | 3.69323 | 27.5 |

(continued)

| Peak No. | 2θ value [° or degree] | d[Å] | Relative intensity (%) |
|---|---|---|---|
| 16 | 25.433 | 3.49933 | 36.0 |
| 17 | 26.111 | 3.41003 | 14.4 |
| 18 | 26.454 | 3.36657 | 14.6 |
| 19 | 28.942 | 3.08259 | 6.2 |

**Example 22: Preparation of Crystal Form J of Compound of Formula (I)**

Compound of formula (I) (10 mg) was added to DMSO (0.2 mL) and dissolved to clarification. The mixture was added with methyl *tert*-butyl ether (0.4 mL) to precipitate a solid, centrifuged, and dried under vacuum to obtain the product. After X-ray powder diffraction detection, the product was defined as crystal form J.

**Example 23: Preparation of Crystal Form K of Compound of Formula (I)**

Compound of formula (I) (10 mg) was added to N-methylpyrrolidone (50 uL) and dissolved to clarification. The mixture was slowly volatilizedat room temperature to obtain the product. After X-ray powder diffraction detection, the product was defined as crystal form K. The XRPD pattern is shown in Figure 12, and its characteristic peak positions are shown in Table 11.

Table 11

| Peak No. | 2θ value [° or degree] | d[Å] | Relative intensity (%) |
|---|---|---|---|
| 1 | 5.156 | 17.12608 | 100.0 |
| 2 | 7.699 | 11.47378 | 66.7 |
| 3 | 10.339 | 8.54876 | 82.2 |
| 4 | 10.735 | 8.23452 | 25.6 |
| 5 | 13.886 | 6.37246 | 33.2 |
| 6 | 14.334 | 6.17431 | 46.9 |
| 7 | 16.203 | 5.46577 | 43.1 |
| 8 | 18.327 | 4.83696 | 33.3 |
| 9 | 23.418 | 3.79575 | 35.9 |
| 10 | 23.725 | 3.74729 | 27.9 |
| 11 | 25.348 | 3.51082 | 18.0 |
| 12 | 25.919 | 3.43482 | 25.7 |
| 13 | 26.446 | 3.36761 | 22.8 |

**Example 24: Preparation of Crystal Form L of Compound of Formula (I)**

Compound of formula (I) (10 mg) was added to N,N-dimethylacetamide (200 uL). After the mixture turned clear, it was allowed to slowly evaporate under room temperature to obtain the product. After X-ray powder diffraction detection, the product was defined as crystal form L. The XRPD pattern is shown in Figure 13, and its characteristic peak positions are shown in Table 12.

Table 12

| Peak No. | 2θ value [° or degree] | d[Å] | Relative intensity (%) |
|---|---|---|---|
| 1 | 4.932 | 17.90379 | 53.0 |
| 2 | 5.360 | 16.47277 | 59.3 |

(continued)

| Peak No. | 2θ value [° or degree] | d[Å] | Relative intensity (%) |
|---|---|---|---|
| 3 | 9.831 | 8.98960 | 100.0 |
| 4 | 10.753 | 8.22126 | 27.2 |
| 5 | 14.844 | 5.96326 | 31.7 |
| 6 | 16.369 | 5.41103 | 18.0 |
| 7 | 18.244 | 4.85878 | 28.9 |
| 8 | 20.104 | 4.41318 | 39.6 |
| 9 | 23.129 | 3.84244 | 6.8 |
| 10 | 24.914 | 3.57098 | 75.6 |

**Example 25: Preparation of Crystal Form M of Compound of Formula (I)**

The crystal form A of the compound of formula (I) (10 mg) was heated to 225°C to obtain the product. After X-ray powder diffraction detection, the product was defined as crystal form M. The XRPD pattern is shown in Figure 14, and its characteristic peak positions are shown in Table 13.

Table 13

| Peak No. | 2θ value [° or degree] | d[Å] | Relative intensity (%) |
|---|---|---|---|
| 1 | 14.202 | 6.23131 | 48.9 |
| 2 | 14.959 | 5.91770 | 95.7 |
| 3 | 16.322 | 5.42636 | 86.5 |
| 4 | 18.410 | 4.81529 | 44.0 |
| 5 | 20.016 | 4.43252 | 27.8 |
| 6 | 20.748 | 4.27773 | 48.6 |
| 7 | 22.067 | 4.02498 | 100.0 |
| 8 | 23.670 | 3.75578 | 54.6 |
| 9 | 24.322 | 3.65655 | 1.7 |
| 10 | 24.839 | 3.58163 | 1.7 |
| 11 | 25.873 | 3.44085 | 17.3 |
| 12 | 26.863 | 3.31618 | 20.0 |
| 13 | 27.811 | 3.20532 | 12.5 |

**Example 26: Preparation of Crystal Form N of Compound of Formula (I)**

Compound of formula (I) (80 mg) was added to a mixed solvent of ethyl acetate and tetrahydrofuran (4.0 mL, V/V = 1:1). The mixture was heated and dissolved to clarification, recrystallized, and filtered. The resulting solid was dried to obtain the product. After X-ray powder diffraction detection, the product was defined as crystal form N. The XRPD pattern is shown in Figure 15, and its characteristic peak positions are shown in Table 14. DSC pattern showed endothermic peaks at 185.49°C and 188.82°C. TGApattern showed a weight loss of 4.86% from 30°C to 225°C.

Table 14

| Peak No. | 2θ value [° or degree] | d[Å] | Relative intensity (%) |
|---|---|---|---|
| 1 | 5.028 | 17.56063 | 74.1 |
| 2 | 7.671 | 11.51522 | 35.7 |
| 3 | 9.942 | 8.88987 | 100.0 |

(continued)

| Peak No. | 2θ value [° or degree] | d[Å] | Relative intensity (%) |
|---|---|---|---|
| 4 | 10.900 | 8.11071 | 37.1 |
| 5 | 12.677 | 6.97719 | 17.6 |
| 6 | 15.046 | 5.88374 | 68.9 |
| 7 | 15.428 | 5.73862 | 74.8 |
| 8 | 16.560 | 5.34887 | 35.0 |
| 9 | 18.410 | 4.81526 | 56.6 |
| 10 | 20.274 | 4.37654 | 90.8 |
| 11 | 21.148 | 4.19761 | 24.0 |
| 12 | 21.765 | 4.08016 | 25.3 |
| 13 | 24.036 | 3.69941 | 18.6 |
| 14 | 25.252 | 3.52401 | 51.6 |
| 15 | 26.385 | 3.37516 | 17.3 |

**Example 27: Preparation of Crystal Form O of Compound of Formula (I)**

Compound of formula (I) (60 mg) was added to acetonitrile (5 mL). Then benzenesulfonic acid (16 mg) was added thereto at room temperature, and the reaction mixture was reacted at 80°C for 8 hours. A solid was precipitated out of the reaction mixture, filtered, and then dried at 45°C under vacuum to obtain the product. After X-ray powder diffraction detection, the product was defined as crystal form O. The XRPD pattern is shown in Figure 16, and its characteristic peak positions are shown in Table 15.

Table 15

| Peak No. | 2θ value [° or degree] | d[Å] | Relative intensity (%) |
|---|---|---|---|
| 1 | 7.843 | 11.26283 | 31.3 |
| 2 | 8.558 | 10.32402 | 23.0 |
| 3 | 11.796 | 7.49634 | 92.7 |
| 4 | 14.106 | 6.27359 | 15.1 |
| 5 | 15.455 | 5.72872 | 24.5 |
| 6 | 16.088 | 5.50482 | 11.8 |
| 7 | 17.082 | 5.18651 | 67.4 |
| 8 | 17.423 | 5.08573 | 100.0 |
| 9 | 18.081 | 4.90224 | 67.1 |
| 10 | 19.136 | 4.63424 | 58.7 |
| 11 | 19.858 | 4.46735 | 23.4 |
| 12 | 20.281 | 4.37519 | 13.6 |
| 13 | 21.055 | 4.21593 | 29.3 |
| 14 | 21.707 | 4.09087 | 46.9 |
| 15 | 22.165 | 4.00735 | 81.0 |
| 16 | 23.743 | 3.74453 | 17.7 |
| 17 | 24.412 | 3.64329 | 33.9 |
| 18 | 25.719 | 3.46112 | 98.6 |
| 19 | 25.934 | 3.43290 | 57.0 |

(continued)

| Peak No. | 2θ value [° or degree] | d[Å] | Relative intensity (%) |
|---|---|---|---|
| 20 | 27.212 | 3.27445 | 21.5 |
| 21 | 27.538 | 3.23639 | 24.1 |
| 22 | 28.521 | 3.12710 | 30.6 |
| 23 | 30.766 | 2.90380 | 21.3 |
| 24 | 31.329 | 2.85290 | 27.4 |
| 25 | 31.868 | 2.80590 | 21.1 |
| 26 | 34.061 | 2.63007 | 8.1 |
| 27 | 36.625 | 2.45162 | 8.4 |
| 28 | 39.995 | 2.25249 | 13.8 |

**Example 28: Preparation of Crystal Form P of Compound of Formula (I)**

The crystal form C of the compound of formula (I) (40 mg) was added to dioxane (2.0 mL), slurried at room temperature, and filtered. The solid was dried under vacuum to obtain the product. After X-ray powder diffraction detection, the product was defined as crystal form P. The XRPD pattern is shown in Figure 17. DSC pattern showed endothermic peaks at 127.49°C, 180.10°C, and 249.23°C. TGA pattern showed a weight loss of 8.79% from 40°C to 230°C.

Table 16

| Peak No. | 2θ value [° or degree] | d[Å] | Relative intensity (%) |
|---|---|---|---|
| 1 | 5.359 | 16.47692 | 86.4 |
| 2 | 5.629 | 15.68836 | 53.7 |
| 3 | 7.491 | 11.79123 | 57.4 |
| 4 | 9.905 | 8.92296 | 22.1 |
| 5 | 10.786 | 8.19548 | 100.0 |
| 6 | 13.192 | 6.70583 | 20.4 |
| 7 | 14.249 | 6.21072 | 63.1 |
| 8 | 16.527 | 5.35944 | 35.1 |
| 9 | 17.729 | 4.99885 | 25.8 |
| 10 | 18.862 | 4.70104 | 16.6 |
| 11 | 20.798 | 4.26747 | 45.3 |
| 12 | 23.799 | 3.73579 | 9.8 |
| 13 | 26.555 | 3.35399 | 16.6 |

**Example 27: Preparation of Crystal Form Q of Compound of Formula (I)**

Compound of formula (I) (150 mg) was added to ethyl acetate (3 mL), slurried at room temperature, and filtered. The solid was dried at 130°C under vacuum to obtain the product. After X-ray powder diffraction detection, the product was defined as crystal form Q. The XRPD pattern is shown in Figure 18, and its characteristic peak positions are shown in Table 17. DSC pattern showed endothermic peaks at 188.58°C and 249.61°C. TGA pattern showed a weight loss of 0.54% from 25°C to 225°C.

Table 17

| Peak No. | 2θ value [° or degree] | d[Å] | Relative intensity (%) |
|---|---|---|---|
| 1 | 5.038 | 17.52734 | 49.1 |

(continued)

| Peak No. | 2θ value [° or degree] | d[Å] | Relative intensity (%) |
|---|---|---|---|
| 2 | 7.682 | 11.49962 | 14.1 |
| 3 | 10.152 | 8.70606 | 30.2 |
| 4 | 11.175 | 7.91146 | 22.0 |
| 5 | 11.902 | 7.42999 | 13.2 |
| 6 | 12.461 | 7.09750 | 12.0 |
| 7 | 13.544 | 6.53264 | 7.0 |
| 8 | 14.218 | 6.22410 | 20.4 |
| 9 | 15.246 | 5.80700 | 51.2 |
| 10 | 15.850 | 5.58692 | 100.0 |
| 11 | 16.574 | 5.34448 | 32.9 |
| 12 | 17.012 | 5.20787 | 11.5 |
| 13 | 18.124 | 4.89081 | 27.5 |
| 14 | 18.892 | 4.69354 | 92.2 |
| 15 | 20.760 | 4.27525 | 28.5 |
| 16 | 21.835 | 4.06714 | 42.5 |
| 17 | 22.440 | 3.95884 | 17.6 |
| 18 | 23.905 | 3.71949 | 24.8 |
| 19 | 25.784 | 3.45248 | 25.2 |
| 20 | 26.418 | 3.37105 | 19.2 |
| 21 | 27.295 | 3.26466 | 8.1 |

**Example 29: Preparation of Crystal Form U of Compound of Formula (I)**

Compound of formula (I) (50 mg) was added to DMSO (0.6 mL). The mixture was heated at 50°C and dissolved to clarification, then added with water (1.2 mL) for crystallization, and then centrifuged. The solid was washed with an additional 2 mL of water, centrifuged, and dried under vacuum to obtain the product. After X-ray powder diffraction detection, the product was defined as crystal form U. The XRPD pattern is shown in Figure 19, and its characteristic peak positions are shown in Table 18. DSC pattern showed endothermic peaks at 183.28°C and 248.22°C. TGA pattern showed a weight loss of 0.62% from 40°C to 215°C.

Table 18

| Peak No. | 2θ value [° or degree] | d[Å] | Relative intensity (%) |
|---|---|---|---|
| 1 | 7.657 | 11.53595 | 10.5 |
| 2 | 14.155 | 6.25201 | 100.0 |
| 3 | 15.745 | 5.62385 | 70.6 |
| 4 | 16.564 | 5.34760 | 25.9 |
| 5 | 17.314 | 5.11762 | 35.7 |
| 6 | 17.997 | 4.92481 | 31.1 |
| 7 | 18.838 | 4.70701 | 73.5 |
| 8 | 20.512 | 4.32631 | 38.7 |
| 9 | 21.415 | 4.14595 | 85.4 |
| 10 | 21.768 | 4.07959 | 61.7 |

(continued)

| Peak No. | 2θ value [° or degree] | d[Å] | Relative intensity (%) |
|---|---|---|---|
| 11 | 23.557 | 3.77361 | 31.0 |
| 12 | 25.711 | 3.46206 | 19.3 |
| 13 | 26.313 | 3.38426 | 23.5 |
| 14 | 28.029 | 3.18080 | 9.9 |

**Example 30: Preparation of Crystal Form U of Compound of Formula (I)**

The crystal form Q of the compound of formula (I) (10 mg) was added to water (1.0 mL), and slurried at room temperature for 1 day to obtain the product. After X-ray powder diffraction detection, the product was defined as crystal form U.

**Example 31: Preparation of Crystal Form X of Compound of Formula (I)**

The crystal form D of the compound of formula (I) (10 mg) was added to n-heptane (1.0 mL), and the mixture was slurried at room temperature and centrifuged. The solid was dried under vacuum to obtain the product. After X-ray powder diffraction detection, the product was defined as crystal form X. The XRPD pattern is shown in Figure 20, and its characteristic peak positions are shown in Table 19. DSC pattern showed endothermic peaks at 158.81°C, 184.14°C, and 249.04°C.

Table 19

| Peak No. | 2θ value [° or degree] | d[Å] | Relative intensity (%) |
|---|---|---|---|
| 1 | 6.864 | 12.86741 | 21.8 |
| 2 | 9.873 | 8.95188 | 22.0 |
| 3 | 10.963 | 8.06358 | 12.8 |
| 4 | 13.475 | 6.56575 | 12.5 |
| 5 | 13.801 | 6.41151 | 13.3 |
| 6 | 16.089 | 5.50446 | 14.7 |
| 7 | 18.006 | 4.92253 | 100.0 |
| 8 | 20.158 | 4.40145 | 15.4 |
| 9 | 20.929 | 4.24113 | 23.1 |
| 10 | 26.203 | 3.39818 | 12.2 |

**Example 32: Preparation of Crystal Form Y of Compound of Formula (I)**

The crystal form Q of the compound of formula (I) (10 mg) was added to *n*-heptane (1.0 mL), slurried at room temperature, and centrifuged. The solid was dried under vacuum to obtain the product. After X-ray powder diffraction detection, the product was defined as crystal form Y. The XRPD pattern is shown in Figure 21, and its characteristic peak positions are shown in Table 20. DSC pattern showed endothermic peaks at 184.97°C and 248.72°C.

Table 20

| Peak No. | 2θ value [° or degree] | d[Å] | Relative intensity (%) |
|---|---|---|---|
| 1 | 5.676 | 15.55652 | 13.3 |
| 2 | 7.666 | 11.52267 | 7.1 |
| 3 | 7.875 | 11.21711 | 6.2 |
| 4 | 9.985 | 8.85146 | 3.5 |
| 5 | 12.634 | 7.00073 | 3.9 |

(continued)

| Peak No. | 2θ value [° or degree] | d[Å] | Relative intensity (%) |
|---|---|---|---|
| 6 | 14.260 | 6.20594 | 7.5 |
| 7 | 16.114 | 5.49605 | 6.8 |
| 8 | 16.562 | 5.34817 | 7.8 |
| 9 | 18.020 | 4.91859 | 100.0 |
| 10 | 18.874 | 4.69807 | 14.9 |
| 11 | 21.802 | 4.07319 | 4.5 |
| 12 | 24.051 | 3.69713 | 3.1 |
| 13 | 25.846 | 3.44430 | 3.2 |
| 14 | 26.425 | 3.37019 | 4.9 |
| 15 | 26.974 | 3.30277 | 3.6 |

**Example 33: Preparation of Crystal Form V of Compound of Formula (I)**

Compound of formula (I) (200 mg) was added to *N,N*-dimethylformamide (0.4 mL). Then acetonitrile (2 mL) was added to the system. The mixture was stirred at 20°C to 30°C, filtered under reduced pressure. The filter cake was dried to obtain the product. After X-ray powder diffraction detection, the product was defined as crystal form V. The XRPD pattern is shown in Figure 22, and its characteristic peak positions are shown in Table 21. DSC pattern showed an endothermic peak at 147.78°C. TGA pattern showed a weight loss of 10.73% from 30°C to 210°C.

Table 21

| Peak No. | 2θ value [° or degree] | d[Å] | Relative intensity (%) |
|---|---|---|---|
| 1 | 5.649 | 15.63089 | 90.2 |
| 2 | 6.154 | 14.34928 | 100.0 |
| 3 | 6.720 | 13.14362 | 77.8 |
| 4 | 9.778 | 9.03863 | 23.2 |
| 5 | 10.830 | 8.16267 | 12.3 |
| 6 | 11.409 | 7.74959 | 38.9 |
| 7 | 11.651 | 7.58921 | 96.2 |
| 8 | 12.390 | 7.13839 | 12.9 |
| 9 | 13.576 | 6.51708 | 17.6 |
| 10 | 13.878 | 6.37593 | 12.9 |
| 11 | 14.272 | 6.20085 | 17.1 |
| 12 | 14.769 | 5.99321 | 4.1 |
| 13 | 16.361 | 5.41358 | 11.8 |
| 14 | 16.815 | 5.26837 | 8.6 |
| 15 | 17.570 | 5.04354 | 20.6 |
| 16 | 18.274 | 4.85076 | 9.8 |
| 17 | 18.757 | 4.72706 | 45.0 |
| 18 | 19.813 | 4.47745 | 51.3 |
| 19 | 20.609 | 4.30614 | 13.0 |
| 20 | 20.727 | 4.28207 | 12.2 |
| 21 | 21.905 | 4.05427 | 15.5 |

(continued)

| Peak No. | 2θ value [° or degree] | d[Å] | Relative intensity (%) |
|---|---|---|---|
| 22 | 22.721 | 3.91056 | 5.2 |
| 23 | 22.947 | 3.87250 | 6.0 |
| 24 | 23.948 | 3.71286 | 33.0 |
| 25 | 24.370 | 3.64956 | 9.2 |
| 26 | 25.825 | 3.44713 | 20.2 |
| 27 | 26.995 | 3.30028 | 20.3 |
| 28 | 27.595 | 3.22984 | 9.5 |
| 29 | 28.859 | 3.09128 | 7.2 |
| 30 | 29.953 | 2.98076 | 5.9 |
| 31 | 31.628 | 2.82660 | 3.1 |
| 32 | 33.136 | 2.70136 | 5.3 |
| 33 | 34.931 | 2.56651 | 10.0 |

**Example 34: Preparation of Crystal Form V of Compound of Formula (I)**

Compound of formula (I) (200 mg) was added to *N,N*-dimethylformamide (1 mL). The mixture was heated to 60°C until the compound was dissolved, then cooled naturally to 20°C-30°C, stirred at 20°C-30°C, and filtered under reduced pressure. The filter cake was rinsed with *N,N*-dimethylformamide (0.2 mL), and dried at 45°C under reduced pressure for 26 hours to obtain the product. After X-ray powder diffraction detection, the product was defined as crystal form V.

**Example 35: Preparation of Crystal Form R of Compound of Formula (I)**

Compound of formula (I) (8 g) was added to tetrahydrofuran (150 mL). The mixture was stirred until the compound was dissolved, and concentrated under reduced pressure until no distillate appeared, thus obtaining the product. After X-ray powder diffraction detection, the product was defined as crystal form R. The XRPD pattern is shown in Figure 23, and its characteristic peak positions are shown in Table 22.

Table 22

| Peak No. | 2θ value [° or degree] | d[Å] | Relative intensity (%) |
|---|---|---|---|
| 1 | 5.132 | 17.20688 | 57.2 |
| 2 | 5.534 | 15.95544 | 100.0 |
| 3 | 7.232 | 12.21394 | 23.7 |
| 4 | 7.611 | 11.60654 | 48.8 |
| 5 | 7.831 | 11.28001 | 29.7 |
| 6 | 10.033 | 8.80950 | 26.8 |
| 7 | 10.437 | 8.46890 | 18.1 |
| 8 | 11.148 | 7.93072 | 12.0 |
| 9 | 11.857 | 7.45771 | 16.1 |
| 10 | 12.737 | 6.94459 | 20.8 |
| 11 | 14.179 | 6.24131 | 10.5 |
| 12 | 15.782 | 5.61066 | 31.9 |
| 13 | 16.575 | 5.34421 | 24.6 |
| 14 | 17.101 | 5.18101 | 36.3 |

(continued)

| Peak No. | 2θ value [° or degree] | d[Å] | Relative intensity (%) |
|---|---|---|---|
| 15 | 19.017 | 4.66306 | 51.1 |
| 16 | 20.073 | 4.42007 | 26.4 |
| 17 | 20.567 | 4.31493 | 27.9 |
| 18 | 21.871 | 4.06049 | 12.5 |
| 19 | 23.692 | 3.75232 | 12.7 |

**Example 36: Preparation of Crystal Form S of Compound of Formula (I)**

Compound of formula (I) was dissolved in *N,N*-dimethylformamide by heating, then precipitated by cooling. The compound was slurried with acetonitrile, and then filtered. The filter cake was slurried with water once, and filtered. The sample was dried at 60°C, added with acetonitrile (1 L), stirred at 20°C-30°C, filtered, and dried to obtain the product. After X-ray powder diffraction detection, the product was defined as crystal form S. The XRPD pattern is shown in Figure 24, and its characteristic peak positions are shown in Table 23.

Table 23

| Peak No. | 2θ value [° or degree] | d[Å] | Relative intensity (%) |
|---|---|---|---|
| 1 | 6.576 | 13.42968 | 41.0 |
| 2 | 7.890 | 11.19569 | 8.5 |
| 3 | 9.082 | 9.72923 | 31.3 |
| 4 | 10.921 | 8.09450 | 39.0 |
| 5 | 11.921 | 7.41792 | 7.3 |
| 6 | 13.592 | 6.50936 | 31.5 |
| 7 | 14.500 | 6.10405 | 4.3 |
| 8 | 15.043 | 5.88470 | 12.5 |
| 9 | 15.951 | 5.55172 | 16.3 |
| 10 | 16.495 | 5.36979 | 18.6 |
| 11 | 16.805 | 5.27157 | 20.9 |
| 12 | 19.965 | 4.44366 | 100.0 |
| 13 | 21.403 | 4.14833 | 34.5 |
| 14 | 24.207 | 3.67365 | 46.0 |
| 15 | 25.662 | 3.46867 | 23.7 |
| 16 | 26.537 | 3.35624 | 9.2 |
| 17 | 27.457 | 3.24575 | 21.5 |
| 18 | 30.646 | 2.91489 | 4.8 |

**Example 37: Preparation of Crystal Form T of Compound of Formula (I)**

Compound of formula (I) (70.9 g) was added to *N,N*-dimethylformamide (200 mL). The mixture was heated to 70°C-80°C until the compound was dissolved, then cooled to 20°C-30°C to precipitate the compound, stirred, and filtered, and the filter cake was added to water (1200 mL) and stirred. After filtering, the filter cake was again added to water (1200 mL) and stirred, followed by filtration. The filter cake was dried at 40°C-45°C. The dried material (45.0 g) was added to acetonitrile (450 mL), and subjected to magnetic stirring at 20°C-30°C for 29 hours. The mixture was filtered under reduced pressure, then dried under reduced pressure for 17 hours to obtain the product. After X-ray powder diffraction detection, the product was defined as crystal form T. The XRPD pattern is shown in Figure 25, and its characteristic peak positions are shown in Table 24. DSC pattern showed endothermic peaks at 164.78°C and 247.66°C.

Table 24

| Peak No. | 2θ value [° or degree] | d[Å] | Relative intensity (%) |
|---|---|---|---|
| 1 | 6.610 | 13.36086 | 37.5 |
| 2 | 6.915 | 12.77296 | 62.8 |
| 3 | 9.177 | 9.62879 | 37.7 |
| 4 | 9.984 | 8.85256 | 94.9 |
| 5 | 11.012 | 8.02791 | 87.7 |
| 6 | 13.595 | 6.50797 | 65.3 |
| 7 | 13.926 | 6.35399 | 45.7 |
| 8 | 15.174 | 5.83405 | 13.8 |
| 9 | 16.156 | 5.48189 | 38.6 |
| 10 | 18.509 | 4.78977 | 12.4 |
| 11 | 20.138 | 4.40594 | 100.0 |
| 12 | 21.037 | 4.21952 | 70.8 |
| 13 | 22.954 | 3.87131 | 7.4 |
| 14 | 24.261 | 3.66563 | 16.7 |
| 15 | 26.391 | 3.37441 | 13.3 |
| 16 | 27.514 | 3.23919 | 10.0 |

**Example 38: Hygroscopicity Study of Crystal Forms D, E, Q, and U of Compound of Formula (I) (October 15, 2021, Nanjing)**

Using the Surface Measurement Systems advantage 2, at a temperature of 25°C, and starting at a humidity of 50%, the humidity range was examined from 0% to 95%, with increments of 10%. The criterion for evaluation was that the mass change dM/dT at each gradient was less than 0.002%, TMAX was 360 min, and the process was cycled twice.

Table 25

| Test sample | 0.0%RH-95.0%RH | 20%RH-80%RH | Crystal form |
|---|---|---|---|
| Crystal form D | 2.24% | 1.26% (slightly hygroscopic) | Untransformed |
| Crystal form E | 2.24% | 0.84% (slightly hygroscopic) | Untransformed |
| Crystal form Q | 0.67% | 0.24% (slightly hygroscopic) | Untransformed |
| Crystal form U | 2.06% | 1.03% (slightly hygroscopic) | Untransformed |

**Example 39: Stability of Factors Influencing Crystal Forms D, E, Q, and U of Compound of Formula (I)**

Each crystal form was spread out in an open exposure setup, and the stability of the samples under conditions of light (4500 Lux), high temperature (40°C, 60°C), and high humidity (RH 75%, RH 92.5%) was examined separately. The sampling inspection period was 30 days.

Table 26

| Condition | Time (days) | Free crystal form D | | |
|---|---|---|---|---|
| | | Color and character | Purity % | Crystal form |
| Onset | 0 | White solid | 97.9 | D |

(continued)

| Condition | Time (days) | Free crystal form D | | |
|---|---|---|---|---|
| | | Color and character | Purity % | Crystal form |
| 40°C | 9 | White solid | 97.3 | D |
| | 15 | White solid | 96.7 | D |
| | 30 | White solid | 95.8 | D |
| 60°C | 9 | White solid | 96.6 | D |
| | 15 | White solid | 95.8 | D |
| | 30 | White solid | 93.9 | D |
| 75% RH | 9 | White solid | 97.8 | D |
| | 15 | White solid | 97.7 | D |
| | 30 | White solid | 97.9 | D |
| 92.5% RH | 9 | White solid | 97.8 | D |
| | 15 | White solid | 97.9 | D |
| | 30 | White solid | 97.8 | D |
| 4500 Lux | 9 | White solid | 97.3 | D |
| | 15 | White solid | 96.8 | D |
| | 30 | White solid | 95.4 | D |
| Condition | Time (days) | Free crystal form E | | |
| | | Color and character | Purity % | Crystal form |
| Onset | 0 | White solid | 97.9 | E |
| 40°C | 9 | White solid | 97.4 | E |
| | 15 | White solid | 97.0 | E |
| | 30 | White solid | 96.4 | E |
| 60°C | 9 | White solid | 96.7 | E |
| | 15 | White solid | 95.9 | E |
| | 30 | White solid | 94.6 | E |
| 75% RH | 9 | White solid | 97.9 | E |
| | 15 | White solid | 97.9 | E |
| | 30 | White solid | 97.8 | E |
| 92.5% RH | 9 | White solid | 97.9 | E |
| | 15 | White solid | 97.8 | E |
| | 30 | White solid | 97.8 | E |
| 4500 Lux | 9 | White solid | 97.7 | E |
| | 15 | White solid | 97.4 | E |
| | 30 | White solid | 94.9 | E |
| Condition | Time (days) | Free crystal form Q | | |
| | | Color and character | Purity % | Crystal form |
| Onset | 0 | White solid | 99.6 | Q |

(continued)

| Condition | Time (days) | Free crystal form Q | | |
|---|---|---|---|---|
| | | Color and character | Purity % | Crystal form |
| 40°C | 7 | White solid | 99.5 | Q |
| | 14 | White solid | 99.4 | Q |
| | 30 | White solid | 99.1 | Q |
| 60°C | 7 | White solid | 99.3 | Q |
| | 14 | White solid | 98.9 | Q |
| | 30 | White solid | 98.5 | Q |
| 75% RH | 7 | White solid | 99.6 | Q |
| | 14 | White solid | 99.6 | Q |
| | 30 | White solid | 99.6 | Q |
| 92.5% RH | 7 | White solid | 99.6 | Q |
| | 14 | White solid | 99.6 | Q |
| | 30 | White solid | 99.5 | Q |
| 4500 Lux | 7 | White solid | 99.5 | Q |
| | 14 | White solid | 98.8 | Q |
| | 30 | White solid | 93.9 | Q |
| Condition | Time (days) | Free crystal form U | | |
| | | Color and character | Purity % | Crystal form |
| Onset | 0 | White solid | 99.7 | U |
| 40°C | 7 | White solid | 99.5 | U |
| | 14 | White solid | 99.3 | U |
| | 30 | White solid | 98.8 | U |
| 60°C | 7 | White solid | 98.9 | U |
| | 14 | White solid | 98.5 | U |
| | 30 | White solid | 97.5 | U |
| 75% RH | 7 | White solid | 99.6 | U |
| | 14 | White solid | 99.7 | U |
| | 30 | White solid | 99.6 | U |
| 92.5% RH | 7 | White solid | 99.7 | U |
| | 14 | White solid | 99.7 | U |
| | 30 | White solid | 99.6 | U |
| 4500 Lux | 7 | White solid | 99.3 | U |
| | 14 | White solid | 99.3 | U |
| | 30 | White solid | 98.8 | U |

Conclusion: The influencing factor experiments demonstrate that the crystal forms D, E, Q, and U of the compound of formula (I) exhibit good physicochemical stability under high humidity conditions of 75% and 92.5%. There is a slight decrease in purity under the conditions of 60°C and light, and the crystal forms remain unchanged.

**Example 40: Long-term/Accelerated Stability of Crystal Forms D, E, Q, and U of Compound of Formula (I)**

The crystal forms D, E, Q, and U of the compound of formula (I) were respectively placed under conditions of 25°C with 60% RH and 40°C with 75% RH to evaluate their stability.

Table 27

| Sample | Placement conditions | Purity % | Purity % | Purity % | Purity % | Purity % | Crystal form |
|---|---|---|---|---|---|---|---|
| | | Onset | 1 month | 2 months | 3 months | 6 months | D |
| | 25°C, 60%RH | 97.9 | 97.8 | 97.8 | 97.8 | 97.8 | D |
| | 40°C, 75%RH | | 97.8 | 97.8 | 97.7 | 97.6 | D |

Table 28

| Sample | Placement conditions | Purity % | Purity % | Purity % | Purity % | Purity % | Crystal form |
|---|---|---|---|---|---|---|---|
| | | Onset | 1 month | 2 months | 3 months | 6 months | E |
| | 25°C, 60%RH | 97.9 | 97.9 | 97.8 | 97.8 | 97.8 | E |
| | 40°C, 75%RH | | 97.8 | 97.8 | 97.8 | 97.4 | E |

Table 29

| Sample | Placement conditions | Purity % | Purity % | Purity % | Purity % | Purity % | Crystal form |
|---|---|---|---|---|---|---|---|
| | | Onset | 1 month | 2 months | 3 months | 6 months | Q |
| | 25°C, 60%RH | 99.6 | 99.5 | 99.5 | 99.5 | 99.4 | Q |
| | 40°C, 75%RH | | 99.5 | 99.4 | 99.4 | 99.1 | Q |

Table 30

| Sample | Placement conditions | Purity % | Purity % | Purity % | Purity % | Purity % | Crystal form |
|---|---|---|---|---|---|---|---|
| | | Onset | 1 month | 2 months | 3 months | 6 months | U |
| | 25°C, 60%RH | 99.7 | 99.7 | 99.6 | 99.6 | 99.6 | U |
| | 40°C, 75%RH | | 99.7 | 99.6 | 99.6 | 99.5 | U |

Conclusion: The free crystal forms D, E, Q, and U demonstrate good physical and chemical stability when subjected to long-term accelerated stability conditions for a duration of six months.

## Claims

**1.** A crystal form A of a compound of formula (I),

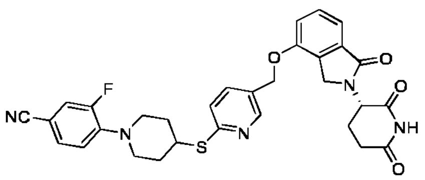

formula I,

wherein the crystal form A has an X-ray powder diffraction pattern, represented by a diffraction angle 2θ, having characteristic peaks at 5.765, 8.061, 9.925, 16.632, 17.900, 19.469, and 21.115, preferably at 5.765, 7.465, 8.061, 9.925, 12.890, 15.085, 16.632, 17.900, 19.469, and 21.115, more preferably at 5.765, 7.465, 8.061, 9.925, 11.674,

12.890, 14.270, 15.085, 16.632, 17.900, 18.715, 19.469, and 21.115; most preferably, the crystal form A has the X-ray powder diffraction pattern represented by the diffraction angle 2θ as shown in Figure 2.

**2.** A crystal form B of a compound of formula (I),

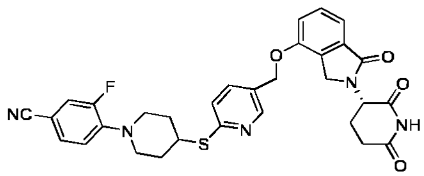

formula I,

wherein the crystal form B has an X-ray powder diffraction pattern, represented by a diffraction angle 2θ, having characteristic peaks at 4.977, 6.788, 10.047, 14.143, 15.684, 18.547, and 20.840, preferably at 4.977, 6.788, 10.047, 14.143, 15.684, 18.547, 20.840, 24.096, and 25.505; more preferably, the crystal form B has the X-ray powder diffraction pattern represented by the diffraction angle 2θ as shown in Figure 3.

**3.** A crystal form C of a compound of formula (I),

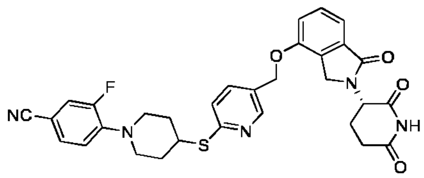

formula I,

wherein the crystal form C has an X-ray powder diffraction pattern, represented by a diffraction angle 2θ, having characteristic peaks at 5.653, 7.974, 9.989, 16.143, 17.860, 18.992, and 20.972, preferably at 5.653, 7.974, 9.989, 11.505, 12.798, 14.265, 16.143, 17.860, 18.992, and 20.972, more preferably at 3.533, 5.653, 7.974, 8.790, 9.989, 11.505, 12.798, 14.265, 15.277, 16.143, 17.860, 18.992, and 20.972; most preferably, the crystal form C has the X-ray powder diffraction pattern represented by the diffraction angle 2θ as shown in Figure 4.

**4.** A crystal form D of a compound of formula (I),

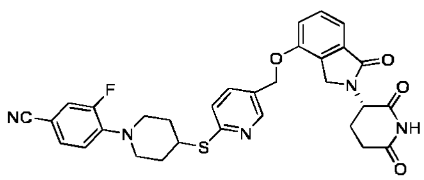

formula I,

wherein the crystal form D has an X-ray powder diffraction pattern, represented by a diffraction angle 2θ, having characteristic peaks at 6.831, 9.845, 13.453, 18.225, 20.117, 20.891, and 23.006, preferably at 6.831, 9.845, 10.927, 13.453, 16.096, 18.225, 20.117, 20.891, 23.006, and 26.132; more preferably, the crystal form D has the X-ray powder diffraction pattern represented by the diffraction angle 2θ as shown in Figure 5.

**5.** A crystal form E of a compound of formula (I),

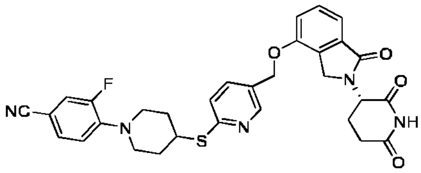

formula I,

wherein the crystal form E has an X-ray powder diffraction pattern, represented by a diffraction angle 2θ, having characteristic peaks at 5.864, 7.573, 8.087, 10.003, 16.444, 19.349, and 20.553, preferably at 5.864, 7.573, 8.087, 10.003, 12.471, 15.165, 16.444, 17.432, 19.349, and 20.553, more preferably at 5.864, 7.573, 8.087, 10.003, 11.701, 12.471, 15.165, 16.444, 17.432, 19.349, 20.553, 21.067, and 21.709; most preferably, the crystal form E has the X-ray powder diffraction pattern represented by the diffraction angle 2θ as shown in Figure 6.

**6.** A crystal form F of a compound of formula (I),

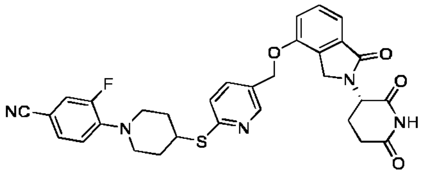

formula I,

wherein the crystal form F has an X-ray powder diffraction pattern, represented by a diffraction angle 2θ, having characteristic peaks at 5.062, 7.820, 10.077, 14.231, 16.672, 18.586, and 20.435, preferably at 5.062, 7.820, 10.077, 14.231, 15.192, 16.672, 18.586, 20.435, 21.868, and 25.442, more preferably at 5.062, 7.820, 10.077, 14.231, 15.192, 16.672, 18.586, 20.435, 21.868, 24.193, 25.442, 26.303, and 28.629; most preferably, the crystal form F has the X-ray powder diffraction pattern represented by the diffraction angle 2θ as shown in Figure 7.

**7.** A crystal form G of a compound of formula (I),

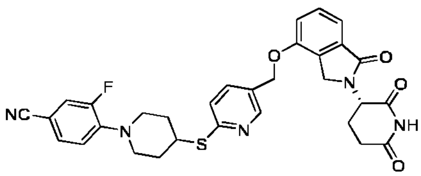

formula I,

wherein the crystal form G has an X-ray powder diffraction pattern, represented by a diffraction angle 2θ, having characteristic peaks at 5.999, 7.972, 9.951, 11.388, 17.812, 20.975, and 25.819, preferably at 5.999, 7.396, 7.972, 8.637, 9.951, 11.388, 15.291, 17.812, 20.975, and 25.819, more preferably at 5.999, 7.396, 7.972, 8.637, 9.951, 11.388, 12.763, 15.291, 17.812, 20.975, 23.408, 25.819, and 27.400; most preferably, the crystal form G has the X-ray powder diffraction pattern represented by the diffraction angle 2θ as shown in Figure 8.

**8.** A crystal form H of a compound of formula (I),

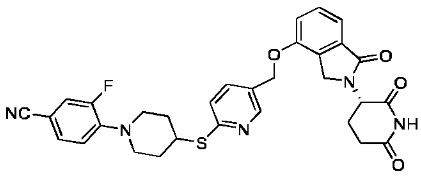

formula I,

wherein the crystal form H has an X-ray powder diffraction pattern, represented by a diffraction angle 2θ, having characteristic peaks at 5.758, 7.533, 9.901, 14.267, 16.420, 18.103, and 26.356, preferably at 5.758, 7.533, 9.901, 14.267, 16.420, 18.103, 18.917, 20.489, 24.049, and 26.356, more preferably at 5.758, 7.533, 9.901, 14.267, 16.420, 18.103, 18.917, 20.489, 21.563, 24.049, and 26.356; most preferably, the crystal form H has the X-ray powder diffraction pattern represented by the diffraction angle 2θ as shown in Figure 9.

**9.** A crystal form I of a compound of formula (I),

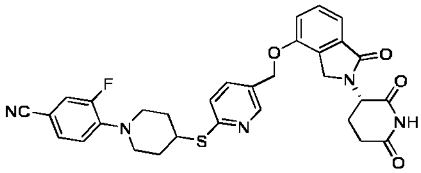

formula I,

wherein the crystal form I has an X-ray powder diffraction pattern, represented by a diffraction angle 2θ, having characteristic peaks at 5.294, 6.826, 7.564, 10.739, 13.699, 16.812, and 20.709; preferably, the crystal form I has the X-ray powder diffraction pattern represented by the diffraction angle 2θ as shown in Figure 10.

**10.** A crystal form J of a compound of formula (I),

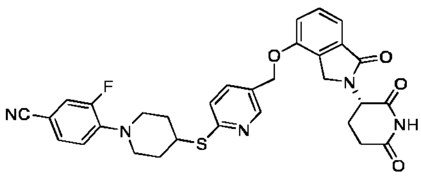

formula I,

wherein the crystal form J has an X-ray powder diffraction pattern, represented by a diffraction angle 2θ, having characteristic peaks at 5.041, 10.068, 16.424, 20.544, 21.190, 24.077, and 25.433, preferably at 5.041, 8.212, 10.068, 14.101, 15.167, 16.424, 20.544, 21.190, 24.077, and 25.433, more preferably at 5.041, 8.212, 10.068, 14.101, 15.167, 16.424, 20.544, 21.190, 22.036, 22.679, 24.077, 25.433, and 26.454; most preferably, the crystal form J has the X-ray powder diffraction pattern represented by the diffraction angle 2θ as shown in Figure 11.

**11.** A crystal form K of a compound of formula (I),

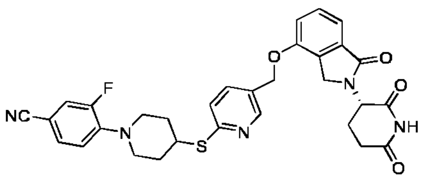

formula I,

wherein the crystal form K has an X-ray powder diffraction pattern, represented by a diffraction angle 2θ, having characteristic peaks at 5.156, 7.699, 10.339, 14.334, 16.203, 18.327, and 23.418, preferably at 5.156, 7.699, 10.339, 14.334, 16.203, 18.327, 23.418, 25.348, 25.919, and 26.446; more preferably, the crystal form K has the X-ray powder diffraction pattern represented by the diffraction angle 2θ as shown in Figure 12.

**12.** A crystal form L of a compound of formula (I),

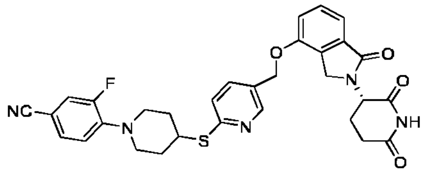

formula I,

wherein the crystal form L has an X-ray powder diffraction pattern, represented by a diffraction angle 2θ, having characteristic peaks at4.932, 5.360, 9.831, 14.844, 18.244, 20.104, and 24.914, preferably at 4.932, 5.360, 9.831, 10.753, 14.844, 16.369, 18.244, 20.104, 23.129, and 24.914; more preferably, the crystal form L has the X-ray powder diffraction pattern represented by the diffraction angle 2θ as shown in Figure 13.

**13.** A crystal form M of a compound of formula (I),

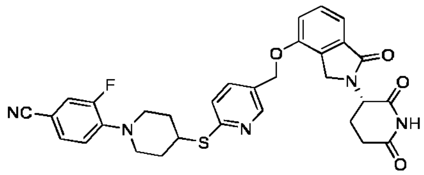

formula I,

wherein the crystal form M has an X-ray powder diffraction pattern, represented by a diffraction angle 2θ, having characteristic peaks at 14.959, 16.322, 18.410, 20.748, 22.067, 23.670, and 26.863, preferably at 14.959, 16.322, 18.410, 20.748, 22.067, 23.670, 24.839, 25.873, 26.863, and 27.811, more preferably at 14.959, 16.322, 18.410, 20.748, 22.067, 23.670, 24.322, 24.839, 25.873, 26.863, and 27.811; most preferably, the crystal form M has the X-ray powder diffraction pattern represented by the diffraction angle 2θ as shown in Figure 14.

**14.** A crystal form N of a compound of formula (I),

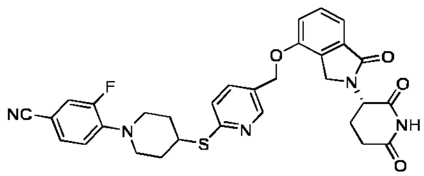

formula I,

wherein the crystal form N has an X-ray powder diffraction pattern, represented by a diffraction angle 2θ, having characteristic peaks at 5.028, 9.942, 10.900, 15.428, 18.410, 20.274, and 25.252, preferably at 5.028, 7.671, 9.942, 10.900, 15.428, 16.560, 18.410, 20.274, 24.036, and 25.252, more preferably at 5.028, 7.671, 9.942, 10.900, 12.677, 15.428, 16.560, 18.410, 20.274, 24.036, 25.252, and 26.385; most preferably, the crystal form N has the X-ray powder diffraction pattern represented by the diffraction angle 2θ as shown in Figure 15.

**15.** A crystal form O of a compound of formula (I),

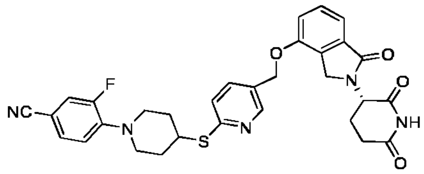

formula I,

wherein the crystal form O has an X-ray powder diffraction pattern, represented by a diffraction angle 2θ, having characteristic peaks at 11.796, 17.423, 18.081, 19.136, 21.707, 22.165, and 25.719, preferably at 7.843, 11.796, 17.423, 18.081, 19.136, 21.707, 22.165, 24.412, 25.719, and 28.521, more preferably at diffraction angles 2θ of 7.843, 11.796, 15.455, 17.423, 18.081, 19.136, 21.055, 21.707, 22.165, 24.412, 25.719, 27.538, and 28.521; most preferably, the crystal form O has the X-ray powder diffraction pattern represented by the diffraction angle 2θ as shown in Figure 16.

**16.** A crystal form P of a compound of formula (I),

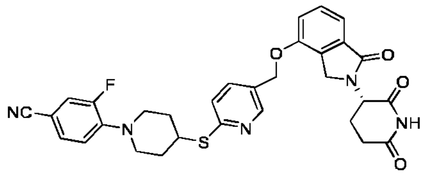

formula I,

wherein the crystal form P has an X-ray powder diffraction pattern, represented by a diffraction angle 2θ, having characteristic peaks at 5.359, 7.491, 10.786, 14.249, 16.527, 17.729, and 20.798, preferably at 5.359, 7.491, 9.905, 10.786, 13.192, 14.249, 16.527, 17.729, 18.862, and 20.798, more preferably at 5.359, 7.491, 9.905, 10.786, 13.192, 14.249, 16.527, 17.729, 18.862, 20.798, 23.799, and 26.555; most preferably, the crystal form P has the X-ray powder diffraction pattern represented by the diffraction angle 2θ as shown in Figure 17.

**17.** A crystal form Q of a compound of formula (I),

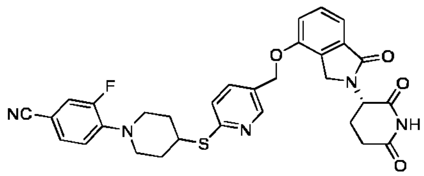

formula I,

wherein the crystal form Q has an X-ray powder diffraction pattern, represented by a diffraction angle 2θ, having characteristic peaks at 5.038, 10.152, 15.850, 16.574, 18.892, 20.760, and 21.835, preferably at 5.038, 10.152, 11.175, 15.850, 16.574, 18.892, 20.760, 21.835, 23.905, and 25.784, more preferably at 5.038, 7.682, 10.152, 11.175, 14.218, 15.850, 16.574, 18.892, 20.760, 21.835, 23.905, 25.784, and 26.418; most preferably, the crystal form Q has the X-ray powder diffraction pattern represented by the diffraction angle 2θ as shown in Figure 18.

**18.** A crystal form U of a compound of formula (I),

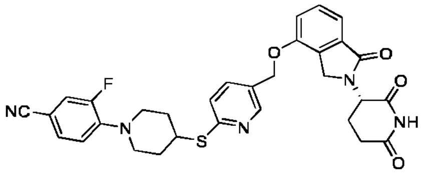

formula I,

wherein the crystal form U has an X-ray powder diffraction pattern, represented by a diffraction angle 2θ, having characteristic peaks at 14.155, 15.745, 17.314, 17.997, 18.838, 20.512, and 21.415, preferably at 14.155, 15.745, 16.564, 17.314, 17.997, 18.838, 20.512, 21.415, 23.557, and 26.313, more preferably at 7.657, 14.155, 15.745, 16.564, 17.314, 17.997, 18.838, 20.512, 21.415, 23.557, 25.711, 26.313, and 28.029; most preferably, the crystal form U has the X-ray powder diffraction pattern represented by the diffraction angle 2θ as shown in Figure 19.

**19.** A crystal form X of a compound of formula (I),

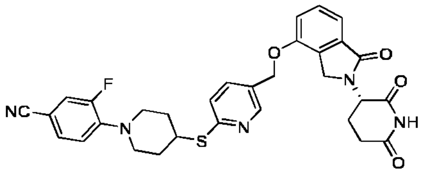

formula I,

wherein the crystal form X has an X-ray powder diffraction pattern, represented by a diffraction angle 2θ, having characteristic peaks at 6.864, 9.873, 10.963, 13.801, 16.089, 18.006, and 20.929, preferably at 6.864, 9.873, 10.963, 13.801, 16.089, 18.006, 20.929, and 26.203; more preferably, the crystal form X has the X-ray powder diffraction pattern represented by the diffraction angle 2θ as shown in Figure 20.

**20.** A crystal form Y of a compound of formula (I),

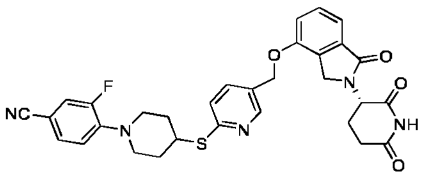

formula I,

wherein the crystal form Y has an X-ray powder diffraction pattern, represented by a diffraction angle 2θ, having characteristic peaks at 5.676, 7.666, 14.260, 16.562, 18.020, 21.802, and 26.425, preferably at 5.676, 7.666, 9.985, 12.634, 14.260, 16.562, 18.020, 21.802, 26.425, and 26.974, more preferably at 5.676, 7.666, 9.985, 12.634, 14.260, 16.562, 18.020, 21.802, 24.051, 25.846, 26.425, and 26.974; most preferably, the crystal form Y has the X-ray powder diffraction pattern represented by the diffraction angle 2θ as shown in Figure 21.

**21.** A crystal form V of a compound of formula (I),

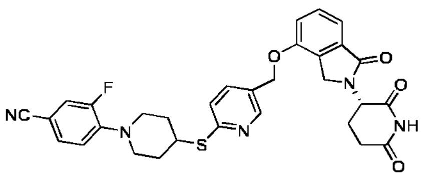

formula I,

wherein the crystal form V has an X-ray powder diffraction pattern, represented by a diffraction angle 2θ, having characteristic peaks at 5.649, 6.154, 6.720, 11.651, 18.757, 19.813, and 23.948, preferably at 5.649, 6.154, 6.720, 9.778, 11.651, 17.570, 18.757, 19.813, 23.948, and 26.995, more preferably at 5.649, 6.154, 6.720, 9.778, 11.651, 13.576, 17.570, 18.757, 19.813, 21.905, 23.948, 25.825, and 26.995; most preferably, the crystal form V has the X-ray powder diffraction pattern represented by the diffraction angle 2θ as shown in Figure 22.

**22.** A crystal form R of a compound of formula (I),

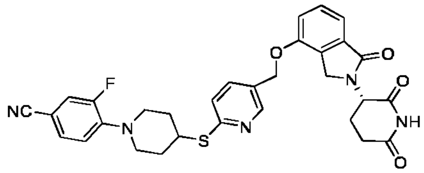

formula I,

wherein the crystal form R has an X-ray powder diffraction pattern, represented by a diffraction angle 2θ, having characteristic peaks at 5.534, 7.611, 10.033, 15.782, 17.101, 19.017, and 20.567, preferably at 5.534, 7.611, 10.033, 11.857, 12.737, 15.782, 17.101, 19.017, 20.567, and 23.692, more preferably at 5.534, 7.611, 10.033, 11.148, 11.857, 12.737, 14.179, 15.782, 17.101, 19.017, 20.567, 21.871, and 23.692; most preferably, the crystal form R has the X-ray powder diffraction pattern represented by the diffraction angle 2θ as shown in Figure 23.

**23.** A crystal form S of a compound of formula (I),

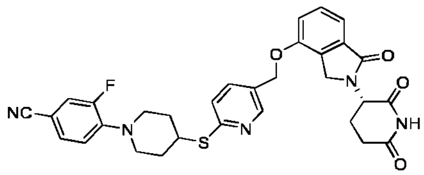

formula I,

wherein the crystal form S has an X-ray powder diffraction pattern, represented by a diffraction angle 2θ, having characteristic peaks at 6.576, 9.082, 10.921, 13.592, 19.965, 21.403, and 24.207, preferably at 6.576, 9.082, 10.921, 13.592, 16.805, 19.965, 21.403, 24.207, 25.662, and 27.457, more preferably at 6.576, 7.890, 9.082, 10.921, 13.592, 15.043, 16.805, 19.965, 21.403, 24.207, 25.662, 26.537, and 27.457; most preferably, the crystal form S has the X-ray powder diffraction pattern represented by the diffraction angle 2θ as shown in Figure 24.

**24.** A crystal form T of a compound of formula (I),

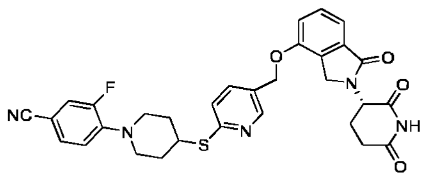

formula I,

wherein the crystal form T has an X-ray powder diffraction pattern, represented by a diffraction angle 2θ, having characteristic peaks at 6.915, 9.177, 9.984, 11.012, 13.595, 16.156, and 20.138, preferably at 6.915, 9.177, 9.984, 11.012, 13.595, 15.174, 16.156, 20.138, 24.261, and 26.391, more preferably at 6.915, 9.177, 9.984, 11.012, 13.595, 15.174, 16.156, 18.509, 20.138, 22.954, 24.261, 26.391, and 27.514; most preferably, the crystal form T has the X-ray powder diffraction pattern represented by the diffraction angle 2θ as shown in Figure 25.

**25.** The crystal form according to any one of claims 1 to 24, wherein 2θ angle has an error range of ± 0.20.

**26.** A pharmaceutical composition prepared from the crystal form according to any one of claims 1 to 25.

**27.** A pharmaceutical composition comprising the crystal form according to any one of claims 1 to 25, and optionally a pharmaceutically acceptable carrier, a diluent, or an excipient.

**28.** A method for preparing a pharmaceutical composition, comprising the step of mixing the crystal form according to any one of claims 1 to 25 with a pharmaceutically acceptable carrier, a diluent, or an excipient.

**29.** A use of the crystal form according to any one of claims 1 to 25, the composition according to claim 26 or 27, or the composition prepared by the method according to claim 28 in the manufacture of a medicament for treating and/or preventing a CRBN protein-related disease.

**30.** A use of the crystal form according to any one of claims 1 to 25, the composition according to claim 26 or 27, or the composition prepared by the method according to claim 28 in the manufacture of a medicament for treating and/or preventing a cancer, an angiogenesis-related condition, pain, macular degeneration or related syndrome, a skin disease, a pulmonary disease, an asbestos-related disease, a parasitic disease, an immunodeficiency disease, a CNS disease, a CNS injury, atherosclerosis or related condition, sleep disorder or related condition, an infectious disease, hemoglobinopathy or related condition, or a TNFa-related condition; preferably, a use in the manufacture of a medicament for treating and/or preventing a cancer or a CNS injury.

**31.** The use according to claim 30, wherein the cancer is selected from the group consisting of leukemia, myeloma, lymphoma, melanoma, skin cancer, liver cancer, kidney cancer, lung cancer, nasopharyngeal cancer, gastric cancer, esophageal cancer, colorectal cancer, gallbladder cancer, bile duct cancer, chorionic epithelioma, pancreatic cancer, polycythemia vera, pediatric tumor, cervical cancer, ovarian cancer, breast cancer, bladder cancer, urothelial cancer, ureteral tumor, prostate cancer, seminoma, testicular tumor, head and neck tumor, head and neck squamous cell carcinoma, endometrial cancer, thyroid cancer, sarcoma, osteoma, neuroblastoma, neuroendocrine cancer, brain tumor, CNS cancer, astrocytoma, and glioma; preferably, the liver cancer is hepatocellular carcinoma; the colorectal cancer is colon cancer or rectal cancer; the sarcoma is osteosarcoma or soft tissue sarcoma; and the glioma is glioblastoma.

**32.** The use according to claim 30, wherein the myeloma is multiple myeloma (MM) and myelodysplastic syndrome (MDS); preferably, the multiple myeloma is relapsed, refractory, or resistant.

**33.** The use according to claim 32, wherein the multiple myeloma is refractory or resistant to lenalidomide or pomalidomide.

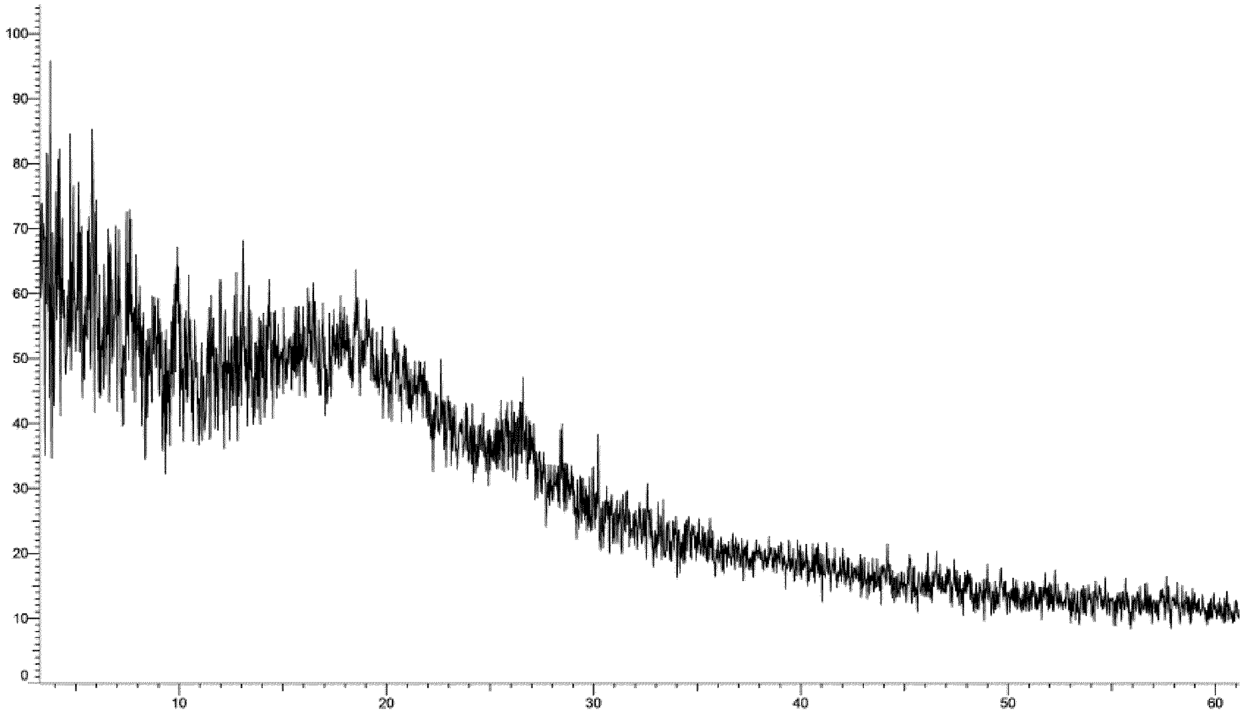


Figure 1

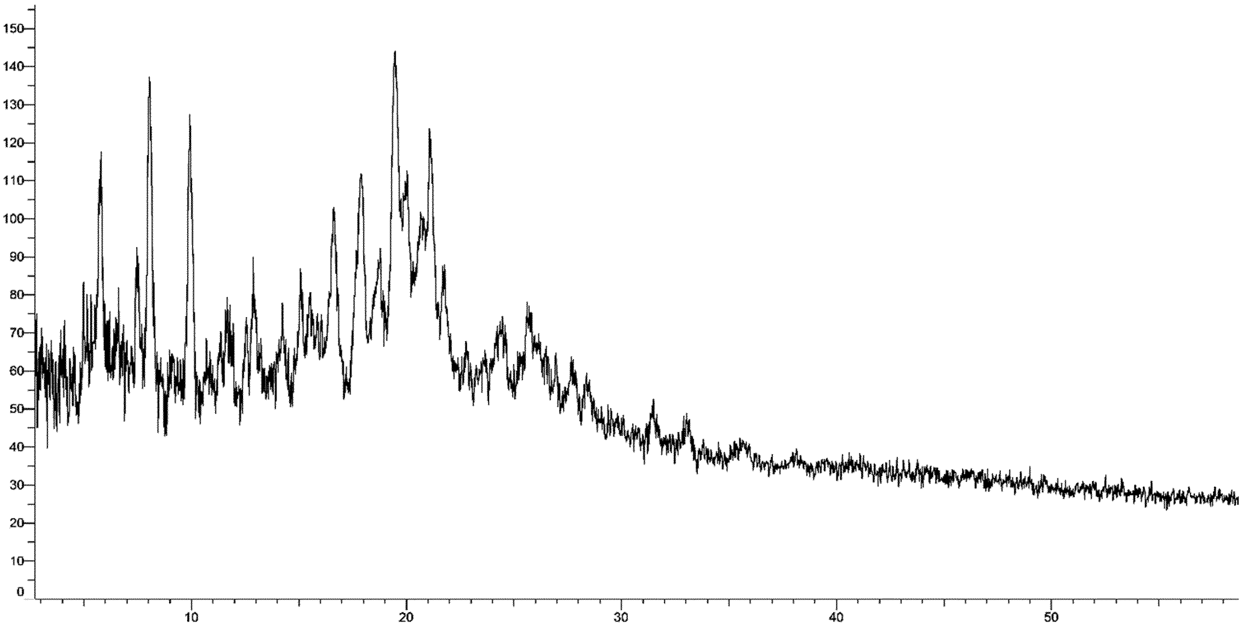


Figure 2

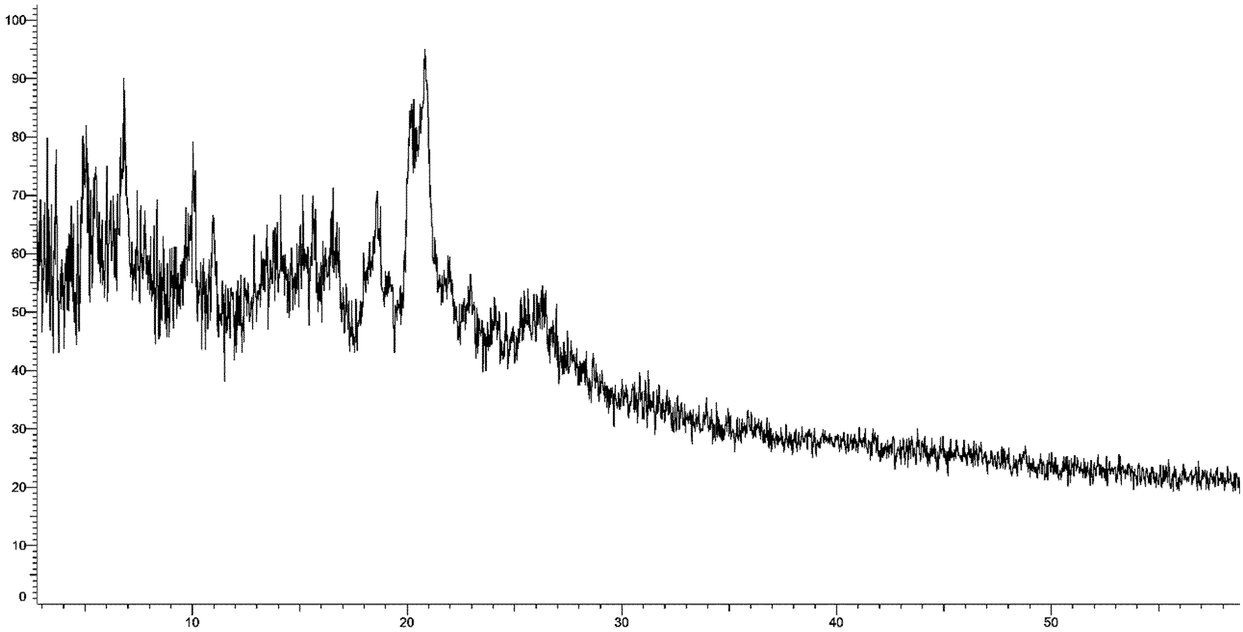


Figure 3

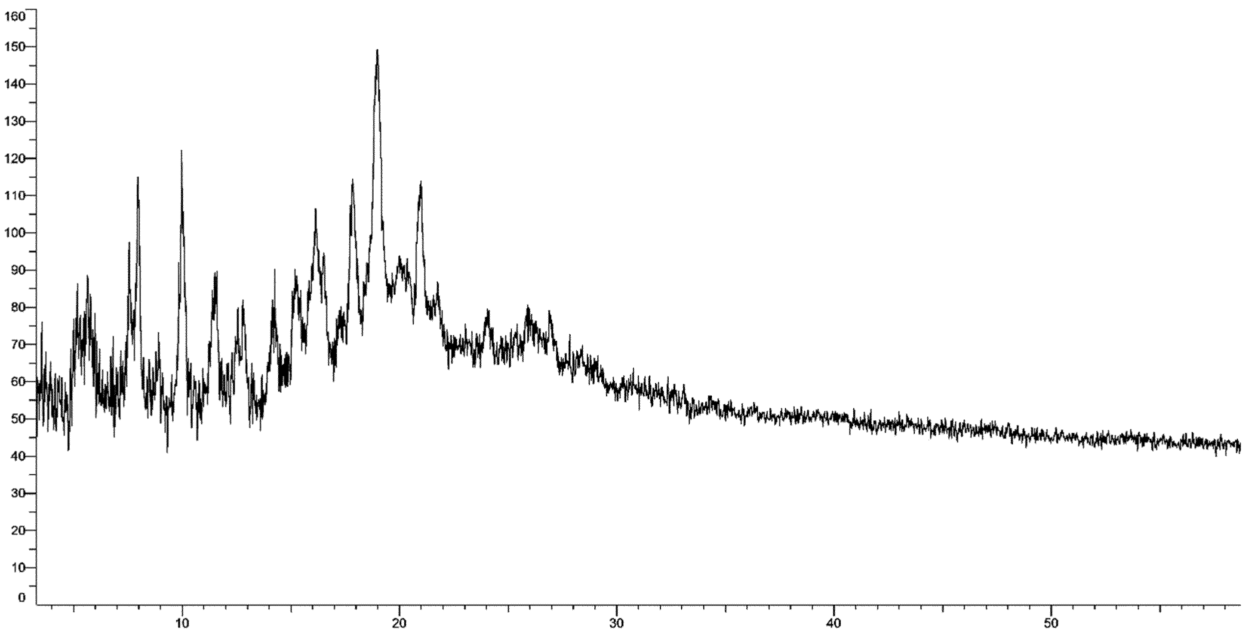
160
150
140
130
120
110
100
90
80
70
60
50
40
30
20
10
0
10
20
30
40
50

Figure 4

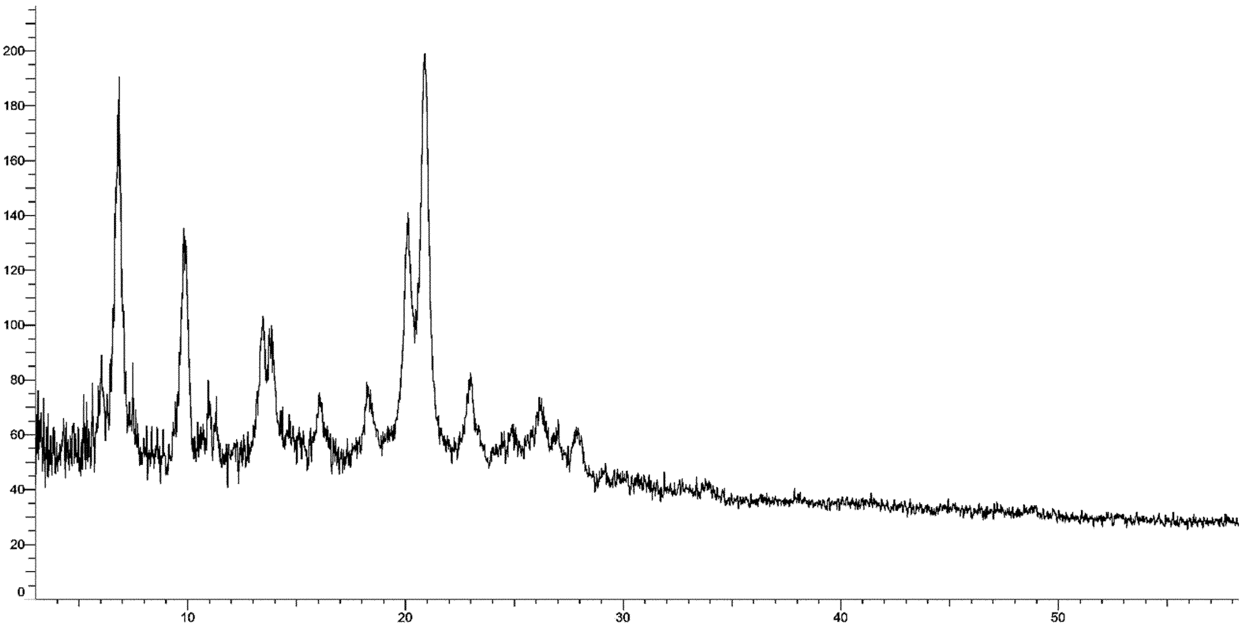


Figure 5

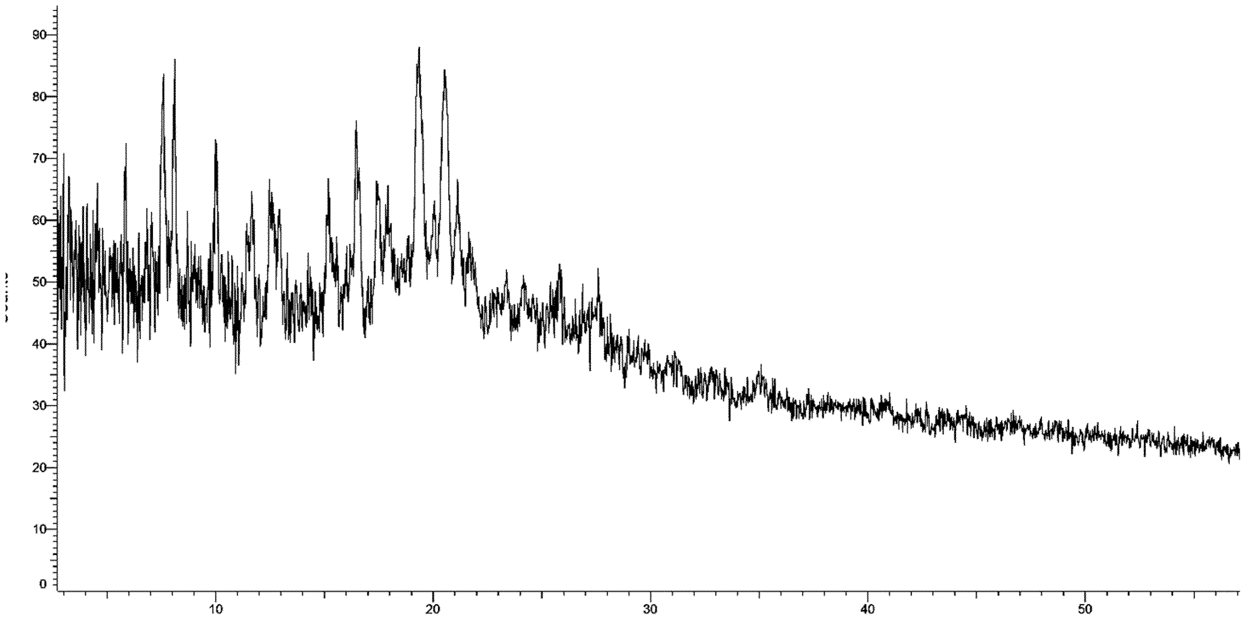


Figure 6

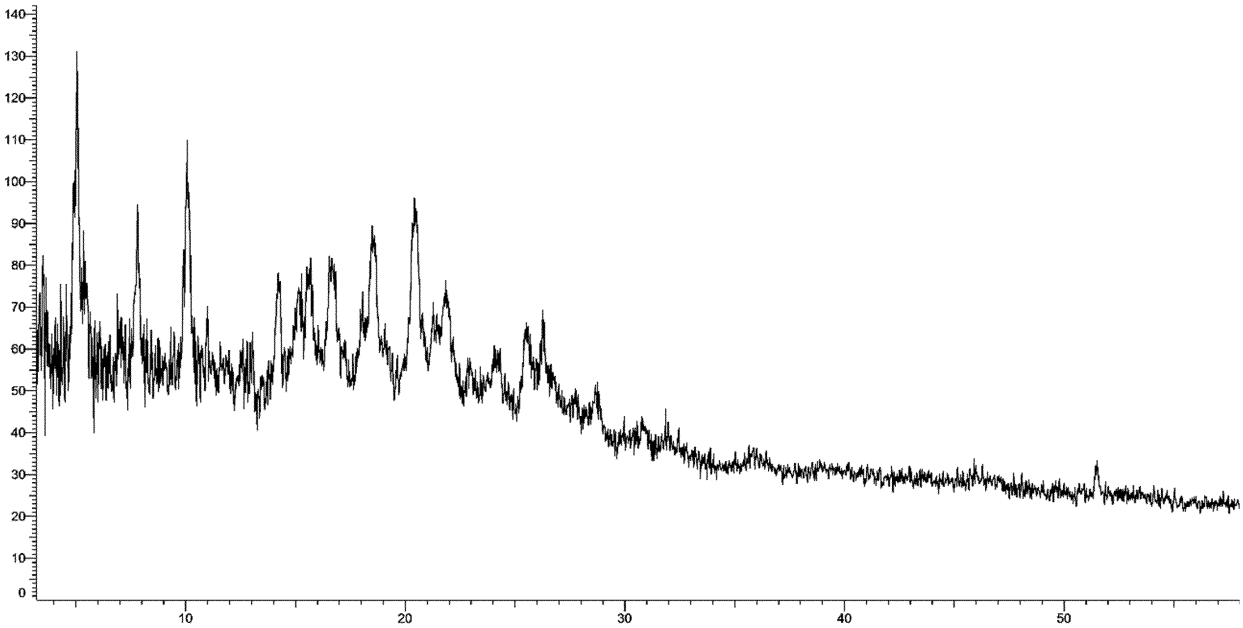


Figure 7

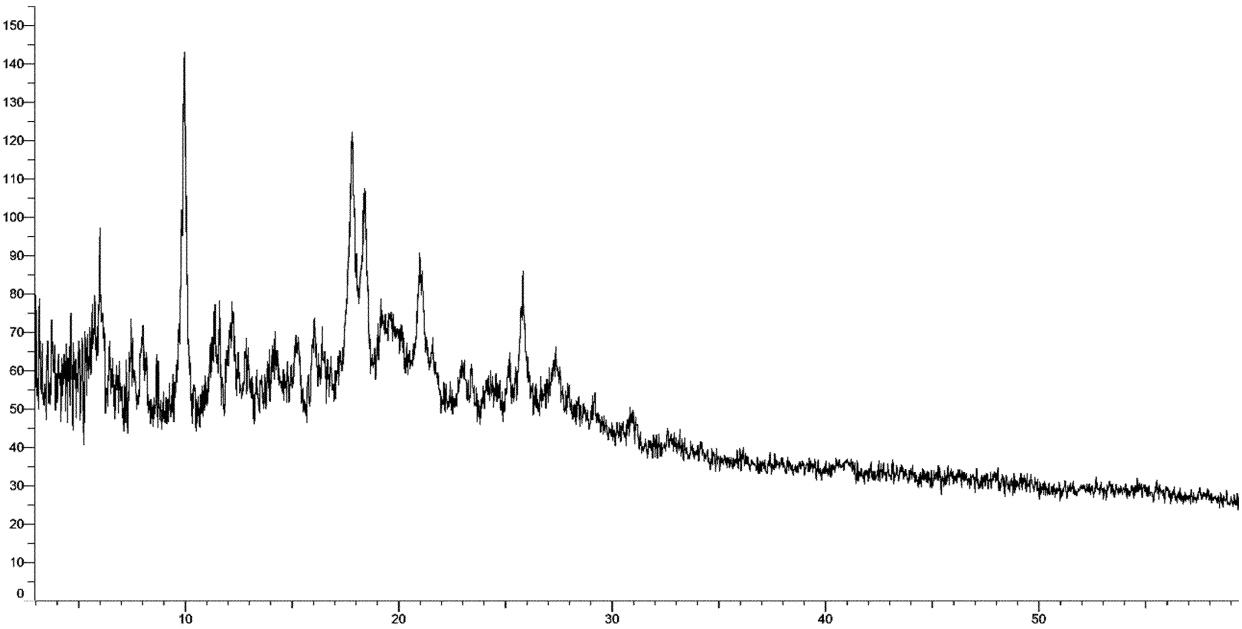


Figure 8

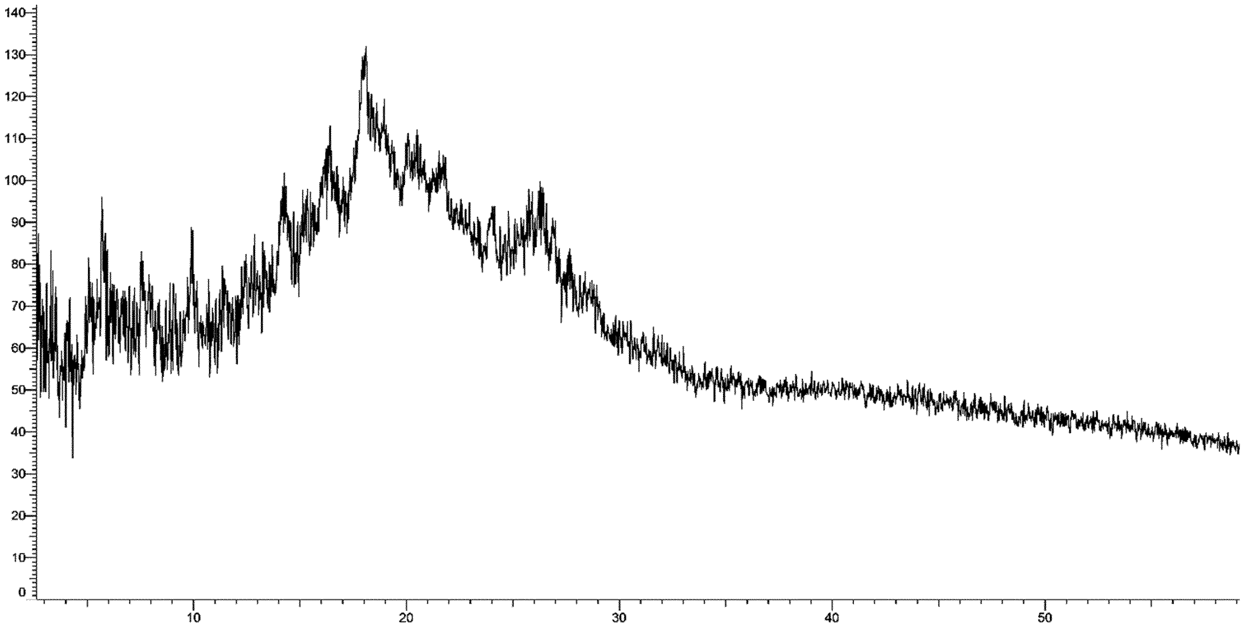


Figure 9

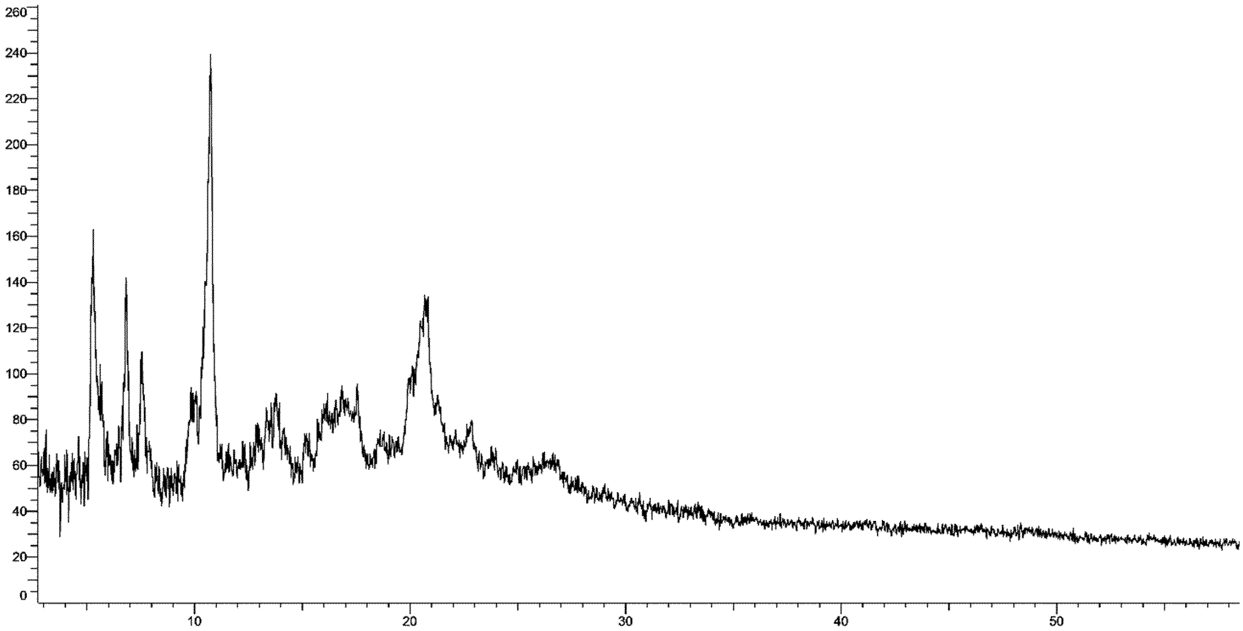


Figure 10

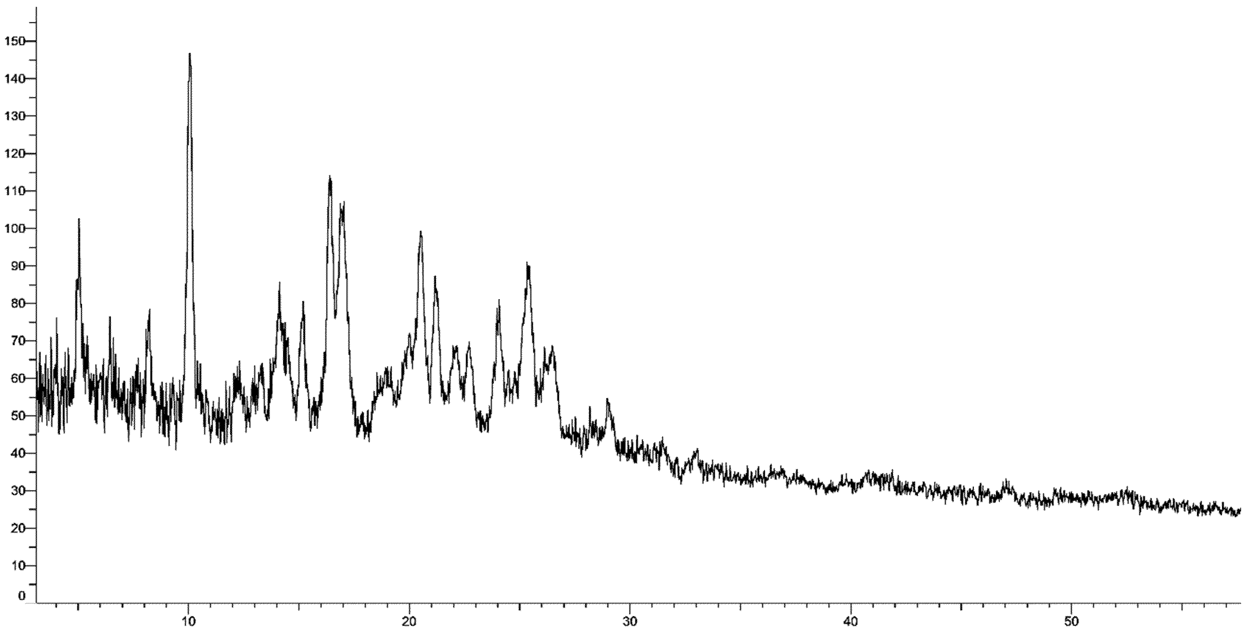


Figure 11

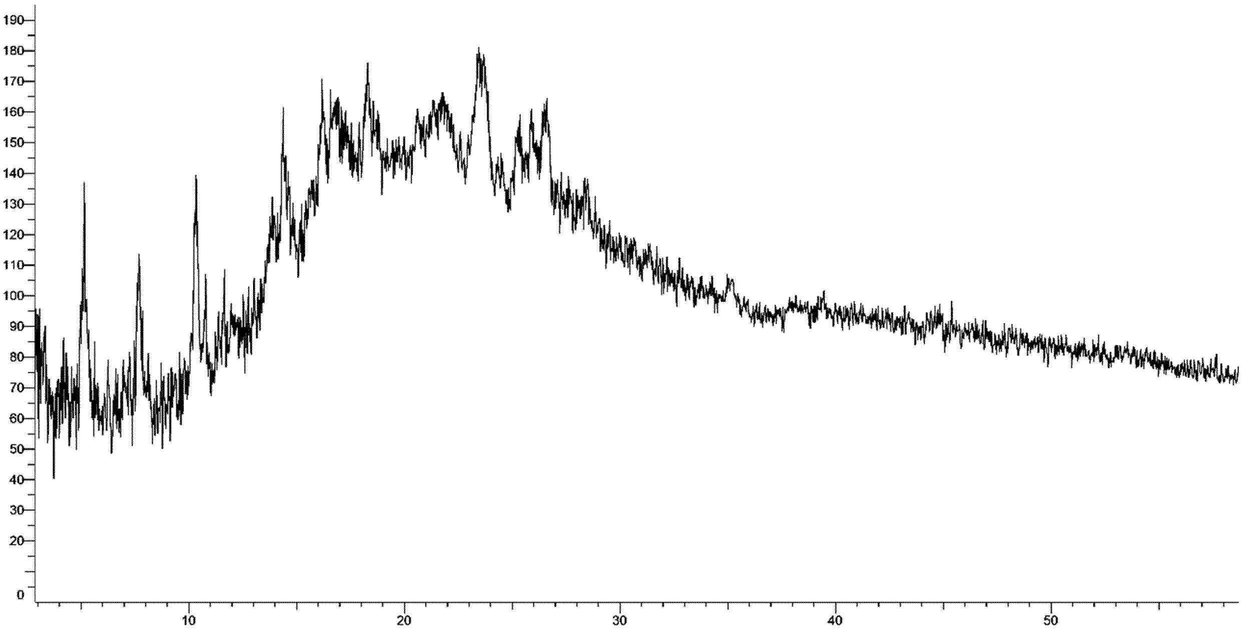
190
180
170
160
150
140
130
120
110
100
90
80
70
60
50
40
30
20
0
10
20
30
40
50

Figure 12

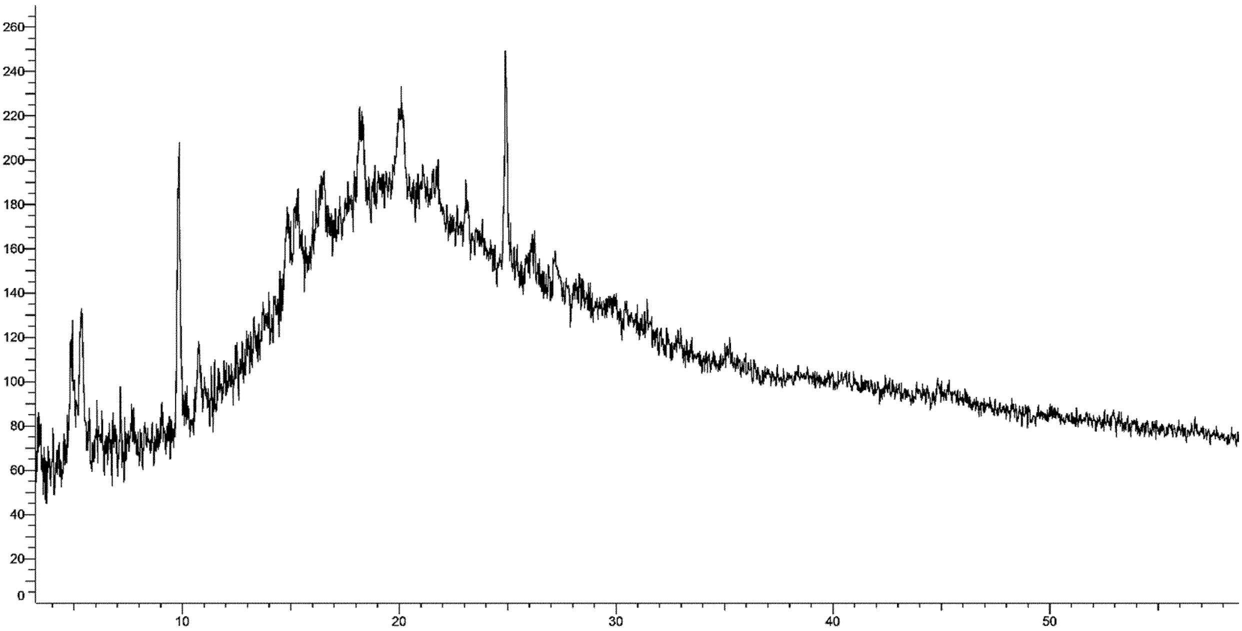


Figure 13

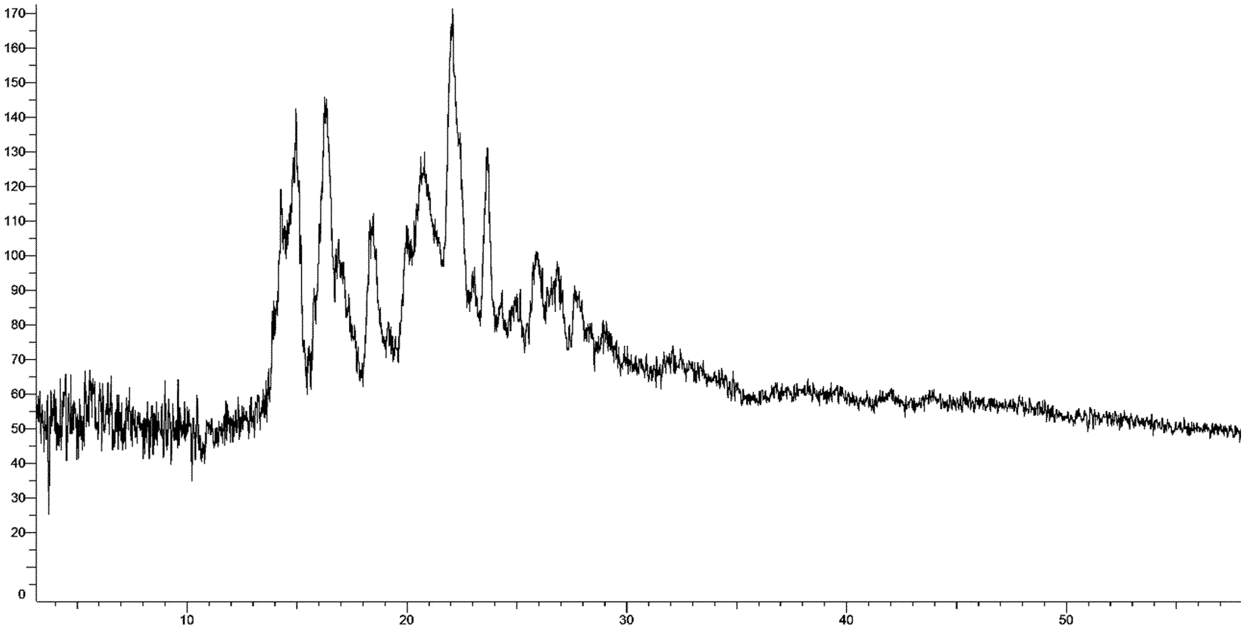
170
160
150
140
130
120
110
100
90
80
70
60
50
40
30
20
0
10
20
30
40
50

Figure 14

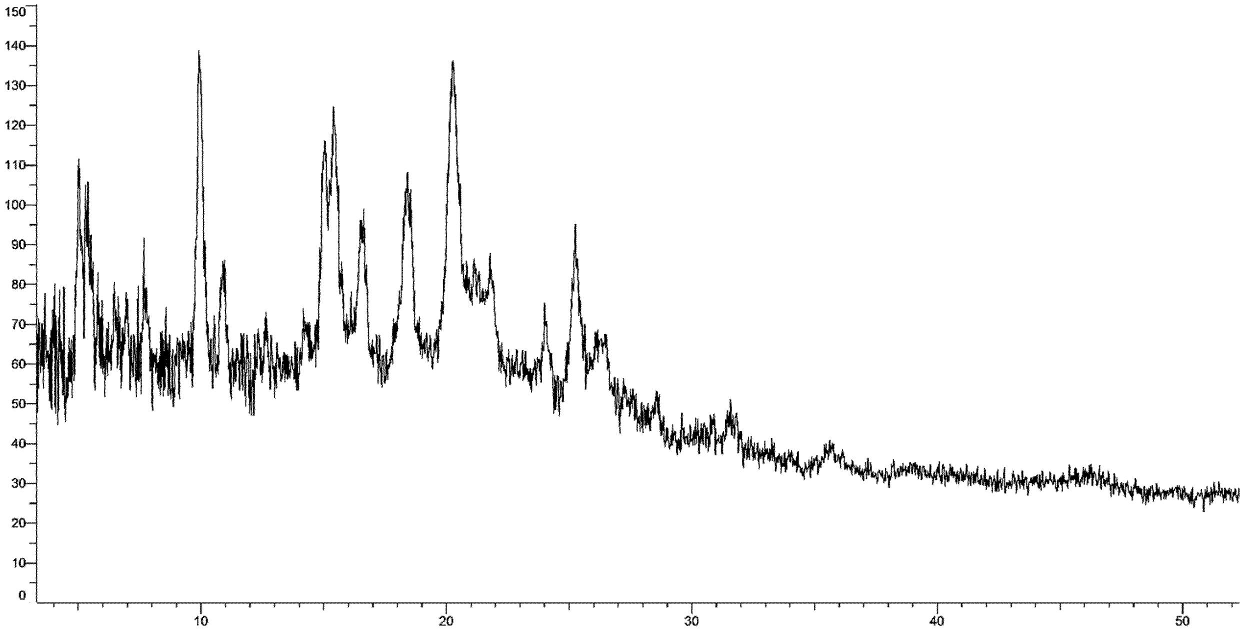


Figure 15

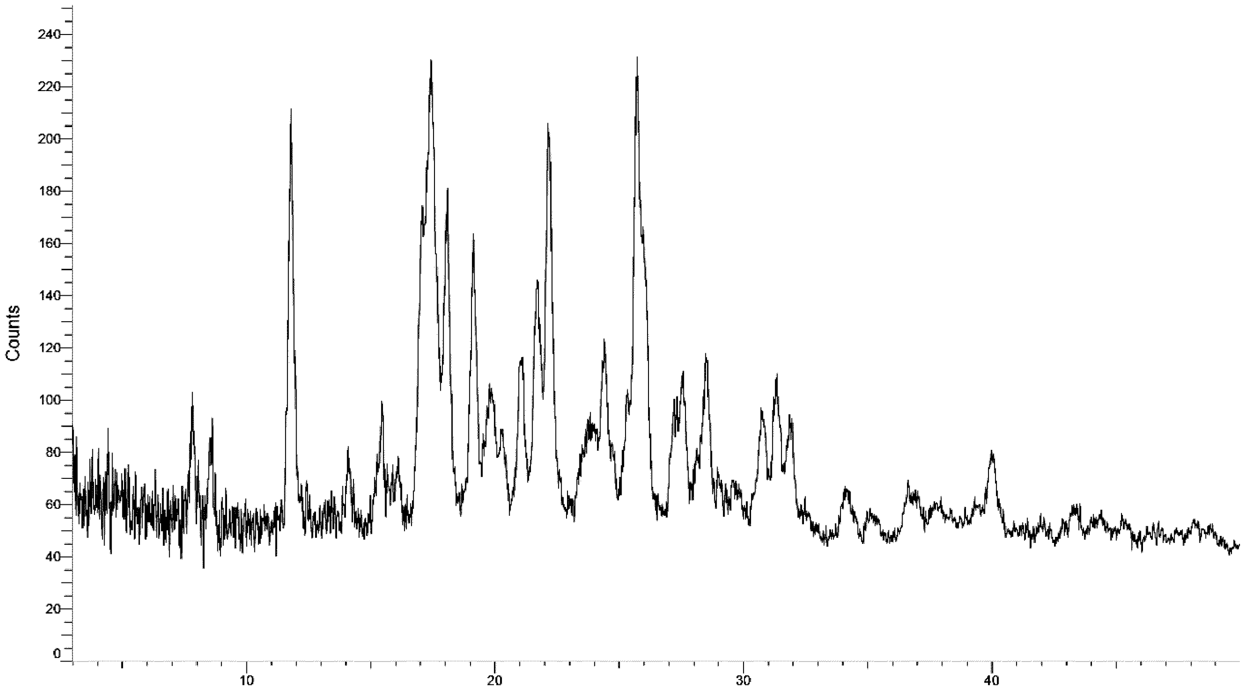


Figure 16

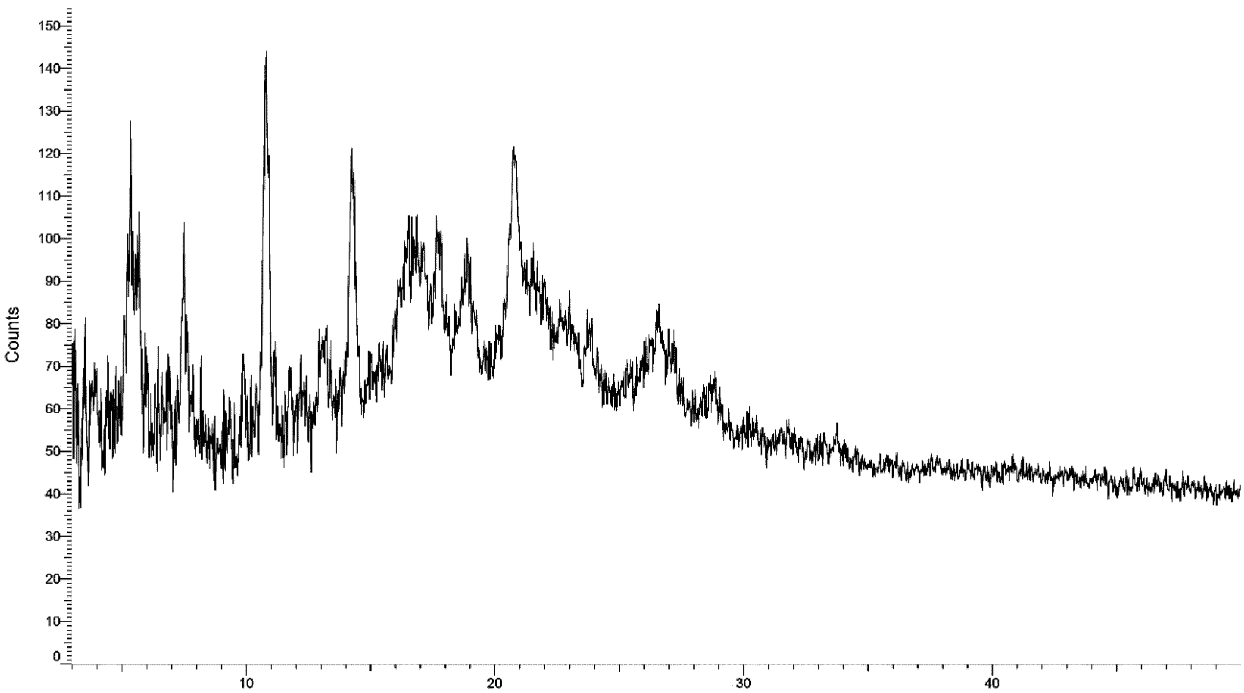
Counts
150
140
130
120
110
100
90
80
70
60
50
40
30
20
10
0
10
20
30
40

Figure 17

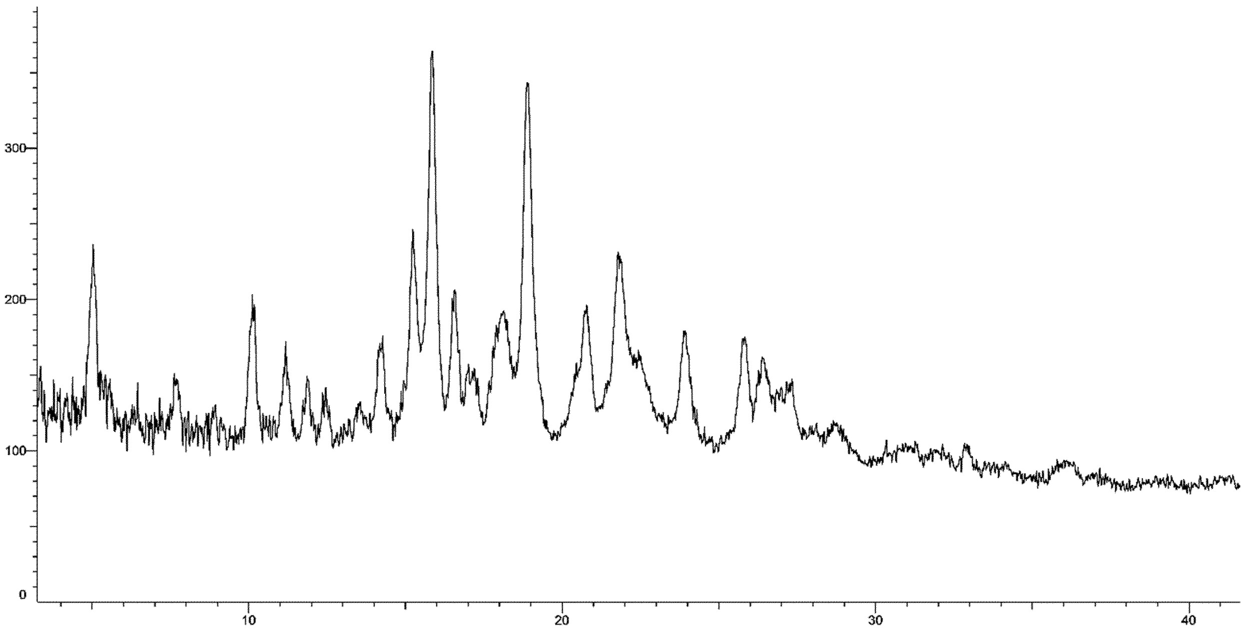


Figure 18

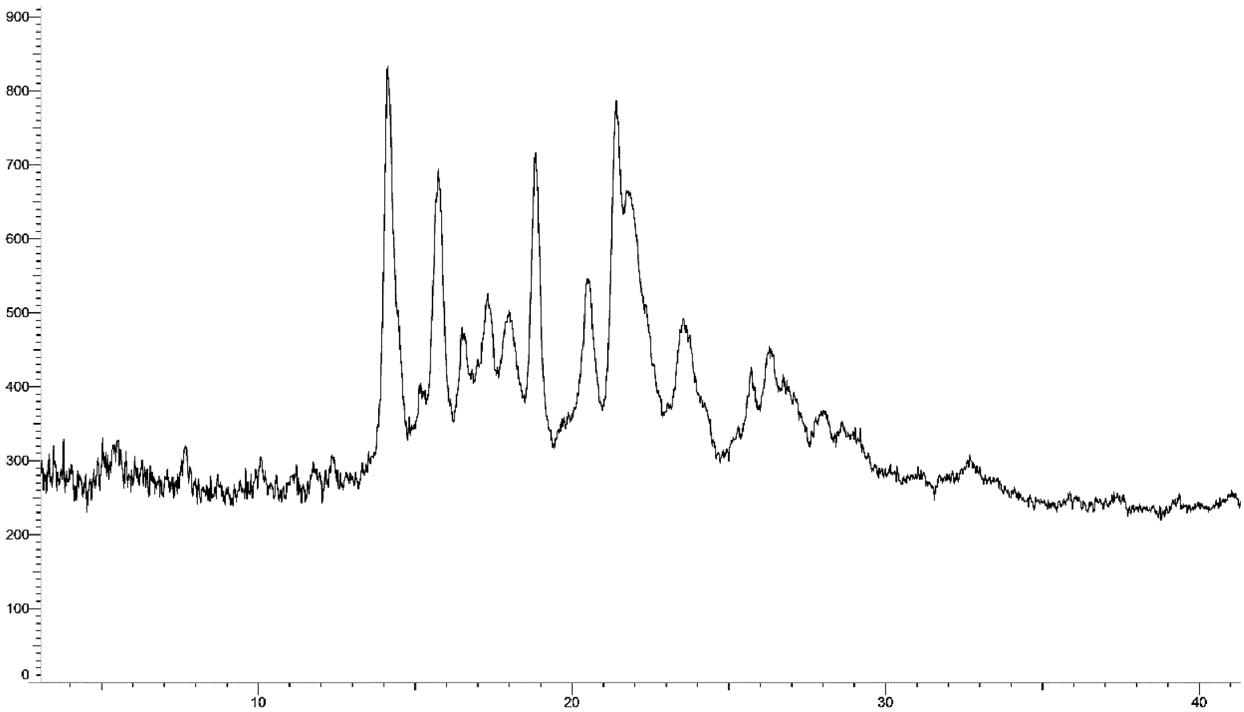


Figure 19

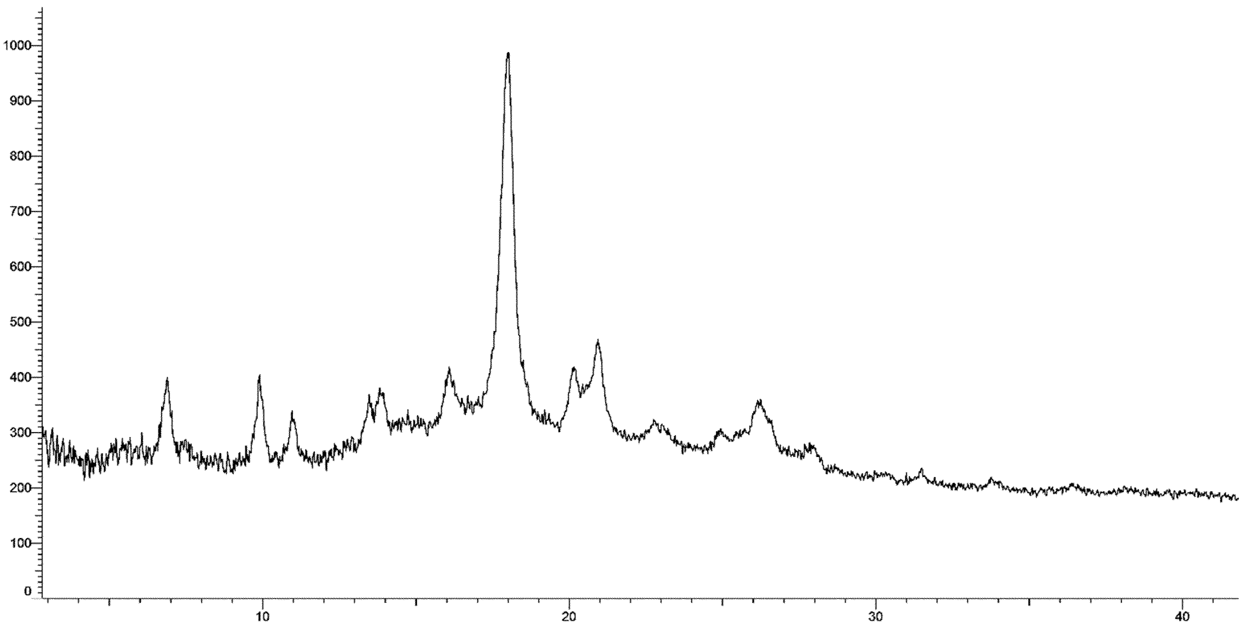


Figure 20

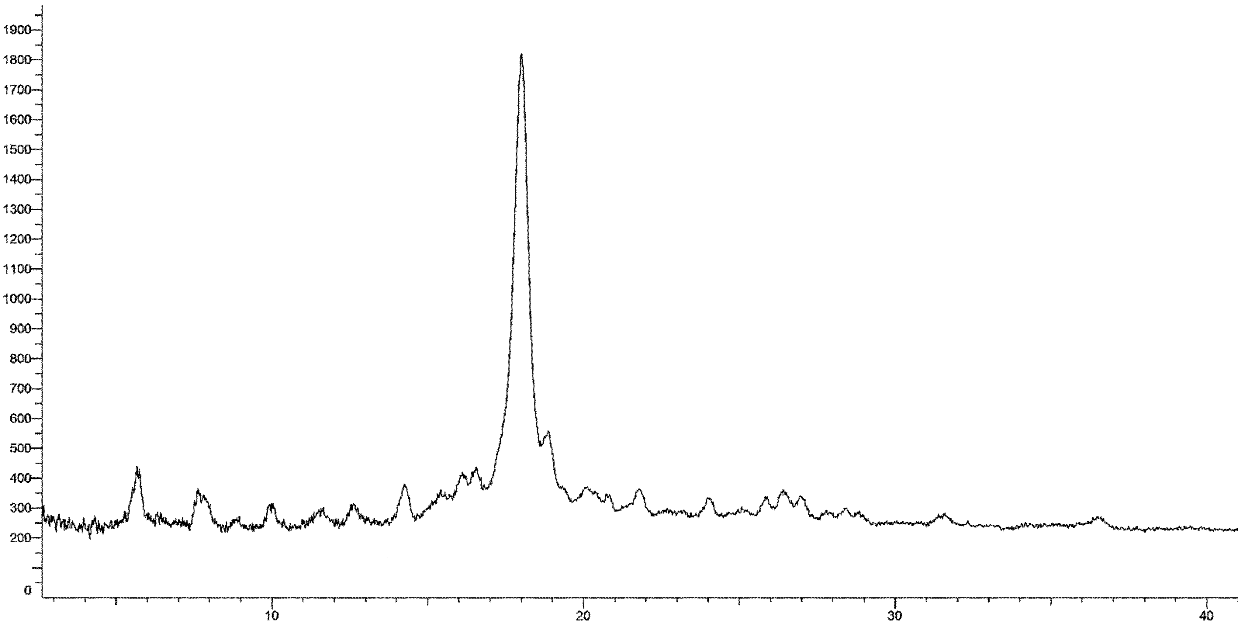
1900
1800
1700
1600
1500
1400
1300
1200
1100
1000
900
800
700
600
500
400
300
200
0
10
20
30
40

Figure 21

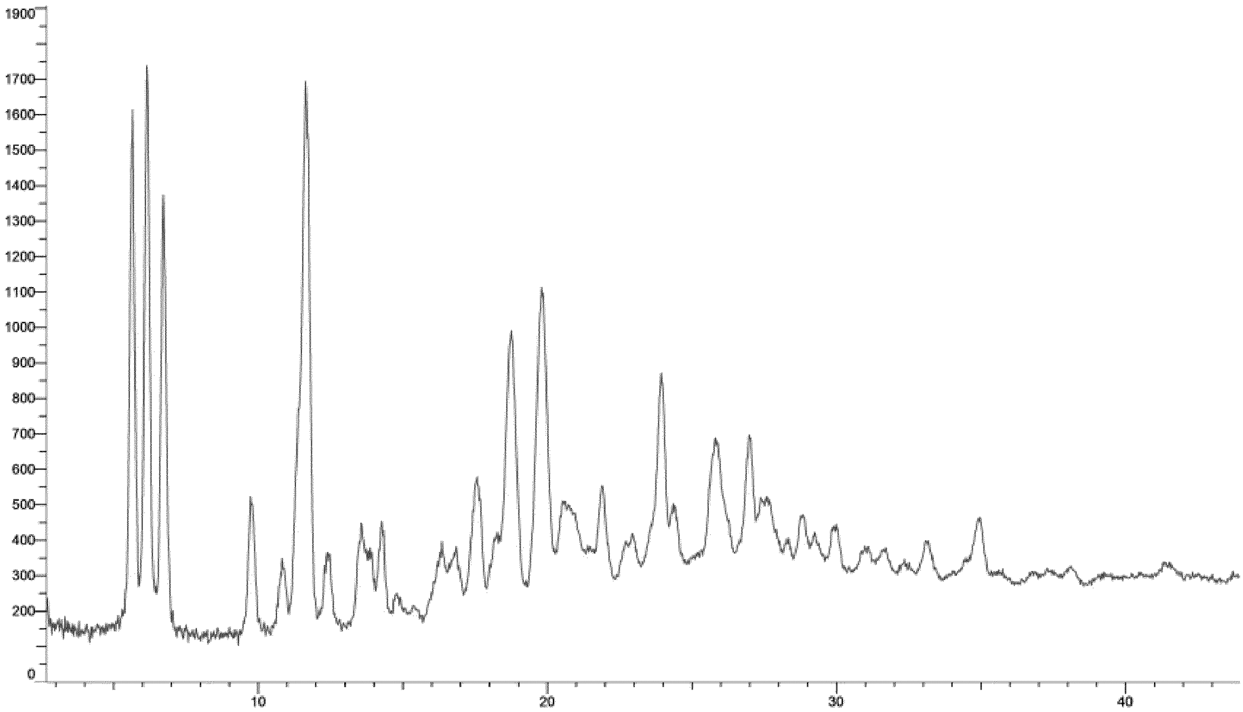
1900
1700
1600
1500
1400
1300
1200
1100
1000
900
800
700
600
500
400
300
200
0
10
20
30
40

Figure 22

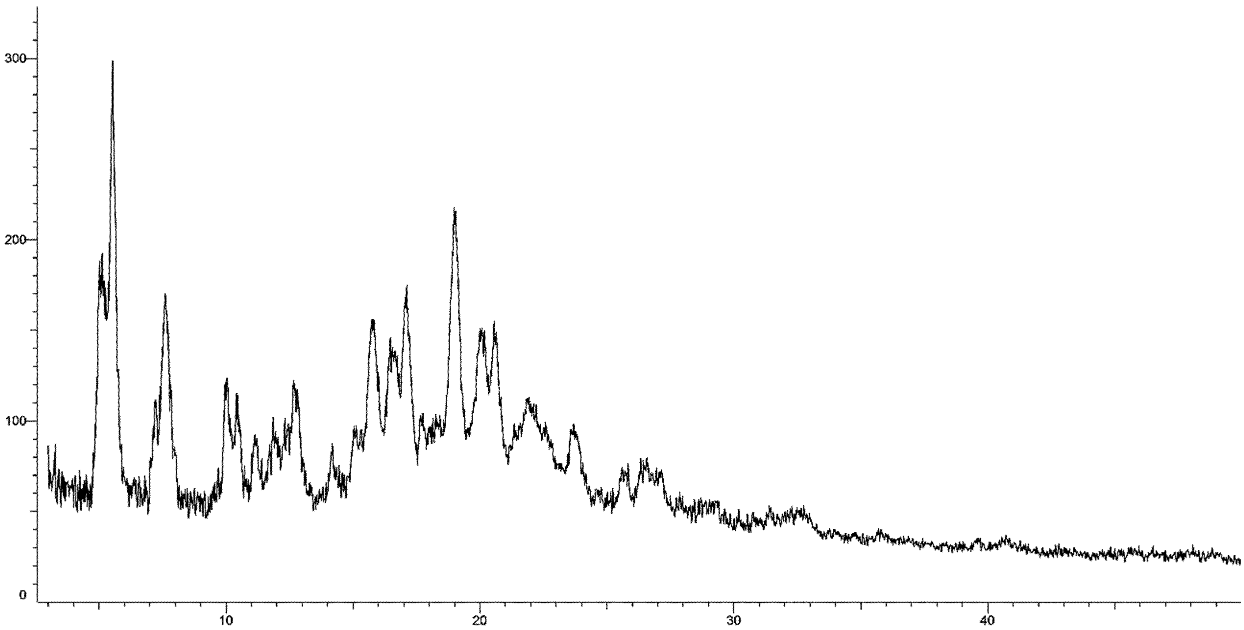


Figure 23

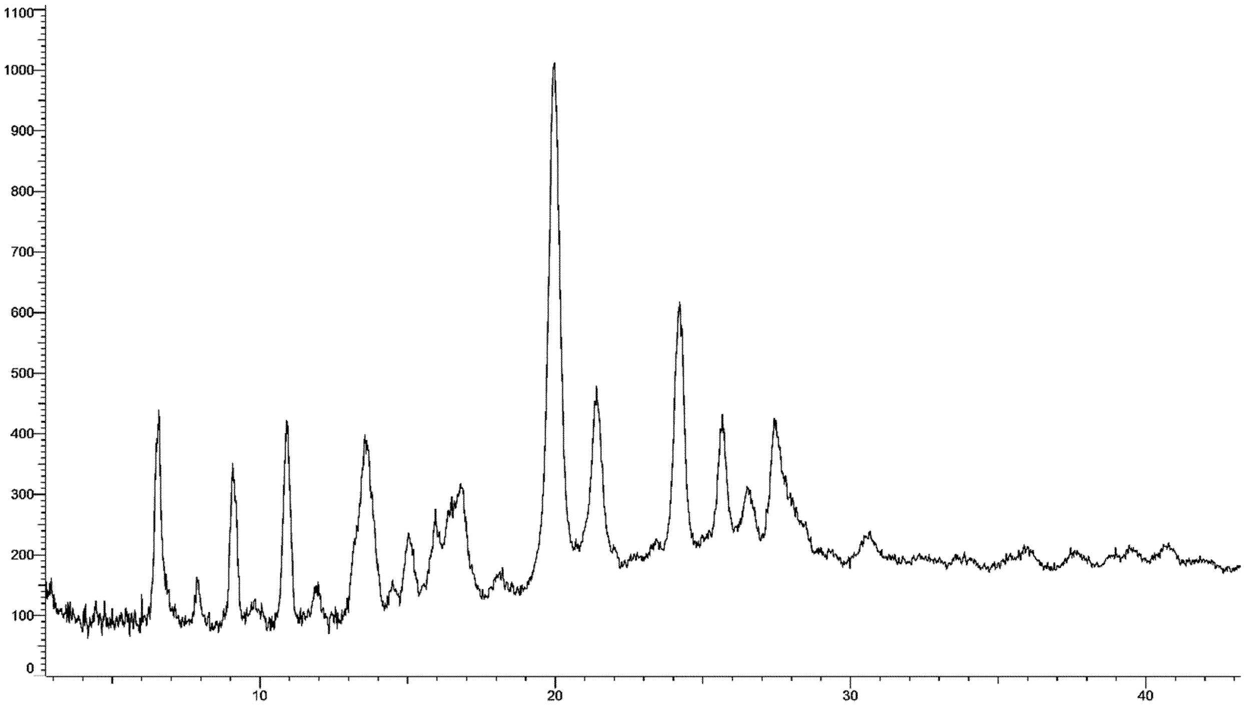


Figure 24

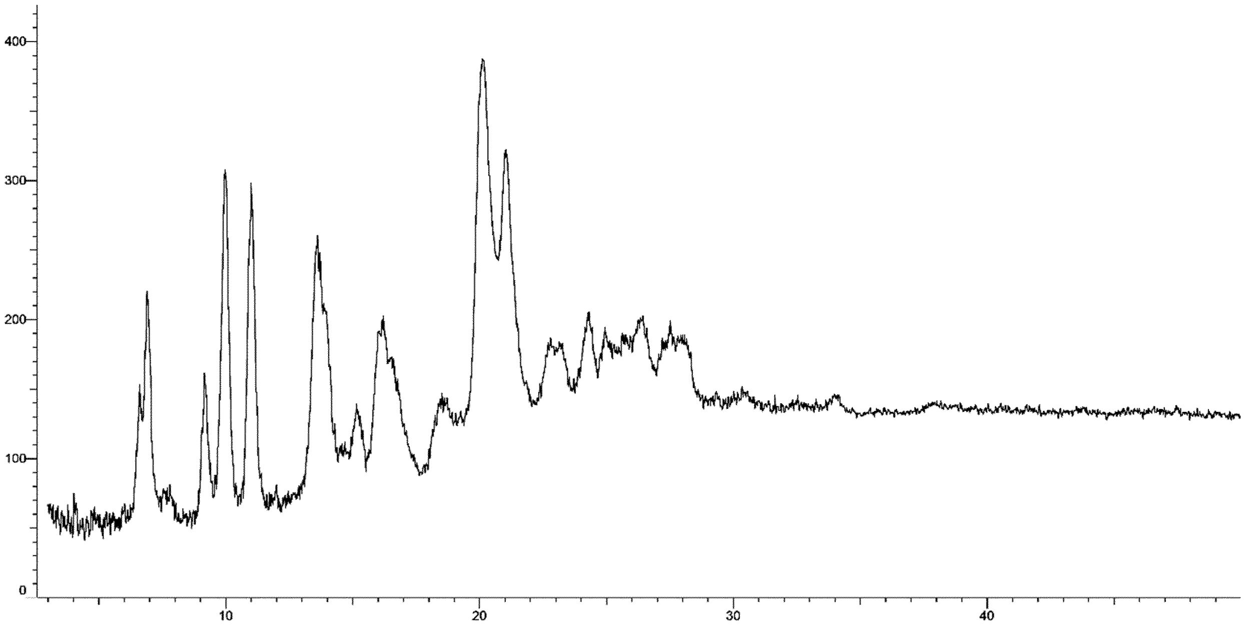


Figure 25

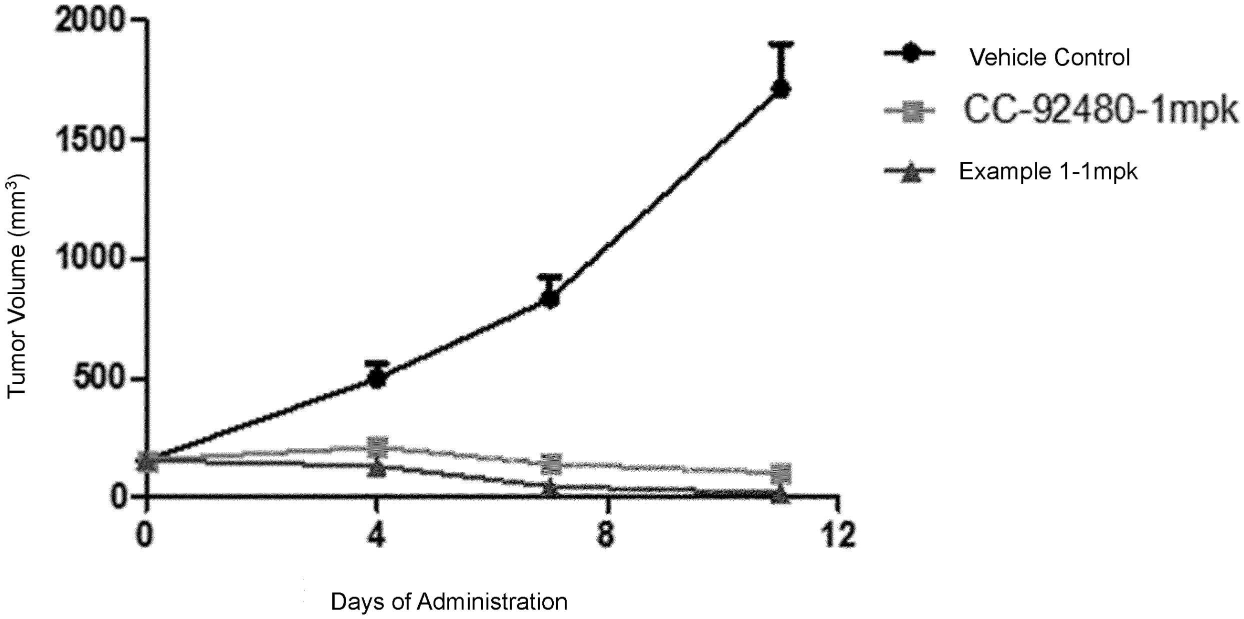
2000
1500
1000
500
0
Tumor Volume (mm3)
0
4
8
12
Days of Administration
Vehicle Control
CC-92480-1mpk
Example 1-1mpk

Figure 26

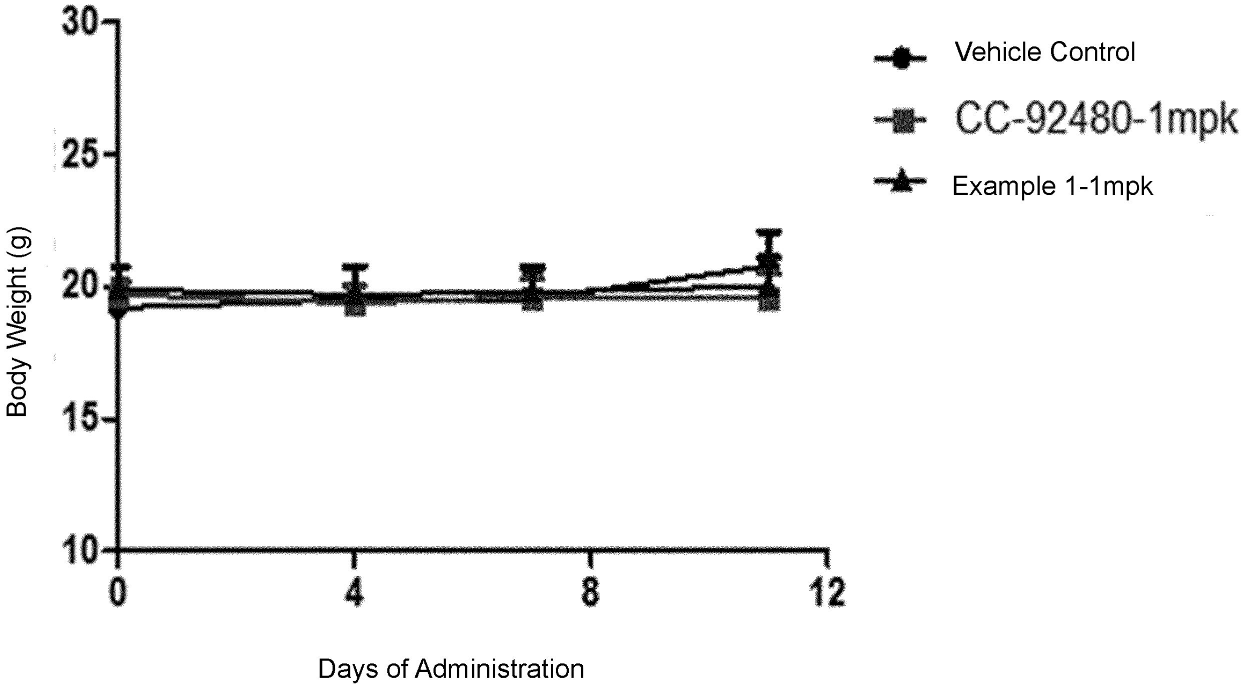
30
25
20
15
10
Body Weight (g)
0
4
8
12
Days of Administration
Vehicle Control
CC-92480-1mpk
Example 1-1mpk

Figure 27

**INTERNATIONAL SEARCH REPORT**

International application No.
**PCT/CN2023/073077**

**A. CLASSIFICATION OF SUBJECT MATTER**

C07D401/14(2006.01)i;A61K31/454(2006.01)i;A61P35/00(2006.01)i

According to International Patent Classification (IPC) or to both national classification and IPC

**B. FIELDS SEARCHED**

Minimum documentation searched (classification system followed by classification symbols)

C07D401/- A61K31/- A61P35/-

Documentation searched other than minimum documentation to the extent that such documents are included in the fields searched

Electronic data base consulted during the international search (name of data base and, where practicable, search terms used)

CNKI, CNABS, VEN, DWPI, STN(REGISTRY, CAPLUS): 江苏恒瑞医药股份有限公司, 上海恒瑞医药有限公司, 异吲哚啉, 多发性骨髓瘤, 癌症, 肿瘤, isoindoline, cereblon, myeloma, tumour, tumor, cancer, 2758028-21-4, 2758028-22-5, 2758028-23-6, 基于权利要求1的structural formula search based on claim 1

**C. DOCUMENTS CONSIDERED TO BE RELEVANT**

| Category* | Citation of document, with indication, where appropriate, of the relevant passages | Relevant to claim No. |
|---|---|---|
| PX | WO 2022017365 A1 (JIANGSU HENGRUI MEDICINE CO., LTD. et al.) 27 January 2022 (2022-01-27)<br>claims 1, 15 and 22-27, and description, p. 15, compound 6 | 1-33 |
| A | CN 112492874 A (CELGENE CORP.) 12 March 2021 (2021-03-12)<br>entire document | 1-33 |
| A | CN 102822165 A (CELGENE CORP.) 12 December 2012 (2012-12-12)<br>entire document | 1-33 |
| A | CN 110869021 A (CELGENE CORP.) 06 March 2020 (2020-03-06)<br>entire document | 1-33 |
| A | WO 2008115516 A2 (CELGENE CORP. et al.) 25 September 2008 (2008-09-25)<br>entire document | 1-33 |
| A | WO 2021055756 A1 (THE REGENTS OF THE UNIVERSITY OF MICHIGAN) 25 March 2021 (2021-03-25)<br>entire document | 1-33 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 May 2023** | **10 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/073077**

| Patent document cited in search report | Publication date (day/month/year) | Patent family member(s) | Publication date (day/month/year) |
|---|---|---|---|
| WO 2022017365 A1 | 27 January 2022 | BR 112023000817 A2 | 07 February 2023 |
| | | CA 3184711 A1 | 27 January 2022 |
| | | TW 202214594 A | 16 April 2022 |
| | | AU 2021314401 A1 | 09 March 2023 |
| | | KR 20230042057 A | 27 March 2023 |
| CN 112492874 A | 12 March 2021 | AU 2019272751 A1 | 10 December 2020 |
| | | MX 2020012548 A | 13 May 2021 |
| | | CA 3101174 A1 | 28 November 2019 |
| | | IL 278854 A | 31 January 2021 |
| | | SG 11202011568 XA | 30 December 2020 |
| | | US 2019361005 A1 | 28 November 2019 |
| | | US 10969381 B2 | 06 April 2021 |
| | | WO 2019226770 A1 | 28 November 2019 |
| | | EP 3796909 A1 | 31 March 2021 |
| | | EP 3796909 A4 | 16 March 2022 |
| | | BR 112020023756 A2 | 09 February 2021 |
| | | KR 20210024454 A | 05 March 2021 |
| | | JP 2021525362 A | 24 September 2021 |
| | | US 2021181184 A1 | 17 June 2021 |
| CN 102822165 A | 12 December 2012 | ES 2730763 T3 | 12 November 2019 |
| | | HUE 033009 T2 | 28 November 2017 |
| | | PT 2536706 T | 20 July 2017 |
| | | NZ 717149 A | 30 June 2017 |
| | | RU 2012138709 A | 20 March 2014 |
| | | RU 2567753 C2 | 10 November 2015 |
| | | US 2020231567 A1 | 23 July 2020 |
| | | US 11414399 B2 | 16 August 2022 |
| | | SI 3202461 T1 | 31 May 2019 |
| | | ES 2738776 T3 | 27 January 2020 |
| | | AR 081058 A1 | 06 June 2012 |
| | | US 2022411402 A1 | 29 December 2022 |
| | | EP 3599236 A1 | 29 January 2020 |
| | | SG 183257 A1 | 27 September 2012 |
| | | CY 1119177 T1 | 14 February 2018 |
| | | HUE 042011 T2 | 28 June 2019 |
| | | SI 2536706 T1 | 30 October 2017 |
| | | NZ 700054 A | 31 March 2016 |
| | | EP 3202460 A1 | 09 August 2017 |
| | | EP 3202460 B1 | 12 June 2019 |
| | | LT 2536706 T | 25 October 2017 |
| | | JP 2016172746 A | 29 September 2016 |
| | | JP 2013519675 A | 30 May 2013 |
| | | HRP 20190368 T1 | 19 April 2019 |
| | | ECSP 12012098 A | 30 October 2012 |
| | | CO 6571916 A2 | 30 November 2012 |
| | | ES 2713482 T3 | 22 May 2019 |
| | | KR 20130010888 A | 29 January 2013 |
| | | KR 101812356 B1 | 26 December 2017 |
| | | US 2016159768 A1 | 09 June 2016 |
| | | US 9828361 B2 | 28 November 2017 |

Form PCT/ISA/210 (patent family annex) (July 2022)

**INTERNATIONAL SEARCH REPORT**
**Information on patent family members**

International application No.
**PCT/CN2023/073077**

| Patent document cited in search report | Publication date (day/month/year) | Patent family member(s) | | | Publication date (day/month/year) |
|---|---|---|---|---|---|
| | | EP | 3202461 | A1 | 09 August 2017 |
| | | EP | 3202461 | B1 | 26 December 2018 |
| | | ME | 03441 | B | 20 January 2020 |
| | | US | 2018037567 | A1 | 08 February 2018 |
| | | US | 10189814 | B2 | 29 January 2019 |
| | | UA | 115220 | C2 | 10 October 2017 |
| | | IL | 248843 | A0 | 31 January 2017 |
| | | IL | 248843 | B | 31 December 2019 |
| | | US | 2013324518 | A1 | 05 December 2013 |
| | | US | 9309219 | B2 | 12 April 2016 |
| | | TR | 201903027 | T4 | 21 March 2019 |
| | | HRP | 20171078 | T1 | 06 October 2017 |
| | | NI | 201200132 | A | 22 April 2013 |
| | | MX | 367522 | B | 26 August 2019 |
| | | US | 2019135780 | A1 | 09 May 2019 |
| | | US | 10669257 | B2 | 02 June 2020 |
| | | US | 2016159772 | A1 | 09 June 2016 |
| | | US | 9822094 | B2 | 21 November 2017 |
| | | NZ | 601289 | A | 31 October 2014 |
| | | DK | 2536706 | T3 | 14 August 2017 |
| | | ME | 02766 | B | 20 January 2018 |
| | | PL | 2536706 | T3 | 31 October 2017 |
| | | MX | 337169 | B | 16 February 2016 |
| | | RS | 56232 | B1 | 30 November 2017 |
| | | RS | 59275 | B1 | 31 October 2019 |
| | | UA | 114856 | C2 | 10 August 2017 |
| | | PL | 3202461 | T3 | 31 July 2019 |
| | | DK | 3202461 | T3 | 18 March 2019 |
| | | PT | 3202460 | T | 05 August 2019 |
| | | RS | 58523 | B1 | 30 April 2019 |
| | | SG | 10202012179 | RA | 28 January 2021 |
| | | HRP | 20191312 | T1 | 18 October 2019 |
| | | IL | 220992 | A | 30 November 2016 |
| | | LT | 3202460 | T | 10 October 2019 |
| | | JP | 2017031165 | A | 09 February 2017 |
| | | JP | 6270944 | B2 | 31 January 2018 |
| | | PH | 12014501082 | A1 | 28 September 2015 |
| | | SI | 3202460 | T1 | 30 October 2019 |
| | | EP | 2536706 | A1 | 26 December 2012 |
| | | EP | 2536706 | B1 | 14 June 2017 |
| | | ES | 2638517 | T3 | 23 October 2017 |
| | | JP | 2017200944 | A | 09 November 2017 |
| | | KR | 20180000338 | A | 02 January 2018 |
| | | KR | 101931468 | B1 | 20 December 2018 |
| | | MX | 2012009237 | A | 23 August 2012 |
| | | DK | 3202460 | T3 | 29 July 2019 |
| | | AU | 2011215877 | A1 | 16 August 2012 |
| | | AU | 2011215877 | B2 | 07 November 2013 |
| | | AU | 2011215877 | C1 | 19 January 2017 |
| | | SG | 10201501062 | SA | 29 April 2015 |

Form PCT/ISA/210 (patent family annex) (July 2022)

**INTERNATIONAL SEARCH REPORT**
**Information on patent family members**

International application No.
**PCT/CN2023/073077**

| Patent document cited in search report | Publication date (day/month/year) | Patent family member(s) | Publication date (day/month/year) |
|---|---|---|---|
| | | PL 3202460 T3 | 31 December 2019 |
| | | HUE 044652 T2 | 28 November 2019 |
| | | CA 2787823 A1 | 18 August 2011 |
| | | CA 2787823 C | 10 April 2018 |
| | | PT 3202461 T | 19 March 2019 |
| | | CR 20120414 A | 31 October 2012 |
| | | US 2011196150 A1 | 11 August 2011 |
| | | US 8518972 B2 | 27 August 2013 |
| | | LT 3202461 T | 25 April 2019 |
| | | EP 3106460 A1 | 21 December 2016 |
| | | EP 3106460 B1 | 10 April 2019 |
| | | WO 2011100380 A1 | 18 August 2011 |
| | | JP 2016106106 A | 16 June 2016 |
| | | JP 6215976 B2 | 18 October 2017 |
| CN 110869021 A | 06 March 2020 | US 2022280505 A1 | 08 September 2022 |
| | | CL 2020000060 A1 | 31 July 2020 |
| | | US 2020253964 A1 | 13 August 2020 |
| | | US 11185543 B2 | 30 November 2021 |
| | | KR 20200026980 A | 11 March 2020 |
| | | TW 201907923 A | 01 March 2019 |
| | | IL 271889 A | 27 February 2020 |
| | | IL 271889 B | 01 November 2022 |
| | | CA 3069138 A1 | 17 January 2019 |
| | | ECSP 20001149 A | 28 February 2020 |
| | | SG 11202000143 PA | 27 February 2020 |
| | | US 2019282567 A1 | 19 September 2019 |
| | | US 10675281 B2 | 09 June 2020 |
| | | WO 2019014100 A1 | 17 January 2019 |
| | | JP 2020526534 A | 31 August 2020 |
| | | JP 7258009 B2 | 14 April 2023 |
| | | AU 2022287569 A1 | 02 February 2023 |
| | | ZA 202000157 B | 25 August 2021 |
| | | EP 3651766 A1 | 20 May 2020 |
| | | US 2019008852 A1 | 10 January 2019 |
| | | US 10357489 B2 | 23 July 2019 |
| | | AU 2018301335 A1 | 30 January 2020 |
| | | AU 2018301335 B2 | 15 September 2022 |
| | | BR 112020000442 A2 | 21 July 2020 |
| | | CO 2020000193 A2 | 24 April 2020 |
| WO 2008115516 A2 | 25 September 2008 | KR 20090121400 A | 25 November 2009 |
| | | US 2018155316 A1 | 07 June 2018 |
| | | US 10385037 B2 | 20 August 2019 |
| | | NZ 579890 A | 25 May 2012 |
| | | PE 20081894 A1 | 26 January 2009 |
| | | ZA 200906497 B | 24 November 2010 |
| | | EP 3101017 A1 | 07 December 2016 |
| | | EP 3101017 B1 | 12 June 2019 |
| | | US 2009004209 A1 | 01 January 2009 |
| | | US 8153659 B2 | 10 April 2012 |
| | | CL 2008000805 A1 | 22 May 2009 |

Form PCT/ISA/210 (patent family annex) (July 2022)

**INTERNATIONAL SEARCH REPORT**
**Information on patent family members**

International application No.
**PCT/CN2023/073077**

| Patent document cited in search report | Publication date (day/month/year) | Patent family member(s) | Publication date (day/month/year) |
|---|---|---|---|
| | | ES 2734253 T3 | 05 December 2019 |
| | | JP 2018021046 A | 08 February 2018 |
| | | JP 6448726 B2 | 09 January 2019 |
| | | MX 2009010082 A | 19 October 2009 |
| | | WO 2008115516 A3 | 13 November 2008 |
| | | WO 2008115516 A8 | 26 November 2009 |
| | | KR 20150046384 A | 29 April 2015 |
| | | KR 101791757 B1 | 30 October 2017 |
| | | US 2014073669 A1 | 13 March 2014 |
| | | US 9181216 B2 | 10 November 2015 |
| | | UY 30977 A1 | 31 October 2008 |
| | | ES 2601131 T3 | 14 February 2017 |
| | | EP 2142534 A2 | 13 January 2010 |
| | | EP 2142534 B1 | 10 August 2016 |
| | | US 2012165536 A1 | 28 June 2012 |
| | | US 8614328 B2 | 24 December 2013 |
| | | RU 2009138500 A | 27 April 2011 |
| | | RU 2471794 C2 | 10 January 2013 |
| | | AU 2008229383 A1 | 25 September 2008 |
| | | AU 2008229383 B2 | 05 September 2013 |
| | | BRPI 0809011 A2 | 16 September 2014 |
| | | BRPI 0809011 A8 | 15 January 2019 |
| | | NI 200900170 A | 09 September 2010 |
| | | CA 2681633 A1 | 25 September 2008 |
| | | CA 2681633 C | 09 January 2018 |
| | | US 2016024048 A1 | 28 January 2016 |
| | | US 9920027 B2 | 20 March 2018 |
| | | MY 180812 A | 09 December 2020 |
| | | AR 065810 A1 | 01 July 2009 |
| | | JP 2014224118 A | 04 December 2014 |
| | | JP 5927241 B2 | 01 June 2016 |
| | | ECSP 099663 A | 30 October 2009 |
| | | USRE 46639 E | 19 December 2017 |
| | | NZ 599199 A | 25 October 2013 |
| | | CR 20140368 A | 25 August 2014 |
| | | JP 2010522170 A | 01 July 2010 |
| | | JP 2016065067 A | 28 April 2016 |
| | | JP 6373818 B2 | 15 August 2018 |
| | | IL 200990 A0 | 17 May 2010 |
| | | IL 200990 A | 31 May 2016 |
| | | CO 6231033 A2 | 20 December 2010 |
| | | UA 97976 C2 | 10 April 2012 |
| | | CR 11036 A | 06 October 2009 |
| | | KR 20170093996 A | 16 August 2017 |
| WO 2021055756 A1 | 25 March 2021 | EP 4031241 A1 | 27 July 2022 |
| | | JP 2022548775 A | 21 November 2022 |
| | | AU 2020348849 A1 | 07 April 2022 |
| | | CA 3155010 A1 | 25 March 2021 |
| | | US 2022380368 A1 | 01 December 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210060175
- WO 2008115516 A2
- WO 2011100380 A1
- WO 2019226770 A1
- WO 2019014100 A1
- WO 2020064002 A1
- WO 2022017365 A


### Non-patent literature cited in the description


- *Journal of Medicinal Chemistry*, 2020, vol. 63 (13), 6648-6676